# EUROPEAN PATENT APPLICATION

(11) **EP 2 325 295 A2**
(43) Date of publication of application: **25.05.2011**
(21) Application number: 10180101.7
(22) Date of filing: 25.08.2006
(51) Int. Cl.: C12N 1/20, A23G 1/02, C12R 1/02, C12R 1/01, C12P 39/00

(54) **Cacao starter cultures and fermentation method**

(30) Priority: 12.09.2005 WO PCT/EP2005/009777
(62) Divisional of application: 06791676.7
(71) Applicant: Vrije Universiteit Brussel, 1050 Brussel (BE); Barry Callebaut AG, 8005 Zurich (CH)
(72) Inventor: De Vuyst, Luc, 1050 Brussel (BE); Camu, Nicolas, 9280 Lebbeke-Wieze (BE)
(74) Representative: Michalík, Andrej

(57) **Abstract**

The present invention relates to a method for regulating fermentation of plant material comprising beans and/or pulp derived from fruit pods of the species *Theobroma* cacao, said method comprising adding to the plant material a composition comprising at least one strain of lactic acid bacteria and/or at least one strain of acetic acid bacteria.

SEQUENCE LISTING

<110>  Vrije Universiteit Brussel
       Barry Callebaut AG

<120>  STARTER CULTURES AND FERMENTATION METHOD

<130>  VUB-021-EP-DIV

<150>  PCT/EP2005/009777
<151>  2005-09-12

<160>  12

<170>  PatentIn version 3.3

<210>  1
<211>  1442
<212>  DNA
<213>  A. Ghanensis

<400>  1
agcgaacgct ggcggcatgc ttaacacatg caagtcgcac gaacctttcg gggttagtgg      60

cggacgggtg agtaacgcgt aggaatctgt ccatgggtgg gggataactc tgggaaactg     120

gagctaatac cgcatgatac ctgagggtca aaggcgcaag tcgcctgtgg aggagcctgc     180

gttcgattag ctagttggtg gggtaaaggc ctaccaaggc gatgatcgat agctggtttg     240

agaggatgat cagccacact gggactgaga cacggcccag actcctacgg gaggcagcag     300

tggggaatat tggacaatgg gggcaaccct gatccagcaa tgccgcgtgt gtgaagaagg     360

tcttcggatt gtaaagcact ttcgacgggg acgatgatga cggtacccgt agaagaagcc     420

ccggctaact tcgtgccagc agccgcggta atacgaaggg ggctagcgtt gctcggaatg     480

actgggcgta aagggcgtgt aggcggtttg tacagtcaga tgtgaaatcc ccgggcttaa     540

cctgggagct gcatttgata cgtgcagact agagtgtgag agagggttgt ggaattccca     600

gtgtagaggt gaaattcgta gatattggga agaacaccgg tggcgaaggc ggcaacctgg     660

ctcattactg acgctgaggc gcgaaagcgt ggggagcaaa caggattaga taccctggta     720

gtccacgctg taaacgatgt gtgctagatg ttgggtaact ttgttattca gtgtcgcagt     780

taacgcgtta agcacaccgc ctggggagta cggccgcaag gttgaaactc aaaggaattg     840

acgggggccc gcacaagcgg tggagcatgt ggtttaattc gaagcaacgc gcagaacctt     900

accagggctt gaatgtagag gctgtattca gagatggata tttcccgcaa gggacctcta     960

acacaggtgc tgcatggctg tcgtcagctc gtgtcgtgag atgttgggtt aagtcccgca    1020

acgagcgcaa cccctatctt tagttgccag cacgtttggg tgggcactct agagagactg    1080

ccggtgacaa gccggaggaa ggtggggatg acgtcaagtc ctcatggccc ttatgtcctg    1140

ggctacacac gtgctacaat ggcggtgaca gtgggaagct agatggtgac atcgtgctga    1200

tctctaaaag ccgtctcagt tcggattgca ctctgcaact cgagtgcatg aaggtggaat    1260

cgctagtaat cgcggatcag catgccgcgg tgaatacgtt cccgggcctt gtacacaccg    1320

cccgtcacac catgggagtt ggtttgacct taagccggtg agcgaacccg caaggggcgc    1380

agccgaccac ggtcgggtca gcgactgggg tgaagtcgta acaaggtagc cgtaggggaa    1440

cc                                                                   1442

<210>  2
<211>  1442
<212>  DNA
<213>  A. Senegalensis

<400>  2
agcgaacgct ggcggcatgc ttaacacatg caagtcgcac gaaggtttcg gccttagtgg      60

cggacgggtg agtaacgcgt aggaatctat ccatgggtgg gggataactc tgggaaactg     120

gagctaatac cgcatgatac ctgagggtca aaggcgcaag tcgcctgtgg aggagcctgc     180

gttcgattag cttgttggtg gggtaatggc ctaccaaggc gatgatcgat agctggtctg     240

agaggatgat cagccacact gggactgaga cacggcccag actcctacgg gaggcagcag     300

tggggaatat tggacaatgg gggcaaccct gatccagcaa tgccgcgtgt gtgaagaagg     360

ttttcggatt gtaaagcact ttcggcgggg acgatgatga cggtacccgc agaagaagcc     420

ccggctaact tcgtgccagc agccgcggta atacgaaggg ggctagcgtt gctcggaatg     480

actgggcgta aagggcgtgt aggcggtttg tacagtcaga tgtgaaatcc ccgggcttaa     540

cctgggagct gcatttgata cgtgcagact agagtgtgag agagggttgt ggaattccca 600
gtgtagaggt gaaattcgta gatattggga agaacaccgg tggcgaaggc ggcaacctgg 660
ctcatgactg acgctgaggc gcgaaagcgt ggggagcaaa caggattaga taccctggta 720
gtccacgctg taaacgatgt gtgctggatg ttgggcaact tagttgttca gtgtcgtagc 780
taacgcgata agcacaccgc ctggggagta cggccgcaag gttgaaactc aaaggaattg 840
acgggggccc gcacaagcgg tggagcatgt ggtttaattc gaagcaacgc gcagaacctt 900
accagggctt gtatgtgtag gctgtgtcca gagatgggca tttcccgcaa gggacctaca 960
gcacaggtgc tgcatggctg tcgtcagctc gtgtcgtgag atgttgggtt aagtcccgca 1020
acgagcgcaa cccctatctt tagttgccag catgtttggg tgggcactct agagagactg 1080
ccggtgacaa gccggaggaa ggtggggatg acgtcaagtc ctcatggccc ttatgtcctg 1140
ggctacacac gtgctacaat ggcggtgaca gtgggaagct agatggcgac atcgtgctga 1200
tctctaaaag ccgtctcagt tcggattgca ctctgcaact cgagtgcatg aaggtggaat 1260
cgctagtaat cgcggatcag catgccgcgg tgaatacgtt cccgggcctt gtacacaccg 1320
cccgtcacac catgggagtt ggtttgacct taagccggtg agcgaacccg caaggggcgc 1380
agccgaccac ggtcgggtca gcgactgggg tgaagtcgta acaaggtagc cgtaggggaa 1440
cc 1442

<210> 3
<211> 18
<212> DNA
<213> Artificial

<220>
<223> primer

<400> 3
agcagtagga atcttcca 18

<210> 4
<211> 18
<212> DNA
<213> Artificial

<220>
<223> primer

<400> 4
atttcaccgc tacacatg 18

<210> 5
<211> 22
<212> DNA
<213> Artificial

<220>
<223> primer

<400> 5
gtcgtcagct cgtgtcgtga ga 22

<210> 6
<211> 21
<212> DNA
<213> Artificial

<220>
<223> primer

<400> 6
cccgggaacg tattcaccgc g 21

<210> 7
<211> 40
<212> DNA
<213> Artificial

<220>
<223> GC-rich sequence

<400> 7
cgcccgccgc gccccgcgcc cggcccgccg cccccgcccc 40

<210> 8
<211> 18
<212> DNA
<213> Artificial

<220>
<223>  primer


<220>
<221>  modified_base
<222>  (1)..(1)
<223>  a is I

<220>
<221>  modified_base
<222>  (2)..(2)
<223>  a is I

<220>
<221>  modified_base
<222>  (3)..(3)
<223>  a is I

<220>
<221>  modified_base
<222>  (4)..(4)
<223>  a is I

<220>
<221>  modified_base
<222>  (7)..(7)
<223>  a is I

<220>
<221>  modified_base
<222>  (10)..(10)
<223>  a is I

<220>
<221>  modified_base
<222>  (15)..(15)
<223>  a is I

<400>  8
aaaacgacga catcaggc                                                    18


<210>  9
<211>  17
<212>  DNA
<213>  Artificial

<220>
<223>  primer


<220>
<221>  modified_base
<222>  (1)..(1)
<223>  a is I

<220>
<221>  modified_base
<222>  (2)..(2)
<223>  a is I

<220>
<221>  modified_base
<222>  (3)..(3)
<223>  a is I

<220>
<221>  misc_feature
<222>  (6)..(6)
<223>  n is a, c, g, or t

<220>
<221>  misc_feature
<222>  (9)..(9)
<223>  n is a, c, g, or t

<220>
<221>  misc_feature
<222>  (14)..(14)
<223>  n is a, c, g, or t

<400>  9
aaacgncgnc atcnggc                                                     17


<210>  10
<211>  15
<212>  DNA
<213>  Artificial

<220>
<223>  primer

<400>  10
gtggtggtgg tggtg                                                       15

<210>  11
<211>  20
<212>  DNA
<213>  Artificial

<220>
<223>  primer

<400>  11
agagtttgat cctggctcag                                                    20


<210>  12
<211>  20
<212>  DNA
<213>  Artificial

<220>
<223>  primer

<400>  12
aaggaggtga tccagccgca                                                    20

## Description

### FIELD OF THE INVENTION

The invention relates to the field of fermentation of organic material, particularly plant material and more specifically cocoa material, such as beans and pulp. The invention relates to methods of regulating the fermentation of such materials, in particular using compositions comprising specific microorganisms, such as bacteria and/or yeast.

### BACKGROUND OF THE INVENTION

Cocoa beans are the principal raw material for chocolate production. These seeds are derived from the fruit pods of the tree *Theobroma* cacao, which is cultivated in plantations in the equatorial zone, e.g., in Ivory Coast, Ghana, and Indonesia. The cocoa beans are embedded in a mucilaginous pulp inside the pods. Raw cocoa beans have an astringent, unpleasant taste and flavour, and have to be fermented, dried, and roasted to obtain the desired characteristic cocoa flavour and taste. The chocolate flavour is influenced by the origin of the cocoa beans, the cocoa cultivar, the on-the-farm fermentation and drying process, and the roasting and further processing performed by the chocolate manufacturer.

After removal of the beans from the pods, the first step in cocoa processing is a spontaneous 3 to 10-day fermentation of beans and pulp in heaps, boxes, baskets, or trays, heap fermentation being the most dominant method in Ghana, whereas in Ivory Coast the box fermentation is the most preferred. A microbial succession of yeasts, lactic acid bacteria (LAB), and acetic acid bacteria (AAB) takes place during fermentation. The yeasts depectinise the pulp and produce ethanol from sugars and citric acid under anaerobic conditions in an acid, carbohydrate-rich environment. As more pulp is drained away, more ethanol is produced and both temperature and pH increase, creating ideal conditions for the growth of LAB and AAB. LAB convert sugars and organic acids into lactic acid. As more air is coming in, AAB start to develop and oxidize the ethanol initially produced by the yeasts to acetic acid. Ethanol and acetic acid diffuse into the beans and this, in combination with the heat produced by this exothermic bioconversion, causes the death of the seed embryo. This in turn initiates biochemical changes in the beans leading to the formation of precursor molecules for the development of a characteristic aroma, flavour, and colour of the beans. These properties are further developed during drying, roasting, and final processing of well-fermented cocoa beans. The activity of yeast, LAB, and AAB is thus essential for the production of high-quality cocoa.

However, the spontaneous cocoa fermentation process is very inhomogeneous and suffers from great variations in both microbial counts and species composition and hence metabolites. These variations seem to depend on many factors including country, farm, pod ripeness, post-harvest pod age and storage, pod diseases, type of cocoa, variations in pulp/bean ratio, the fermentation method, size of the batch, season and weather conditions, the turning frequency or no turning, the fermentation time, etc. which makes reproducibility of fermentation particularly difficult.

In view of the above, controlling cocoa fermentation is therefore very challenging and requires detailed understanding of the microbial contributions to this process. Yeasts are the most studied microorganisms involved in cocoa fermentation and a number of important yeast species have been found. For example, *Saccharomyces cerevisiae* and *Candida zemplinina* seem to play a role In box fermentations and *Hanseniaspora guilliermondii, Pichia membranifaciens* and *Candida krusei* may play a role in heap fermentations.

In contrast, few studies focused on isolation/identification of LAB and AAB that are also important for cocoa fermentation. For example, it is difficult to isolate and grow AAB, the taxonomy of AAB is not fully established, the older phenotype-based methods cannot always reliably distinguish separate species or strains, and molecular identification methods of AAB is far from routine.

To solve the above deficiencies, the present inventors developed a genotyping method based on REP-PCR using the (GTG)₅ primer to genotypically distinguish LAB and AAB strains. They applied the method, in combination with phenotypic methods and culture-independent methods such as PCR-DGGE, to evaluate the microbial composition of cocoa fermentations in Ghana. It is recognised that cocoa from Ghana is of the best quality. Therefore, the understanding of the microbial contributions to the fermentation of this cocoa enables the inventors to formulate compositions (such as starter cultures) which allow to regulate cocoa fermentation and thereby the characteristics of the fermented cocoa beans and the resulting processed chocolate. The invention provides such compositions and their use to regulate and control cocoa fermentation.

According to the inventors' best knowledge, there exists no successful demonstration of the use of starter cultures to regulate the fermentation of cocoa. Previously, a Brazilian group (Prof. Dr. R.F. Schwan, Cocoa Research Centre/SETEA, Bahia, Brazil) analysed the cocoa fermentation process in wooden boxes in Brazil and experimented with a defined microbial cocktail inoculum, without a detailed analysis of the microbial consortium beforehand (Schwan et al.: Appl Envir Microbiol 64, 1477-1483, 1998; J Appl Bacteriol 79: 96S-107S, 1995; Enz Microbial Technol 21: 234-244, 1997; 14th International Cocoa Research Conference, p. 79, 2003; J Food Sci 51: 1583-1584, 1986). Other attempts to use starter cultures for cocoa fermentations were performed without removal of the natural microbiota and using non-fermentative yeasts or yeasts from culture collections with no success, and without adding the necessary LAB or AAB, which were not studied. There was no evidence that the fermentation could be improved or accelerated with these approaches (Sanchez et al. Lebensmitteln Wissenschaft und Technology 18: 69-76, 1985; Samah et al. J Food Sci Technol 29: 341-343, 1992). Further, GB2059243 describes a process for fermentation of cocoa beans with the consecutive use of a pectinolytic yeast and an acetic acid bacterium in an aqueous medium, with agitation and aeration. GB2241146 deals with the mechanical depulping of the cocoa beans before fermentation.

### SUMMARY OF THE INVENTION

In an aspect the invention provides a method for regulating fermentation of organic material comprising adding to the organic material a composition comprising at least one strain of lactic acid bacteria and/or acetic acid bacteria. By regulating the fermentation, the present invention allows for controlling or manipulating various aspects of fermentation, such as by means of example and not limitation, the rate of fermentation, the extent of fermentation, rapidity and productivity of the fermentation, the quality and/or quantity of both desirable and undesirable substances present in the fermented material, and characteristics of the fermented material and/or products obtained by further processing of the fermented material. Hereby, the present invention advantageously allows for countering the non-reproducible nature of spontaneous fermentation and for regulating the fermentation process and the characteristics of the fermented material and products obtained there from.

In a particular embodiment the invention relates to a method for regulating fermentation of plant material essentially consisting of beans and/or pulp derived from fruit pods of the species *Theobroma cacao,* said method comprising adding to the plant material a composition A comprising at least one strain of lactic acid bacteria and/or at least one strain of acetic acid bacteria, whereby said composition A is added at the start of the fermentation.

In another embodiment the invention relates to a method for regulating fermentation of plant material essentially consisting of beans and/or pulp derived from fruit pods of the species *Theobroma cacao,* said method comprising:
adding to the plant material a composition B comprising at least one strain of lactic acid bacteria and/or at least one strain of acetic acid bacteria, whereby said composition B is added at the start and/or during the first 24 hours of the fermentation, and/or
adding a composition C comprising at least one strain of lactic acid bacteria and/or at least one strain of acetic acid bacteria, whereby said composition is added after 24 hours of fermentation.

In a further aspect, the invention provides compositions comprising at least one strain of lactic acid bacteria and/or acetic acid bacteria, and optionally further comprising at least one strain of yeast, which are suitable for regulating fermentation of organic material. The invention provides compositions that are particularly suitable for being added at the start of fermentation of plant material, such as cocoa beans and pulp. The invention also provides compositions that are particularly suitable for being added at the start and/or early during fermentation of plant material, such as cocoa beans and pulp. The invention also provides compositions that are particularly suitable for being added late during fermentation of plant material such as cocoa beans and pulp.

In a further aspect, the invention provides for use of the said compositions for regulating fermentation of organic material.

The present invention also provides fermented organic material obtainable by the method of the invention and products produced from said material.

In further aspects, the invention provides specific novel isolates of lactic acid bacteria and acetic acid bacteria, which belong to either previously existing or previously undisclosed taxa.

In particularly useful embodiments, the methods and compositions of the present invention are useful in fermentation of plant material, in particular cocoa material, and allow for advantageously controlling the characteristics of the cocoa material and products produced there from, for example, all kind of organoleptic, nutritional, and technological properties and flavour and quality assets, i.e. taste, flavour, fatty acid composition, polyphenol content, etc., of the fermented beans as well as of the resulting cocoa products, such as chocolate.

The invention is described here below with reference to its particular embodiments.

### DETAILED DESCRIPTION OF THE INVENTION

In an aspect the invention provides a method for regulating fermentation of organic material comprising adding to the organic material a composition comprising at least one strain of lactic acid bacteria and/or acetic acid bacteria.

As used herein, the term "strain" refers in general to a closed population of organisms of the same species. Accordingly, the term "strain of lactic acid bacteria and/or acetic acid bacteria" generally refers to a strain of a species of lactic acid bacteria and/or acetic acid bacteria. More particularly, the term "strain" refers to members of a microbial species, wherein such members have different genotypes and/or phenotypes. Herein, the term "genotype" encompasses both the genomic and the recombinant DNA content of a microorganism. Herein, the term "phenotype" refers to observable physical characteristics dependent upon the genetic constitution of a microorganism. As one skilled in the art would recognize, microbial strains are thus composed of individual microbial cells having a common genotype and/or phenotype. Further, individual microbial cells may have specific characteristics (e.g., a specific rep-PCR pattern) which may identify them as belonging to their particular strain.

A microbial strain may comprise one or more isolates of a microorganism. As used herein, the term "isolate" refers to cultured microorganisms grown from a single colony taken from a primary isolation plate. An isolate, which is presumed to be derived from a single microorganism.

As used herein, the terms "microorganism" or "microbial" cover any generally unicellular organism, which can be propagated and manipulated in a laboratory. In particular, the terms may refer to bacteria, yeast, fungi, algae, protozoa, human, animal and plant cells. In the present invention, the terms preferably relate to bacteria and/or yeast and/or fungi. In the present invention, the term microorganism typically denotes a live microorganism, i.e., capable of propagation and/or having metabolic activity.

The term "organic material" as used herein refers to living organisms or their remains and in particular relates to plant material. The term "plant material" includes anything that is or was live vegetation, in particular plants and any parts thereof, the latter including but not restricted to leafs, flowers, roots, seeds, stems, fruits, pods, beans, berries, grains, pulp and the like.

In the present invention, the plant material is preferably derived from any species of the genera *Theobroma* or *Herrania* or inter- and intra-species crosses thereof, and more preferably from the species *Theobroma cacao* and *Theobroma grandiflorum.* The species *Theobroma* cacao as used herein comprises all varieties, particularly all commercially useful varieties, including but not limited to Criollo, Forastero, Trinitario, Arriba, and crosses and hybrids thereof. Cocoa beans derived from the fruit pods of *Theobroma cacao* are the principal raw material for chocolate production. The cocoa beans are embedded in a mucilaginous pulp inside the pods. After the pods are harvested, the cocoa beans (usually including at least a portion of the surrounding pulp) are recovered from the pods. Accordingly, the plant material used in the method of the invention may preferably comprise cocoa beans derived from the fruit pods of *Theabroma cacao,* and may further comprise the pulp derived from the said fruit pods. In an embodiment, the plant material may consist essentially of cocoa beans and the pulp derived from the fruit pods of *Theobroma cacao.*

It is noted that the terms "cocoa" and "cacao" as used herein are considered as synonyms.

As used herein, the term "fermentation" refers generally to any activity or process involving enzymatic decomposition (digestion) of organic materials by microorganisms. The term fermentation as used herein is to be understood to include any type of fermentation including but not limited to substantially zero growth fermentation, i.e. a fermentation process wherein the cell density of the microorganisms applied in the fermentation process will be substantially zero and wherein the microorganisms will not undergo a significant population growth, including under-fermentation, i.e. a fermantation process wherein the fermentation is perfomed during an insufficient period of time, and including over-fermentation, e.g. a fermentation process wherein fermentation is performed for a too long period of time. The fermentation process may also involve production of useful compounds and substances, typically organic compounds and substances, by said microorganisms. The said compounds may advantageously influence or determine one or more characteristics of the fermented material and/or materials or products prepared by further processing involving the said fermented material. By means of example and not limitation, such characteristics may involve various sensorial, organoleptic, nutritional, technological, compositional, or qualitative properties of the fermented material and/or further products there from, e.g., contents of particular compounds, taste, flavour, aroma, texture, colour, rheology, etc. The term "fermentation" encompasses both anaerobic and aerobic processes, as well as processes involving a combination or succession of one or more anaerobic and/or aerobic stages. In the present invention, fermentation preferably involves the decomposition (digestion) of plant material as defined above.

In a preferred embodiment, the present invention relates to the fermentation of plant material comprising the beans derived from *Theobroma cacao* and/or plant material consisting essentially of the beans and pulp derived from *Theobroma* cacao. As described above, in cacao production after removal of the beans (and the surrounding pulp) from the pods, the beans and pulp are typically subjected to a spontaneous fermentation, which is important for the characteristics of the resulting cocoa, such as its aroma, flavor and color. It is thus an object of the present invention to provide a method for regulating the fermentation of organic material, preferably plant material, even more preferably plant material comprising the beans derived from *Theobroma cacao* and/or plant material consisting essentially of the beans and pulp derived from *Theobroma cacao* (the latter two plant materials are collectively referred to herein as "cocoa beans and pulp").

The term "regulating" as used herein in relation to the fermentation of organic material encompasses but is not limited to initiating a fermentation process and/or initiating a particular stage of the fermentation process; accelerating or decelerating a fermentation process and/or accelerating or decelerating a particular stage of the fermentation process; initiating and/or accelerating or decelerating the transition from one stage of a fermentation process to another stage of the fermentation process (e.g., the transition from mainly yeast-mediated fermentation to mainly LAB-mediated fermentation, or from the mainly LAB-mediated fermentation to mainly AAB-mediated fermentation during the fermentation process of cocoa beans and pulp); altering the conditions of the fermentation, such as, e.g., temperature or pH; altering the composition of the fermented material (e.g., altering the decomposition or production of particular substances present in the fermented material); altering the identity and/or quantity of microbial strains present in and/or carrying out the fermentation process; enhancing or suppressing the growth of particular microorganisms etc.

By regulating the above and other aspects of fermentation, the present invention allows for controlling or manipulating, by means of example and not limitation, the rate of fermentation, the extent of fermentation, rapidity and productivity of the fermentation, the quality and/or quantity of both desirable and undesirable substances present in the fermented material, and characteristics of the fermented material and/or products obtained by further processing of the fermented material.

In a preferred embodiment, by using a composition according to the present invention the fermentation of the cocoa plant material as defined herein can be significantly accelerated, for instance fermentation may be up to 1.3, 1.5, 1.8, 2, 2.5, or even 3 times faster compared to hitherto known processes. In another embodiment, by use of a composition according to the present invention fermentation of the cocoa plant material as defined herein can significantly influence one or more fermentation processes/stages and thus fermentation productivity, and increase productivity preferably with at least 5%, preferably with at least 10%, at least 15%, or even at least 20% compared to hitherto known processes.

In a preferred embodiment, by using a composition according to the present invention the composition of the fermented cocoa beans can be significantly modified. For instance the amount of remaining polyphenols obtained by regulating fermentation according to the present invention may be two times higher compared to hitherto known processes. Additionally the polyphenol composition in the fermented beans obtained by regulating fermentation according to the present invention can be optimised by changing the balance in hydrolysable and condensed tannins. By using the composition according to the present invention the amount of flavan-3-ols and oligomeric proanthocyanidins (dimers, trimmers, tetramers and pentamers) can be increased with at least 10%, or even at least 20% compared to hitherto known processes.

In a preferred embodiment, by using a composition according to the present invention the amount and composition of organic acids in fermented cocoa beans can be altered. The amount of organic acid e.g. lactic acid may be 5%, preferably with at least 10%, at least 15%, or even at least 20% compared to hitherto known processes.

In a preferred embodiment the present invention provides a method for regulating the fermentation of cocoa beans and pulp. The said method allows for controlling or manipulating the development of desirable characteristics of the fermented cocoa beans and the cocoa products prepared there from. By means of example and not limitation, the present method may allow for controlling the index of fermentation of cocoa beans (cut test, coloration), appearance and sensorial properties of the fermented beans; for, e.g., sensorial properties, organic acids, sugar alcohols, polyphenol, theobromine, or caffeine content of roasted beans; and for, e.g., organoleptic characteristics (taste, flavour, aroma, texture, colour, rheology, crystallisation behaviour, etc.), nutritional characteristics, technological properties, quality assets (aroma, taste, flavour, fatty acid composition, polyphenol content, theobromine content, caffeine content, etc.) of cocoa products, such as, particularly chocolate.

The method of the invention provides for regulating fermentation of organic material, preferably plant material, more preferably cocoa beans and pulp, by adding thereto a composition comprising at least one strain of lactic acid bacteria and/or acetic acid bacteria. In a preferred embodiment, the composition is added at the start of the fermentation. Alternatively or in combination therewith, several compositions comprising at least one strain of lactic acid bacteria and/or acetic acid bacteria may be added either at the start or in early stages (e.g. during first 24 h) and/or at later stages (e.g. after 24 h) of fermentation.

Lactic acid bacteria play an important role In the succession of microbial species during the fermentation of cocoa beans and pulp and will be useful for regulating the said fermentation.

As defined herein, the term "tactic acid bacteria" (LAB) includes all species of the genera *Bifidobacterium, Camobacterium, Enterococcus, Lactobacillus, Lactococcus, Leuconostoc, Oenococcus, Pediococcus, Streptococcus, Tetragenococcus, Vagococcus* and *Weissella,* and *Brevibacterium* and *Propionibacterium,* and preferably refers to the genera of the *Enterococcus, Lactobacillus, Leuconostoc,* and *Weissella* (for taxonomy of lactic acid bacteria, see for example, Stiles and Holzapfel. Lactic acid bacteria of foods and their current taxonomy. Int J Food Microbiol 36: 1-29, 1997). The majority of species belonging to lactic acid bacteria may possess, or may have originally possessed in their wild-type form, the capacity to ferment carbohydrates to produce organic acids, such as lactic acid (predominantly), acetic acid, formic acid, succinic acid, or propionic acid, etc and other organic compounds such as ethanol. The majority of the species belonging to lactic acid bacteria may be characterised as Gram positive, catalase negative, usually microaerophilic or anaerobic, non-motile bacteria which may be cocci or rods. The anaerobic genus *Bifidobacterium* is also typically included in the group of lactic acid bacteria.

In a preferred embodiment, the at least one strain of lactic acid bacteria may belong to a species chosen from the group comprising *Lactobacillus acidophilus, Lactobacillus brevis, Lactobacillus buchneri, Lactobacillus casei, Lactobacillus casei* subspecies *pseudoplantarum, Lactobacillus collinoides, Lactobacillus cellobiosus, Lactobacillus delbrueckii, Lactobacillus fermentum, Lactobacillus fructivorans, Lactobacillus gasseri, Lactobacillus hilgardii, Lactobacillus kandleri, Lactobacillus lactis, Lactobacillus mali, Lactobacillus parabrevis, Lactobacillus paraplantarum, Lactobacillus pentosus, Lactobacillus plantarum, Lactococcus lactis, Leuconostoc carnosum, Leuconostoc durionis, Leuconostoc mesenteroides, Leuconostoc oenos, Leuconostoc paramesenteroides, Leuconostoc pseudomesenteroides, Oenococcus oeni, Pediococcus acidilactici, Pediococcus cerevisiae, Pediococcus dextrinicus, Weissella cibaria, Weissella confusa, Weissella kimchii, Weissella paramesenteroides, Enterococcus casseliflavus, Enterococcus columbae, or Enterococcus faecium, and preferably of the group comprising Lactobacillus brevis, Lactobacillus fermentum, Lactobacillus mali, Lactobacillus paraplantarum, Lactobacillus plantarum, Leuconostoc mesenteroides, Leuconostoc paramesenteroides, Leuconostoc pseudomesenteroides, Enterococcus casseliflavus, or Enterococcus faecium,* and more preferably of the group comprising *Lactobacillus brevis, Lactobacillus fermentum, Lactobacillus mali, Lactobacillus plantarum, Leuconostoc mesenteroides, Leuconostoc pseudomesenteroides, Enterococcus casseliflavus, or Enterococcus faecium.*

In another preferred embodiment, the at least one strain of lactic acid bacteria may belong to a species chosen from the group comprising *Lactobacillus fermentum, Lactobacillus plantarum, Leuconostoc pseudomesenteroides or Enterococcus casseliflavus.* The inventors surprisingly realised that these four LAB species were the most represented among the isolates taken from cocoa bean and pulp fermentations as described in the Examples section. Accordingly, these species may significantly contribute to the fermentation of cocoa beans and pulp and will be useful for regulating the said fermentation process.

In a further preferred embodiment, the at least one strain of lactic acid bacteria may belong to a species chosen from the group comprising *Lactobacillus fermentum* or Lactobacillus *plantarum.* The inventors surprisingly found that these two LAB species were the most represented among the isolates taken from cocoa bean and pulp fermentations as described in the Examples section. Accordingly, these species may play a major role in the fermentation of cocoa beans and pulp and will be useful for regulating the said fermentation process.

In a further preferred embodiment, the at least one strain of lactic acid bacteria may belong to a species chosen from the group comprising *Leuconostoc pseudomesenteroides* or *Enterococcus casseliflavus.* The inventors surprisingly found that these LAB species, which were to the inventors best knowledge never before reported to occur in fermentations of cocoa bean and pulp, were unexpectedly considerably represented among the isolates taken from such fermentations as described in the Examples section. Accordingly, these species may, which is surprising, significantly contribute to the fermentation of cocoa beans and pulp and will be particularly useful for regulating the said fermentation process.

The inventors also discovered that cocoa fermentations were usually characterised by a high level of LAB present at the start of the fermentation; LAB grew during fermentation to maximum populations typically after 30 h of fermentation (mid-crop fermentations) or 40 h of fermentation (main-crop fermentations). This was followed by a slight decrease and/or stabilising of LAB upon prolonged fermentation. Accordingly, in a preferred embodiment of the method for regulating fermentation of organic material, more particularly plant material, even more particularly cocoa beans and pulp, comprises adding thereto a composition comprising at least one strain of lactic acid bacteria at the start and/or earlier stages of the fermentation, e.g., at between 0 and 150h, 0 and 140h, 0 and 130h, 0 and 120h, 0 and 100h, 0 and 90h, 0 and 80h, 0 and 70h, 0 and 60h, 0 and 50h, 0 and 48h, 0 and 40h, 0 and 30h, 0 and 24h, 0 and 20h, 0 and 10h, 0 and 5h, or 0 and 1h or at 0h after the start of fermentation. The "start of fermentation" corresponds essentially to the time when the organic material is deposited in a fermentor (herein broadly encompassing any area, location, space or compartment where fermentation is effected, irrespective of whether it is in contact with or isolated from the environment) e.g., in a heap or in a box.

In addition, while *Lactobacillus fermentum* and *Lactobacillus plantarum* were the most abundant species throughout fermentation of cocoa beans and pulp, the inventors surprisingly discovered that *Lb. fermentum* increased while *Lb. plantarum* decreased during fermentation as a function of time. It is therefore believed that *Lb. plantarum* may play a major role at the start and/or in the earlier stages of the fermentation, while *Lb. fermentum* may become more important in the later stages and/or towards the end of the fermentation. Accordingly, a preferred embodiment of the method for regulating fermentation of organic material, more particularly plant material, even more particularly cocoa beans and pulp, comprises adding thereto a composition comprising at least one strain of *Lb. plantarum* at the start and/or earlier stages of the fermentation, e.g., at between 0 and 150h, 0 and 140h, 0 and 130h, 0 and 120h, 0 and 100h, 0 and 90h, 0 and 80h, 0 and 70h, 0 and 60h, 0 and 48h, 0 and 50h, 0 and 40h, 0 and 30h, 0 and 24h, 0 and 20h, 0 and 10h, 0 and 5h, or 0 and 1 h or at 0h after the start of fermentation.

In another preferred embodiment of the method for regulating fermentation of organic material, more particularly plant material, even more particularly cocoa beans and pulp, comprises adding thereto a composition comprising at least one strain of *Lb. fermentum* at later stages of the fermentation, e.g., at between 0 and 150h, 10 and 150h, 20 and 150h, 24 and 150h, 30 and 150h, 40 and 150h, 48 and 150h, 50 and 150h, 60 and 150h, 70 and 150h, 80 and 150h, 90 and 150h, 100 and 150h, 110 and 150h, 120 and 150h, 130 and 150h, 140 and 150h, or at 150h after the start of fermentation.

The inventors further found that *Leuconostoc pseudomesenteroides* and *Enterococcus casseliflavus* were found in the beginning of almost every fermentation and that their numbers may decline relatively rapidly. It is therefore believed that *Leuconostoc pseudomesenteroides* and/or *Enterococcus casseliflavus* may play a role particularly (though not only) at the start and/or in the earlier stages of the fermentation. Accordingly, a preferred embodiment of the method for regulating fermentation of organic material, more particularly plant material, even more particularly cocoa beans and pulp, comprises adding thereto a composition comprising at least one strain of *Leuconostoc pseudomesenteroides* and/or *Enterococcus casseliflavus* at the start and/or earlier stages of the fermentation, e.g., at between 0 and 150h, 0 and 140h, 0 and 130h, 0 and 120h, 0 and 100h, 0 and 90h, 0 and 80h, 0 and 70h, 0 and 60h, 0 and 50h, 0 and 48h, 0 and 40h, 0 and 30h, 0 and 24h, 0 and 20h, 0 and 10h, 0 and 5h, or 0 and 1 h or at 0h after the start of fermentation.

The inventors further surprisingly found among the isolates taken from cocoa bean and pulp fermentations as described in the Examples section an isolate corresponding to a previously undisclosed taxon related to *Weissella,* with a herein proposed designation *Weissella ghanensis.* The said isolate was deposited on September 7, 2005 under the Budapest Treaty with the Belgian Coordinated Collections of Microorganisms (BCCM) under accession number LMG P- 23179. The new taxon according to the present invention has the biological characteristics of the said deposit. Accordingly, the present invention also relates to a method for regulating fermentation of organic material, more particularly plant material, even more particularly cocoa beans and pulp, comprising adding thereto a composition comprising at least the strain represented by the isolate deposited with BCCM under accession number LMG P-23179, or a mutant or variant thereof. In a related aspect, the present invention relates to a bacterial strain represented by the isolate deposited with BCCM under accession number LMG P-23179 and to mutants or variants thereof. In particular, the said bacterial strain may be preferably substantially isolated from its natural environment. According to the disclosure of this application, persons skilled in the art will be able to discover or prepare a mutant or variants of any bacterial strains and/or isolates described herein, e.g., by means of traditional screening or artificial manipulation known in the art, such as selections under a stress (e.g., a temperature or a chemical agent) or mutagenesis (e.g., using chemical, physical or biological agents). Accordingly, mutants or variants of any bacterial strains and/or isolates described herein fall within the scope of the present invention. The invention also encompasses an (isolated) lactic acid bacterium showing at least 93% 16S rRNA sequence similarity with the strain *Weissella ghanensis* (herein, the 16S rRNA sequence of *Weissella ghanensis* refers primarily to this sequence as comprised in the isolate deposited under accession number LMG P-23179 as described above). In further embodiments, such lactic acid bacterium may show at least 93%. e.g., at least 94%, at least 95%. e.g., at least 96%, at least 97%, e.g., at least 97.5%, 98%, at least 98.5%, or at least 99% 16S rRNA sequence similarity with the strain *Weissella ghanensis.* Sequence similarity may typically be determined by known as such alignment algorithms, such as BLAST, and involves the proportion of positions at which two nucleic acid sequences share the same base pair. Further, the invention also relates to the strain *Weissella ghanensis,* its mutants and variants, and bacteria having at least 93% 16S rRNA sequence homology to the said strain, when further modified using recombinant DNA techniques.

As will be understood by a skilled person, the above defined novel strain denoted *Weissella ghanensis,* its mutants and variants, and the bacteria having at least 93% 16S rRNA sequence similarity to the said strain, and any of the above when further modified by recombinant DNA techniques, is claimed herein for any and all uses, such as by means of example and not limitation, in fermentation of organic materials (e.g., cocoa fermentation), food production (e.g., brewing), and production of pharmaceutical substances. In a preferred embodiment, the present method relates to the use of the lactic acid bacteria *Weissella ghanensis* strain as defined herein, for regulating fermentation of plant material essentially consisting of beans and/or pulp derived from fruit pods of the species *Theobroma cacao,* and preferably for regulating fermentation of plant material consisting of beans and/or pulp derived from fruit pods of the species *Theobroma cacao.*

In one preferred embodiment, the present method for regulating fermentation of cocoa beans and pulp may comprise adding at the start of the fermentation a composition comprising at least one strain of lactic acid bacteria selected from the genera of the *Enterococcus, Lactobacillus, Leuconostoc,* and *Weissella,* and/or any combination thereof.

In another embodiment, the present method for regulating fermentation of cocoa beans and pulp may comprise adding at the start and/or during the first 24 hours of the fermentation, and for instance during the first 1, 5, 7, 10, 12, 15, 18, 20 and 22 hours of the fermentation, a composition which comprises at least one strain of lactic acid bacteria selected from the genera of the *Enterococcus, Lactobacillus, Leuconostoc,* and *Weissella,* and/or any combination thereof. Preferably the method comprises adding at the start and/or during the first 24 hours of the fermentation, and for instance during the first 1, 5, 7, 10, 12, 15, 18, 20 and 22 hours of the fermentation, a composition which comprises one or more strains of lactic acid bacteria belonging to a species chosen from the group comprising *Lactobacillus plantarum, Lactobacillus fermentum; Leuconostoc pseudomesenteroides, Enterococcus casseliflavus* and *Weissella ghanensis, Enterococcus faecium; Lactobacillus brevis; Lactobacillus mali;* and *Leuconostoc mesenteroides;* and more preferably belonging to a species chosen from the group comprising *Lactobacillus plantarum, Lactobacillus fermentum; Leuconostoc pseudomesenteroides, Enterococcus casseliflavus* and *Weissella ghanensis,* and even more preferably *Lactobacillus plantarum* and/or *Lactobacillus fermentum.*

In another preferred embodiment, the present method for regulating fermentation of cocoa beans and pulp may comprise adding after 24 hours of the fermentation, and for instance after 30, 35, 40, 45, 48, 50, 55, 60, 65, 70, 72 hours of the fermentation, another composition which comprises at least one strain of lactic acid bacteria selected from the genera of the *Enterococcus, Lactobacillus, Leuconostoc,* and *Weissella,* and/or any combination thereof. Preferably the method comprises adding after 24 hours of the fermentation, and for instance after 30, 35, 40, 45, 48, 50, 55, 60, 65, 70, 72 hours of the fermentation, another composition which comprises one or more strains of lactic acid bacteria belonging to a species chosen from the group comprising *Lactobacillus plantarum, Lactobacillus fermentum; Leuconostoc pseudomesenteroides, Enterococcus casseliflavus* and *Weissella ghanensis;* and more preferably *Lactobacillus fermentum.*

In a preferred embodiment, the LAB applied in the present invention are LAB which are capable of assimilating citrate (citrate-utilizing strains). *Lactobacillus plantarum* strains are able to assimilate citrate. The inventors also surprisingly showed that certain strains of *Lb. fermentum* are also capable of assimilating citrate, especially during cocoa fermentation processes. It has surprisingly be seen that under other fermentation conditions of e.g. other plant material, the said strains of *Lb. fermentum* do not show this capacity of assimilating citrate. It is therefore believed that *Lb. plantarum* and especially *Lb. fermentum* may also play a major role already at the start of the fermentation and throughout the whole fermentation of cocoa beans and/or pulp. In view hereof, in one embodiment, the present invention also encompasses the addition of *Lb. plantarum* and/or *Lb. fermentum* at the start of the cocoa fermentation process.

In yet another embodiment the method for regulating fermentation of organic material, more particularly plant material, even more particularly cocoa beans and pulp, comprises adding thereto a composition comprising at least one strain of *Lb. fermentum* at the start and/or earlier stages of the fermentation, e.g., at between 0 and 150h, 0 and 140h, 0 and 130h, 0 and 120h, 0 and 100h, 0 and 90h, 0 and 80h, 0 and 70h, 0 and 60h, 0 and 50h, 0 and 48h, 0 and 40h, 0 and 30h, 0 and 24h, 0 and 20h, 0 and 10h. 0 and 5h, or 0 and 1 h or at 0h after the start of fermentation.

In still another embodiment, the invention involves the addition of a mixture of a) strains of *Lb. plantarum* and/or *Lb. fermentum* which are capable of fermenting citrate during cocoa fermentation processes, and b) strains of *Lb. plantarum* and/or *Lb*. *fermentum* which are not capable of fermenting citrate during cocoa fermentation processes. Such mixture of *Lb. plantarum* and/or *Lb. fermentum* strains could advantageouysly be added either at the start of the fermentation and/or at early stages of fermentation, e.g., e.g., at between 0 and 150h, 0 and 140h, 0 and 130h, 0 and 120h, 0 and 100h, 0 and 90h, 0 and 80h, 0 and 70h, 0 and 60h, 0 and 50h, 0 and 48h, 0 and 40h, 0 and 30h, 0 and 24h, 0 and 20h, 0 and 10h, 0 and 5h, or 0 and 1h or at 0h after the start of fermentation, and for instance during the first 1, 5, 7, 10, 12, 15, 18, 20, 22 or 24 hours of the fermentation. Examples of strains of *Lb. plantarum* capable of fermenting citrate include but are not limited to strains 80, 297, 270, and 56. Examples of strains of *Lb. fermentum* capable of fermenting citrate include but are not limited to starins 48, 12, 222, 84, 238, 296 and 238. Examples of strains of *Lb. plantarum* not capable of fermenting citrate include but are not limited to strains 273, 454, 146, and 189. Examples of strains of *Lb. fermentum* not capable of fermenting citrate include but are not limited to starins 55 and 28.

As detailed, the method of the invention provides for regulating fermentation of organic material, preferably plant material, more preferably cocoa beans and pulp; by adding thereto a composition comprising at least one strain of lactic acid bacteria and/or acetic acid bacteria. Acetic acid bacteria play an important role in the succession of microbial species during the fermentation of cocoa beans and pulp and will be useful for regulating the said fermentation.

As defined herein, the term "acetic acid bacteria" (AAB) includes all species of the genera *Acetobacter, Acidiphilium, Acidocella, Acidomonas, Craurococcus, Frateuria, Gluconacetobacter, Gluconobacter, Paracraurococcus, Rhodopila, Roseococcus, Stella, Zavarzinia, Asaia, Kozakia, Neoasaia, Saccharibacter* and *Swaminathania,* and preferably *Acetobacter, Acidiphilium, Acidocella, Acidomonas, Gluconacetobacter, Gluconobacter,* or *Saccharibacter.* The majority of species belonging to acetic acid bacteria may possess, or may have originally possessed in their wild-type form, the capacity of oxidative conversion of ethanol to acetic acid. The majority of the species belonging to acetic acid bacteria may be characterised as Gram negative, rod shaped, often motile, aerobic bacteria. Some genera of acetic acid bacteria (e.g., *Acetobacter)* may be capable of oxidizing acetic acid to CO2 and water. Other genera (e.g., *Gluconobacter*) may not be capable of such oxidation of acetic acid.

In a preferred embodiment, the at least one strain of acetic acid bacteria may belong to a species of a genus chosen from the group comprising *Acetobacter* or *Gluconobacter* or *Gluconacetobacter.*

In a further preferred embodiment, the at least one strain of acetic acid bacteria may belong to a species chosen from the group comprising *Acetobacter rancens, Acetobacter xylinum, Acetobacter ascendens, Acetobacter lovaniensis, Acetobacter aceti, Acetobacter aceti* subsp. *liquefaciens, Acetobacter cerevisiae, Acetobacter cibinongensis, Acetobacter estunensis, Acetobacter indonesiensis, Acetobacter lovaniensis, Acetobacter malorum, Acetobacter oeni, Acetobacter orienta*/*is, Acetobacter orleaniensis, Acetobacter pasteurianus, Acetobacter peroxydans, Acetobacter pomorum, Acetobacter suboxydans, Acetobacter roseum, Acetobacter syzygii, Acetobacter tropicalis, Acidomonas methanolica, Asaia bogorensis, Asaia siamensis, Gluconacetobacter azotocaptans, Gluconacetobacter diazotrophicus, Gluconacetobacter europaeus, Gluconacetobacter hansenii, Gluconacetobacter intermedius, Gluconacetobacter johannae, Gluconacetobacter liquefaciens, Gluconacetobacter oboediens, Gluconacetobacter rhaeticus, Gluconacetobacter sacchari, Gluconacetobacter swingsii, Gluconacetobacter xylinus, Gluconobacter oxydans, Gluconobacter oxydans* subsp. *suboxydans,* and preferably *Acetobacter aceti, Acetobacter lovaniensis, Acetobacter pasteurianus, Acetobacter peroxydans, Acetobacter pomorum, Acetobacter suboxydans, Acetobacter syzygii, Acetobacter tropicalis, Gluconacetobacter liquefaciens, Gluconacetobacter xylinus, Gluconobacter oxydans,* and even more preferably *Acetobacter pasteurianus, Acetobacter pomorum, Acetobacter syzygii,* and *Acetobacter tropicalis.*

In another preferred embodiment, the at least one strain of acetic acid bacteria may belong to a species chosen from the group comprising *Acetobacter pasteurianus, Acetobacter syzygii* or *Acetobacter tropicalis.* The inventors surprisingly realised that these three AAB species were the most represented among the isolates taken from cocoa bean and pulp fermentations as described in the Examples section. Accordingly, these species may significantly contribute to the fermentation of cocoa beans and pulp and will be useful for regulating the said fermentation process.

In a further preferred embodiment, the at least one strain of acetic acid bacteria may belong to a species chosen from the group comprising *Acetobacter syzygii* or *Acetobacter tropicalis.* The inventors surprisingly found that these AAB species, which were never before reported to occur in fermentations of cocoa bean and pulp, were unexpectedly considerably represented among the isolates taken from such fermentations as described in the Examples section. Accordingly, these species may, which is surprising, significantly contribute to the fermentation of cocoa beans and pulp and will be particularly useful for regulating the said fermentation process.

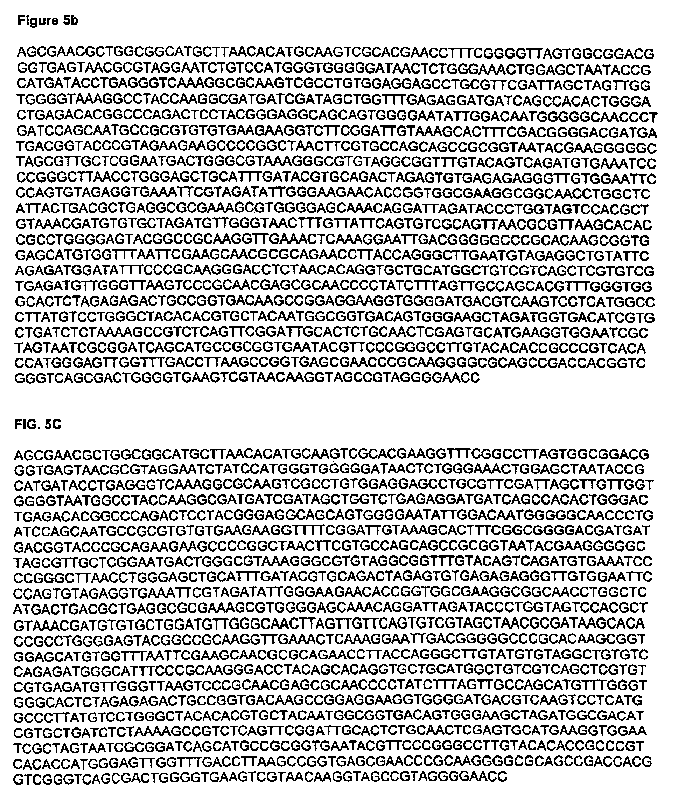

The inventors further surprisingly found among the isolates taken from cocoa bean and pulp fermentations as described in the Examples section several Isolates corresponding to a previously undisclosed taxon related to *Acetobacter syzygii (Acetobacter syzygii* like), with a herein proposed designation *Acetobacter ghanensis.* A representative isolate was deposited on September 7, 2005 under the Budapest Treaty with the Belgian Coordinated Collections of Microorganisms (BCCM) under accession number LMG P-23175. The new taxon according to the present invention has the biological characteristics of the said deposit. Accordingly, the present invention also relates to a method for regulating fermentation of organic material, more particularly plant material, even more particularly cocoa beans and pulp, comprising adding thereto a composition comprising at least the strain *Acetobacter ghanensis* represented by the isolate deposited with BCCM under accession number LMG P-23177, or a mutant or variant thereof. In a related aspect, the present invention relates to a bacterial strain represented by the isolate deposited with BCCM under accession number LMG P-23177 and to mutants or variants thereof. In particular, the said bacterial strain may be preferably substantially isolated from its natural environment. According to the disclosure of this application, persons skilled in the art will be able to discover or prepare a mutant or variant of the said strain. The invention also encompasses an (isolated) acetic acid bacterium showing at least 93% 16S rRNA sequence similarity with the strain *Acetobacter ghanensis* (herein, the 16S rRNA sequence of *Acetobacter ghanensis* refers primarily to this sequence as comprised in the isolate deposited under LMG P-23177 as described above; to the best knowledge of the inventors, the 16S rRNA sequence of this isolate corresponds to SEQ ID NO: 1 shown in Figure 5B; accordingly, in an embodiment, the invention encompasses an acetic acid bacterium showing at least 93% 16S rRNA sequence similarity to the sequence as shown in SEQ ID NO: 1). In further embodiments, such acetic acid bacterium may show at least 93%, e.g., at least 94%, at least 95%, e.g., at least 96%, at least 97%, at least 97.5%. e.g., at least 98%, at least 98.5%, or at least 99% 16S rRNA sequence similarity with the strain *Acetobacter ghanensis.* Further, the invention also relates to the strain *Acetobacter ghanensis,* its mutants and variants, and bacteria having at least 93% 16S rRNA sequence homology to the said strain, when further modified using recombinant DNA techniques.

As will be understood by a skilled person, the above defined novel strain denoted *Acetobacter ghanensis,* its mutants and variants, and the bacteria having at least 93% 16S rRNA sequence similarity to the said strain, and any of the above when further modified by recombinant DNA techniques, is claimed herein for any and all uses, such as by means of example and not limitation, in fermentation of organic materials (e.g., cocoa fermentation), food production (e.g., brewing), and production of pharmaceutical substances. In a preferred embodiment, the present method relates to the use of the acetic acid bacteria *Acetobacter ghanensis* strain as defined herein, for regulating fermentation of plant material essentially consisting of beans and/or pulp derived from fruit pods of the species *Theobroma cacao.*

The inventors further surprisingly found among the isolates taken from cocoa bean and pulp fermentations as described in the Examples section several isolates corresponding to a previously undisclosed taxon related to *Acetobacter tropicalis* (*Acetobacter tropicalis*-like), with a proposed designation *Acetobacter senegalensis.* A representative isolate was deposited on September 7, 2005 under the Budapest Treaty with the Belgian Cooridnated Collections of Microorganisms (BCCM) under accession number LMG P-23176. The new taxon according to the present invention has the biological characteristics of the said deposit. Accordingly, the present invention also relates to a method for regulating fermentation of organic material, more particularly plant material, even more particularly cocoa beans and pulp, comprising adding thereto a composition comprising at least the strain *Acetobacter senegalensis* represented by the isolate deposited with BCCM under accession number LMG P-23176, or a mutant or variant thereof. In a related aspect, the present invention relates to a bacterial strain represented by the isolate deposited with BCCM under accession number LMG P-23176 and to mutants or variants thereof. In particular, the said bacterial strain may be preferably substantially isolated from its natural environment. According to the disclosure of this application, persons skilled in the art will be able to discover or prepare a mutant or variant of the said strain. The invention also encompasses an (isolated) acetic acid bacterium showing at least 93% 16S rRNA sequence similarity with the strain *Acetobacter senegalensis* (herein, the 16S rRNA sequence of *Acetobacter senegalensis* refers primarily to this sequence as comprised in the isolate deposited under accession number DQ887341; to the best knowledge of the inventors, the 16S rRNA sequence of this isolate corresponds to SEQ ID NO: 2 shown in **Figure 5C****;** accordingly, in an embodiment, the invention encompasses an acetic acid bacterium showing at least 93% 16S rRNA sequence similarity to the sequence as shown in SEQ ID NO: 2 further embodiments, such acetic acid bacterium may show at least 93%, e.g., at least 94%, at least 95%, e.g., at least 96%, at least 97%, at least 97.5%, e.g., at least 98%, at least 98.5%, or at least 99% 16S rRNA sequence similarity with the strain *Acetobacter senegalensis.* Further, the invention also relates to the strain *Acetobacter senegalensis,* its mutants and variants, and bacteria having at least 93% 16S rRNA sequence homology to the said strain, when further modified using recombinant DNA techniques.

As will be understood by a skilled person, the above defined novel strain denoted *Acetobacter senegalensis,* its mutants and variants, and the bacteria having at least 93% 16S rRNA sequence similarity to the said strain, and any of the above when further modified by recombinant DNA techniques, is claimed herein for any and all uses, such as by means of example and not limitation, In fermentation of organic materials (e.g., cocoa fermentation), food production (e.g., brewing), and production of pharmaceutical substances. In a preferred embodiment, the present method relates to the use of the acetic acid bacteria *Acetobacter senegalensis* strain as defined herein, for regulating fermentation of plant material essentially consisting of beans and/or pulp derived from fruit pods of the species *Theobroma cacao.*

In another preferred embodiment, the at least one strain of acetic acid bacteria may belong to a species chosen from the group comprising *Acetobacter pasteurianus, Acetobacter ghanensis* or *Acetobacter senegalensis,* and preferably comprise *Acetobacter ghanensis* and/or *Acetobacter senegalensis.*

The inventors also discovered that cocoa fermentations were usually characterised by a gradual development of AAB present from at the start of the fermentation to reach maximum populations typically after 60-80 h of fermentation. This was followed by a slight decrease and/or stabilising of AAB upon prolonged fermentation. AAB develop much more slowly compared to LAB, so their lag phase is much longer. Addition of AAB to the plant material is therefore to be done earlier than the corresponding fermentation stage. Accordingly, in a preferred embodiment of the method for regulating fermentation of organic material, more particularly plant material, even more particularly cocoa beans and pulp, comprises adding thereto a composition comprising at least one strain of acetic acid bacteria at the start and/or earlier stages of the fermentation, e.g., at between 0 and 150h, 0 and 140h, 0 and 130h, 0 and 120h, 0 and 100h, 0 and 90h, 0 and 80h, 0 and 70h, 0 and 60h, 0 and 50h, 0 and 48h, 0 and 40h, 0 and 30h, 0 and 24h, 0 and 20h, 0 and 10h, 0 and 5h, or 0 and 1 h or at 0h after the start of fermentation.

In addition, the inventors surprisingly discovered that *A. tropicalis* was more abundant in the early stages of the fermentation, but was replaced by *A. syzygii* later during the fermentation. It is therefore believed that *A. tropicalis* may play a major role at the start and/or in the earlier stages of the fermentation, while *A. syzygii* may become more important in the later stages and/or towards the end of the fermentation. Accordingly, a preferred embodiment of the method for regulating fermentation of organic material, more particularly plant material, even more particularly cocoa beans and pulp, comprises adding thereto a composition comprising at least one strain of *A. tropicalis* at the start and/or earlier stages of the fermentation, e.g., at between 0 and 150h, 0 and 140h, 0 and 130h, 0 and 120h, 0 and 100h, 0 and 90h, 0 and 80h, 0 and 70h, 0 and 60h, 0 and 50h, 0 and 48h, 0 and 40h, 0 and 30h, 0 and 24h 0 and 20h, 0 and 10h, 0 and 5h, or 0 and 1h or at 0h after the start of fermentation.

In a further embodiment of the method for regulating fermentation of organic material, more particularly plant material, even more particularly cocoa beans and pulp, comprises adding thereto a composition comprising at least one strain of *A. senegalensis* at the start and/or earlier stages of the fermentation, e.g., at between 0 and 150h, 0 and 140h, 0 and 130h, 0 and 120h, 0 and 100h, 0 and 90h, 0 and 80h, 0 and 70h, 0 and 60h, 0 and 50h, 0 and 48h, 0 and 40h, 0 and 30h, 0 and 24h, 0 and 20h, 0 and 10h, 0 and 5h, or 0 and 1h or at 0h after the start of fermentation.

In one embodiment, the present method for regulating fermentation of cocoa beans and pulp may comprise adding at the start of the fermentation a composition comprising at least one strain of acetic acid bacteria selected from the genera of the *Acetobacter* or *Gluconobacter* or *Gluconacetobacter,* and/or any combination thereof.

In another embodiment, the present method for regulating fermentation of cocoa beans and pulp may comprise adding at the start and/or during the first 24 hours, and preferably during the first 1, 5. 7, 10, 12, 15, 18, 20, and 22 hours of the fermentation a composition which comprises at least one strain of acetic acid bacteria selected from the genera of the *Acetobacter* or *Gluconobacter* or *Gluconacetobacter,* and/or any combination thereof. Preferably the method comprises adding at the start and/or during the first 24 hours, and preferably during the first 1, 5, 7, 10, 12, 15, 18, 20, and 22 hours of the fermentation a composition which comprises one or more strains of acetic acid bacteria belonging to a species chosen from the group comprising *A. pasteurianus, A. tropicalis, A. syzygii, A. senegalensis,* and *A. ghanensis,* and more preferably belonging to a species chosen from the group comprising *A. pasteurianus, A. tropicalis* and *A. senegalensis.*

In another preferred embodiment, the present method for regulating fermentation of cocoa beans and pulp may comprise adding after 24 hours, and for instance after 30, 35, 40, 45, 48, 50, 55, 60, 65, 70, 72 hours of the fermentation, of the fermentation a composition which comprises at least one strain of acetic acid bacteria selected from the genera of the *Acetobacter* or *Gluconobacter* or *Gluconacetobacter,* and/or any combination thereof. Preferably the method comprises adding after 24 hours and for instance after 30, 35, 40, 45, 48, 50, 55, 60, 65, 70, 72 hours of the fermentation, a composition which comprises one or more strains of acetic acid bacteria belonging to a species chosen from the group comprising *A. pasteurianus, A. syzygii, and A. ghanensis,* and more preferably *A. pasteurianus* and *A. ghanensis.*

In another preferred embodiment the method comprises adding thereto a composition comprising at least one strain of *A. syzygii* at later stages of the fermentation, e.g., at between 0 and 150h, 10 and 150h, 20 and 150h, 24 and 150h, 30 and 150h, 40 and 150h, 48 and 150h, 50 and 150h, 60 and 150h, 70 and 150h, 80 and 150h, 90 and 150h, 100 and 150h, 110 and 150h, 120 and 150h, 130 and 150h, 140 and 150h, or at 150h after the start of fermentation.

In yet another embodiment the method for regulating fermentation of organic material, more particularly plant material, even more particularly cocoa beans and pulp, comprises adding thereto a composition comprising at least one strain of *A. ghanensis* at later stages of the fermentation, e.g., at between 0 and 150h, 10 and 150h, 20 and 150h, 24 and 150h, 30 and 150h, 40 and 150h, 48 and 150h, 50 and 150h, 60 and 150h, 70 and 150h, 80 and 150h, 90 and 150h, 100 and 150h, 110 and 150h, 120 and 150h, 130 and 150h, 140 and 150h, or at 150h after the start of fermentation.

In addition, optimal conditions for the growth of acetic acid bacteria may be at a pH between 5.4 and 6.3. Accordingly, in an embodiment, the method comprises adding thereto a composition comprising at least one strain of acetic acid bacteria when the pH of the fermented material is between 4.0 and 7.5, preferably between 4.5 and 7.0, more preferably between 5.0 and 6.5 and most preferably between 5.4 and 6.3. In a further embodiment, pH may be adjusted by the addition of appropriate amounts of an alkali or an acid according to well-known procedures.

In addition, acetic acid bacteria grow optimally starting at a temperature of about 30°C. Accordingly, in an embodiment, the method comprises adding thereto a composition comprising at least one strain of acetic acid bacteria when the temperature of the fermented material is more than 30°C, more than 32°C, more than 35°C, more than 38°C, more than 40°C, more than 42°C, more than 45°C, or more than 47°C. In an embodiment, the temperature may be adjusted according to well-known procedures.

A skilled person will readily appreciate that in the above described methods, the expression "composition comprising at least one strain of lactic acid bacteria and/or acetic acid bacteria" encompasses composition comprising one or more LAB strains and (substantially) no AAB strain, as well as compositions comprising one or more AAB strains and (substantially) no LAB strain, as well as compositions comprising both one or more LAB strains and one or more AAB strains. A skilled person will further appreciate that the above described methods may comprise methods in which one or more than one composition comprising the same or different LAB and/or AAB strain(s) is/are added to the fermented material at one or more times, e.g., at the start and/or during the fermentation process. By means of example and not limitation, the same compositions can be added at different times or different compositions may be added at the same or different times.

In a preferred embodiment the invention relates to a method for regulating fermentation of cocoa beans and pulp comprising adding at the start of the fermentation a composition A wherein said composition A comprises:
- at least one strain of lactic acid bacteria selected from the group comprising *Lactobacillus plantarum, Lactobacillus fermentum. Leuconostoc pseudomesenteroides, Enterococcus casseliflavus, Weissella ghanensis, Enterococcus faecium, Lactobacillus brevis, Lactobacillus mali,* and *Leuconostoc mesenteroides,* and preferably comprising *Lactobacillus plantarum, Lactobacillus fermentum, Leuconostoc pseudomesenteroides, Enterococcus casseliflavus,* and *Weissella ghanensis,* and/or
- at least one strain of acetic acid bacteria selected from the group comprising *A. aceti, A. pasteurianus, A. tropicalis, A. syzygii, A. senegalensis,* and *A. ghanensis,* and preferably comprising *A. pasteurianus, A. tropicalis, A. syzygii, A. senegalensis,* and *A. ghanensis..*

In another preferred embodiment the invention relates to a method for regulating fermentation of cocoa beans and pulp comprising adding at the start and/or during the first 24 hours, and for instance during the first 1, 5, 7, 10, 12, 15, 18, 20, 22 or 24 hours of the fermentation a composition B wherein said composition B comprises:
- at least one strain of lactic acid bacteria selected from the group comprising *Lactobacillus plantarum, Lactobacillus fermentum; Leuconostoc pseudomesenteroides, Enterococcus casseliflavus* and *Weissella ghanensis, Enterococcus faecium; Lactobacillus brevis; Lactobacillus mali; Leuconostoc mesenteroides* and preferably comprising *Lactobacillus plantarum, Lactobacillus fermentum; Leuconostoc pseudomesenteroides* and *Enterococcus casseliflavus* and/or
- at least one strain of acetic acid bacteria selected from the group comprising *A. aceti, A. pasteurianus, A. tropicalis, A. syzygii, A. senegalensis,* and *A. ghanensis,* and preferably comprising *A. pasteurianus, A. tropicalis,* and *A. senegalensis.*

In another preferred embodiment the invention relates to a method for regulating fermentation of cocoa beans and pulp comprising adding after 24 hours, and for instance after 30, 35, 40, 45, 48, 50, 55, 60, 65, 70, 72 hours of the fermentation, a composition C wherein said composition C comprises:
- at least one strain of lactic acid bacteria selected from the group comprising *Lactobacillus plantarum, Lactobacillus fermentum; Leuconostoc pseudomesenteroides, Enterococcus casseliflavus* and *Weissella ghanensis* and preferably a *Lactobacillus fermentum* strain, and/or
- at least one strain of acetic acid bacteria selected from the group comprising *A. pasteurianus, A. syzygii, and A. ghanensis* and preferably comprising *A. pasteurianus* and *A. ghanensis* strain.

Further, yeast also play an important role in the succession of microbial species during the fermentation of cocoa beans and pulp and will be useful for regulating the said fermentation.

Accordingly, in an embodiment of the present method the composition comprising at least one strain of LAB and/or AAB further comprises at least one strain of yeast (i.e., one strain of a yeast species). As used herein, the term "yeast" refers to a group of single-celled fungi, most of which are in the class Ascomycetes, and others in the class Basidiomycetes.

In an embodiment, the at least one strain of yeast may belong to a species of a genus chosen from the group comprising *Brettanomyces, Candida, Cryptococcus, Debaryomyces, Geotrichum, Hanseniaspora, Hansenula, lssatchenkia, Kloeckera, Kluyveromyces, Lodderomyces, Pichia, Rhodotorula, Saccharomyces, Saccharomycopsis, Schizosaccharomyces, Scytaladium, Torulaspora, Torulopsis* or *Trichosporon, and preferably Candida, Cryptococcus, Hanseniaspora, Hansenula, Kluyveromyces, Pichia,* or *Saccharomyces,* and more preferably *Candida, Hanseniaspora, Kluyveromyces,* or *Pichia.* Further examples of yeast genera may include but are not limited to *Arxiozyma, Citeromyces, Dekkera, Holleya, Kodameae, Satumispora, Starmera, Tetrapisispora, Williopsis* or *Zygosaccharomyces.*

In a further embodiment, the at least one strain of yeast may belong to a species chosen from the group comprising *Candida bombi, Candida pelliculosa, Candida rugopelliculosa, Candida rugosa, Candida intermedia, Candida krusei, Candida parapsilosis, Candida quercitrusa, Candida silvicola, Candida stellimalicola, Candida boidinii, Candida cacoai, Candida guilliermondii, Candida reukaufii, Candida amapae, Candida diddensiae, Candida friedrichii, Candida humicola, Candida mycoderma, Candida neodendra, Candida tammaniensis, Candida tropicalis, Candida valida, Candida zemplinina, Geotrichum candidum, Kloeckera apiculata, Kloeckera africana, Kloeckera corticis, Kloeckera javanica, Kluyveromyces marxianus, Kluyveromyces thermotolerans, Lodderomyces elongisporus, Pichia fermentans, Saccharomyces cerevisiae, Saccharomyces cerevisiae* var. *chevalieri, Torulaspora pretoriensis, Cryptococcus humicola, Cryptococcus laurentii, Hanseniaspora guilliermondii, Hanseniaspora opuntiae, Pichia galeiformis, Pichia guilliermondii, Pichia kluyveri, Pichia membranifaciens, Rhodotorula glutinis, Rhodotorula rubra, Trichosporon asahii, Brettanomyces clausenii, Kloeckera apis, Schizosaccharomyces malidevorans, lssatchenkia hanoiensis, Saccharomycopsis vini* or *Scytaladium hyalinum,* and preferably *Candida bombi, Candida pelliculosa, Candida rugosa, Candida intermedia, Candida krusei, Candida cacoai, Candida guilliermondii, Pichia fermentans, Pichia guilliermondii, Pichia membranifaciens, Hanseniaspora guilliermondii, Hanseniaspora opuntiae, Saccharomyces cerevisiae, Kluyveromyces marxianus,* or *Kluyveromyces thermotolerans,* and more preferably *Candida krusei, Pichia membranifaciens, Hanseniaspora guilliermondii,* or *Kluyveromyces marxianus.* A skilled person will know which species are more likely for pectinolysis (e.g. *Saccharomyces cerevisiae, S*. *cerevisiae* var. chevalieri, *Kluyveromyces marxianus,* and *Candida norvegensis*) and to produce ethanol (e.g. *Saccharomyces* cerevisiae, *S*. *cerevisiae* var. *chevalieri,* and *Kluyveromyces marxianus*)*.* Also, a skilled person will know which species are more often found in box fermentations (e.g., *Saccharomyces cerevisiae* and *Candida zemplinina*) and which species are more often found in heap fermentations (e.g., *Hanseniaspora guilliermondii, Pichia membranifaciens* and *Candida krusei*) and may be, e.g., employed accordingly.

Typically, the above methods of the invention may relate to regulating of fermentation processes, which apart from the said regulating, are spontaneous. A "spontaneous" fermentation or "natural fermentation" or fermentation process as used herein is one that employs microorganisms naturally present in and/or unconsciously introduced into the fermented organic material at the start or during the fermentation. By means of example and not limitation, in spontaneous fermentation of cocoa beans and pulp, microorganisms may be introduced after the beans and the pulp are released from the pods from natural microbiota present, for example, on workers' hands, tools (knifes, shovels, unwashed baskets, etc.) and in places of previous fermentations. Accordingly, in the above methods an otherwise spontaneous fermentation may be regulated by addition of a composition comprising at least one LAB and/or AAB strain, and optionally further comprising at least one yeast strain, to organic material e.g., cocoa beans and pulp. Hereby, the microbial presence in the materials is altered and the fermentation is thereby regulated (manipulated or modulated). The microbial strains introduced by means of the said compositions may be the same or similar (e.g., of the same species and/or genus) to those naturally found in the organic material and/or may be different (e.g., of a different species and/or genus).

In an alternative embodiment, the methods may relate to regulating fermentation which would not be initiated without said regulating. In a typical example, this may occur when natural (indigenous) microbiota are removed or suppressed from the organic material prior to adding the said composition comprising at least one strain of LAB and/or AAB, and optionally further comprising at least one strain of yeast. By means of example and not limitation, the natural microbiota present in the cocoa fermentation may be largely removed by de-pulping the recovered beans by various methods of the art, e.g., as known from GB2241146.

In an embodiment of the present invention, the fermentation of the plant material comprising beans derived from *Theobroma cacao* and/or plant material consisting, preferably essentially, of the beans and pulp derived from *Theobroma* cacao (collectively "cocoa beans and pulp") may be carried out in heaps, boxes (e.g., wooden or steel boxes), baskets, trays, and other means, such as generally used in the art. In a preferred embodiment, the said fermentation may be carried out in heaps or boxes, more preferably heaps.

In general, the compositions of the present invention may be "defined" compositions. The notion of defined compositions is well-known to a skilled microbiologist and in the present invention generally refers to compositions in which the microbial component consists essentially of those strain or strains of lactic acid bacteria and/or acetic acid bacteria, and optionally yeast, that have been introduced intentionally into such compositions, e.g., by operator. Defined compositions do not substantially contain microorganisms, such as bacteria and/or yeast, which have not been intentionally introduced thereto by the operator(s) and/or of the presence of which in the composition the operator(s) is (are) unaware. Such latter microorganisms may represent contamination which may typically constitute less than 20%, e.g., less than 15%, less than 10%, e.g., less than 5%, less than 3%, e.g., less than 2%, or less than 1%, e.g., less than 0.5%, or less than 0.1%, e.g., less than 0.01%, or even less of the microbial component of such composition, or even be entirely absent. Accordingly, the compositions described here below may typically be defined compositions.

In an embodiment, the composition of the invention comprising at least one strain of LAB and/or AAB, and further optionally comprising at least one strain of yeast, may be a starter culture. The term "culture" refers to any sample or specimen which is known to contain or suspected of containing one or more microorganisms. The term as used herein also encompasses starter culture and co-culture.

The term "starter culture" refers to a composition comprising live microorganisms that are capable of initiating or effecting fermentation of organic material, optionally after being cultivated in a separate starter medium for obtaining a high density culture. Accordingly, in an embodiment, the composition of the invention comprising at least one strain of LAB and/or AAB, and further optionally comprising at least one strain of yeast, may be a high density culture obtained by propagating a starter culture in a suitable medium.

A starter culture may be, e.g., a liquid culture, liquid pressed culture, frozen or dried form, including, e.g., freeze dried form and spray/fluid bed dried form, or frozen or freeze-dried concentrated. The culture may be packed in vacuum, or under an atmosphere of, e.g., N2, CO2 and the like. For example, a starter culture may be produced and distributed in sealed enclosures, preferably non-pyrogenic, which can be made of a rigid, non-flexible or flexible suitable plastic or other material, to the fermentation place and may be either added to organic material to be fermented, or optionally first cultivated in a separate starter medium to obtain a high density culture.

A starter culture may also contain, in addition to the microorganisms, buffering agents and growth stimulating nutrients (e.g., an assimilable carbohydrate or a nitrogen source), or preservatives (e.g., cryoprotective compounds) or other carriers, if desired, such as milk powder or sugars.

A starter culture may be a pure culture, i.e., may contain a biomass of one single isolate of LAB and/or AAB according to the invention, i.e. a clone originating in principle from one cell. In another embodiment, a starter culture may be a co-culture, i.e., may comprise more than one strain of LAB and/or AAB, and optionally yeast, of the invention.

It may be preferred that a starter culture or a high density culture contains at least 10² colony forming units (CFU) of one or more bacterial strains (and optionally of one or more yeast strains) of the invention, such as at least 10³ CFU/g, at least 10⁴ CFU/g, e.g., at least 10⁵ CFU/g, at least 10⁶ CFU/g, e.g., at least 10⁷ CFU/g, at least 10⁸ CFU/g, e.g., at least 10⁹ CFU/g, at least 10¹⁰ CFU/g, e.g., at least 10¹¹ CFU/g, at least 10¹² CFU/g, or at least 10¹³ CFU/g.

Typically, a starter culture or a high density culture may be added to a starter medium or to organic material to be fermented in a concentration of viable cells of one or more bacterial strains (and optionally of one or more yeast strains) which is at least 10² (CFU) of one or more bacterial strains (and optionally of one or more yeast strains) of the invention, such as at least 10³ CFU/g, at least 10⁴ CFU/g, e.g., at least 10⁵ CFU/g, at least 10⁸ CFU/g, e.g., at least 10⁷ CFU/g, at least 10⁸ CFU/g, e.g., at least 10⁹ CFU/g, at least 10¹⁰ CFU/g, e.g., at least 10¹¹ CFU/g, at least 10¹² CFU/g, or at least 10¹³ CFU/g of the organic material.

For example, starter culture or high density culture may be added to a starter medium or to organic material to be fermented in a concentration of viable cells of one or more bacterial strains (and optionally of one or more yeast strains) in the range of 10³ to 10¹³ CFU/g of the material, such as 10⁵ to 10⁹ CFU/g of the material, 10⁸ to 10⁸ CFU/g of the material, or 10⁷ to 10⁸ CFU/g of the material.

In a further aspect, the invention provides a composition comprising at least one strain of lactic acid bacteria and/or acetic acid bacteria, and optionally further comprising at least one strain of yeast, as defined in the above embodiments. In an embodiment, said composition may be a starter culture or a high density culture.

In a further aspect, the invention provides for use of a composition comprising at least one strain of lactic acid bacteria and/or at least one strain of acetic acid bacteria, and optionally further comprising at least one strain of yeast, as defined in the any of the above embodiments (the said composition may be a starter culture or a high density culture), for regulating fermentation of organic material, preferably plant material, more preferably cocoa beans and pulp.

In yet another embodiment, the LAB applied in the present invention are acid-tolerant. In yet another embodiment, the LAB applied in the present invention are ethanol-tolerant. In another embodiment, the AAB applied in the present invention are AAB which are acid-tolerant. In yet another embodiment, the AAB applied in the present invention are ethanol-tolerant. In another embodiment, the AAB applied in the present invention are thermoresistant. As used herein "acid-tolerant" bacteria are defined as bacteria of which the metabolic activity is not affected when the bacteria are grown or maintained at pH conditions which are lower than or equal to pH 4. As used herein "ethanol-tolerant" bacteria are defined as bacteria of which the metabolic activity is not affected when the bacteria are grown or maintained at an ethanol concentration equal to or higher than 5 % (vol/vol). As used herein "thermoresistant" bacteria are defined as bacteria of which the metabolic activity is not affected when the bacteria are grown or maintained at temperature equal to or higher than 35°C.

In one embodiment, the invention provides a composition, preferably a composition A, suitable for being added at the start of the fermentation, comprising:
- at least one strain of lactic acid bacteria selected from the group comprising *Lactobacillus plantarum, Lactobacillus fermentum, Leuconostoc pseudomesenteroides, Enterococcus casseliflavus, Weissella ghanensis, Enterococcus faecium, Lactobacillus brevis, Lactobacillus mali, Leuconostoc mesenteroides,* and preferably comprising *Lactobacillus plantarum, Lactobacillus fermentum,* Leuconostoc *pseudomesenteroides,* Enterococcus *casseliflavus,* and *Weissella ghanensis,* and/or
- at least one strain of acetic acid bacteria selected from the group comprising *A. aceti, A. pasteurianus, A. tropicalis, A. syzygii, A. senegalensis,* and *A. ghanensis,* and preferably comprising A. *pasteurianus, A. tropicalis, A. syzygii, A. senegalensis,* and *A. ghanensis.*

In another embodiment, the invention provides a composition, preferably a composition B, suitable for being added at the start and/or during the ffirst 24 hours, and e.g. during the first 1, 5, 7, 10, 12, 15, 18, 20, 22 or 24 hours of the fermentation, comprising:
- at least one strain of lactic acid bacteria selected from the group comprising *Lactobacillus plantarum, Lactobacillus fermentum; Leuconostoc pseudomesenteroides, Enterococcus casseliflavus* and *Weissella ghanensis, Enterococcus faecium; Lactobacillus brevis ; Lactobacillus mali ;* and *Leuconostoc mesenteroides* and preferably comprising *Lactobacillus plantarum, Lactobacillus fermentum; Leuconostoc pseudomesenteroides* and *Enterococcus casseliflavus* and/or
- at least one strain of acetic acid bacteria selected from the group comprising *A. aceti, A. pasteurianus, A. tropicalis, A. syzygii, A. senegalensis,* and *A. ghanensis,* and preferably comprising *A. pasteurianus, A. tropicalis,* and *A. senegalensis.*

In yet another embodiment, the invention provides a composition, preferably a composition C, suitable for being added after 24 hours, and for instance after 30, 35, 40, 45, 48, 50, 55, 60, 65, 70, 72 hours of the fermentation comprising:
- at least one strain of lactic acid bacteria selected from the group comprising *Lactobacillus plantarum, Lactobacillus fermentum; Leuconostoc pseudomesenteroides, Enterococcus casseliflavus* and *Weissella ghanensis* and preferably a *Lactobacillus fermentum* strain, and/or
- at least one strain of acetic acid bacteria selected from the group comprising A. *pasteurianus, A. syzygii, and A. ghanensis* and preferably comprising *A. pasteurinaus* and *A. ghanensis* strain.

**Table 1** provides further non-limitative examples of compositions which are suitable for use in the present invention.

**Table 1**

| **N°** | **LAB** | **AAB** | **Yeast** |
|---|---|---|---|
| 1 | *Lb.plantarum* | *A. pasteurianus; A. syzygii; A. tropicalis* | - |
| 2 | *Lb. fermentum* | *A. pasteurianus; A. syzygii; A. tropicalis* | - |
| 3 | *Leuc. pseudomesenteroides* | *A. pasteurianus; A. syzygii; A. tropicalis* | - |
| 4 | *E.casseliflavus* | *A. pasteurianus; A. syzygii; A. tropicalis* | - |
| 5 | *W. ghanensis* | *A. pasteurianus; A. syzygii; A. tropicalis* | - |
| 6 | *Lb.plantarum* | - | |
| 7 | *Lb.plantarum* | *A.pasteurianus* | |
| 8 | *Lb.plantarum* | *A. pasteurianus and A. syzygii* | |
| 9 | *Lb. plantarum* | *A. pasteurianus and A. tropicalis* | |
| 10 | *Lb.plantarum* | *A. pasteurianus and A. senegalensis* | |
| 11 | *Lb.plantarum* | *A. pasteurianus and A. ghanensis* | |
| 12 | *Lb.plantarum* | *A. syzygii* | |
| 13 | *Lb.plantarum* | *A. tropicalis* | |
| 14 | *Lb.plantarum* | *A. senegalensis* | |
| 15 | *Lb.plentarum* | *A. ghanensis* | |
| 16 | *Lb.plantarum* | *A. syzygii and A. tropicalis* | |
| 17 | *Lb.plantarum* | *A. senegalensis and A. ghanensls* | |
| 18 | *Lb.plantarum* | *A. senegalensis and A. tropicalis* | |
| 19 | *Lb.plantarum* | *A. ghanensis and A. syzygii* | |
| 20 | *Lb.plantarum* | *A. syzygii and A. tropicalis and A. senegalensis and A. tropicalis* | |
| 21 | *Lb. fermentum* | - | - |
| 22 | *Lb. fermentum* | *A. pasteurianus* | - |
| 23 | *Lb. fermentum* | *A. pasteurianus and A. syzygii* | - |
| 24 | *Lb. fermentum* | *A. pasteurianus and A. tropicalis* | - |
| 25 | *Lb. fermentum* | *A. pasteurianus and A. senegalensis* | - |
| 26 | *Lb. fermentum* | *A. pasteurianus and A. ghanensis* | - |
| 27 | *Lb. fermentum* | *A. syzygii* | - |
| 28 | *Lb. fermentum* | *A. tropicalis* | - |
| 29 | *Lb. fermentum* | *A. senegalensis* | - |
| 30 | *Lb. fermentum* | *A. ghanensis* | |
| 31 | *Lb. fermentum* | *A. syzygii and A. tropicalis* | |
| 32 | *Lb. fermentum* | *A. senegalensis and A. ghanensis* | |
| 33 | *Lb. fermentum* | *A. senegalensis and A. tropicalis* | |
| 34 | *Lb. fermentum* | *A. ghanensis and A. syzygii* | |
| 35 | *Lb. fermentum* | *A. syzygii and A. tropicalis and A. senegalensis and A. tropicalis* | |
| 36 | *Lb.plantarum* | *A. pasteurianus; A. ghanensis, A.senegalensis* | - |
| 37 | *Lb. fermentum* | *A. pasteurianus; A. ghanensis, A.senegalensis* | - |
| 38 | *Leuc. pseudomesenteroides* | *A. pasteurianus; A. ghanensis, A.senegalensis* | - |
| 39 | *E.casseliflavus* | *A. pasteurianus; A. ghanensis, A.senegalensis* | - |
| 40 | *W. ghanensis* | *A. pasteurianus; A. ghanensis, A.senegalensis* | - |
| 41 | *Lb.plantarum ; Lb. fermentum ; Leuc. pseudomesenteroides ; E.casseliflavus* | *A. pasteurianus; A. syzygii; A. tropicalis ;* | - |
| 42 | *Lb.plantarum; Lb. fermentum ; Leuc. pseudomesenteroides ; E.casseliflavus* | *A. pasteurianus; A. ghanensis, A. senegalensis* | - |
| 43 | *Lb.plantarum ; Lb. fermentum ; Leuc. pseudomesenteroides ; E.casseliflavus* | *A. pasteurianus; A. syzygii; A. tropicalis,* A. *ghanensis,* A. *senegalensis* | *Candida krusei, Pichia membranifaciens, Hanseniaspora guilliermondii, Kluyveromyces marxianus Saccharomyces cerevisiae* |
| 44 | *Lb.plantarum ;Leuc. pseudomesenteroides ; E. casseliflavus* | *A. pasteurianus, A. syzygii: A. tropicalis* | - |
| 45 | *Lb.plantarum; Lb. fermentum ; Leuc. pseudomesenteroides ; E.casseliflavus; W. ghanensis* | *A. pasteurianus; A. syzygii; A. tropicalis* | - |
| 46 | *Lb.plantarum ; Lb. fermentum ; Leuc.* | *A. pasteurianus; A. syzygii; A. tropicalis ; A. ghanensis, A.* | - |
| | *pseudomesenteroides ; E.casseliflavus; W. ghanensis* | *senegalensis* | |
| 47 | *Lb.plantarum; Lb. fermentum ; Leuc. pseudomesenteroides ; E.casseliflavus; W. ghanensis* | *A. pasteurianus; A. syzygii; A. tropicalis* | *Candida krusei, Pichia membranifaciens, Hanseniaspora guilliermondii, Kluyveromyces marxianus Saccharomyces cerevisiaθ* |
| 48 | *Lb.plantarum ; Lb. fermentum ; Leuc. pseudomesenteroides ; E.casseliflavus; W. ghanensis* | *A. pasteurianus; A. syzygii; A. tropicalis, A. ghanensis, A. senegalensis* | *Candida krusei, Pichia membranifaciens, Hanseniaspora guilliermondii, Kluyveromyces marxianus Saccharomyces cerevisiae* |
| 49 | *Lb.plantarum ; Lb. fermentum ; Lb. brevis ; Lb. mali* | *A. pasteurianus; A. syzygii; A. tropicalis* | - |
| 50 | *Lb.plantarum ; Lb. fermentum ; E. faecium ; Leuc. mesenteroides* | *A, pasteurianus; A. syzygii; A. tropicalis* | - |
| 51 | *Lb.plantarum ; Lb. fermentum ; Lb. brevis ; Lb. mali* | *A. pasteurianus; A. ghanensis, A. senegalensis* | |
| 52 | *Lb.plantarum ; Lb. fermentum : E. faecium ; Leuc. mesenteroides* | *A. pasteurianus; A. ghanensis, A. senegalensis* | - |
| 53 | *Leuc. pseudomesenteroides: E.casseliflavus; Lb. brevis ; Lb. mali* | *A. pasteurianus; A. syzygii; A. tropicalis ; A. ghanensis ; A. senegalensis* | - |
| 54 | *Lb. plantarum; Leuc. pseudomesenteroides: E.casseliflavus; Lb. brevis ; Lb. mali* | *A. pasteurianus; A. syzygii; A. tropicalis ; A. ghanensis; A. senegalensis* | - |
| 55 | *Leuc. pseudomesenteroides. E.casseliflavus; E. faecium ; Leuc. mesenteroides* | *A. pasteurianus; A. syzygii; A. tropicalis ; A. ghanensis; A. senegalensis* | - |
| 56 | *Lb.plantarum ; Leuc.* | *A. pasteurianus; A. syzygii; A.* | - |
| | *pseudomesenteroides: E.casse*/*inavus; E. faecium ; Leuc. mesenteroides* | *tropicalis ; A. ghanensis; A. senegalensis* | |
| 57 | *Lb.plantarum ; Lb. fermentum; E. faecium ; Leuc. mesenteroides ; Lb. brevis ; Lb. mali* | *A. pasteurianus; A. syzygii; A. tropicalis; A. ghanensis, A. senegalensis* | - |
| 58 | *Lb.plantarum ; Lb. fermentum* | *A. pasteurianus; A. syzygii; A. tropicalis ;* | *Saccharomyces cerevisiae* |
| 59 | *Lb.plantarum; Lb. fermentum* | *A. pasteurianus; A. syzygii; A. tropicalis ;* | *Candida krusei* |
| 60 | *Lb.plantarum ; Lb. fermentum* | *A. pasteurianus; A. ghanensis; A. senegalensis* | *Saccharomyces cerevisiae* |
| 61 | *Lb.plantarum ; Lb. fermentum* | *A. pasteurianus; A. ghanensis; A. senegalensis* | *Candida krusei* |
| 62 | *Lb.plantarum ; Lb. fermentum* | *A. pasteurianus; A. ghanensis;* | *Saccheromyces cerevisiae* |
| 63 | *Lb.plantarum ; Lb. fermentum* | *A. pasteurianus; A. senegalensis* | *Saccharomyces cerevisiae* |
| 64 | *Lb.plantarum ; Lb. fermentum* | *A. pasteurianus; A. ghanensis; A. senegalensis,* | - |
| 65 | *Lb.plantarum ; Lb. fermentum* | *A. pasteurianus; A. ghanensis:* | - |
| 66 | *Lb.plantarum ; Lb. fermentum* | *A. pasteurianus; A. senegalensis* | - |
| 67 | *Lb. plantarum ; Lb. fermentum* | *A. pasteurianus* | *Saccharomyces cerevisiae* |
| 68 | *Lb.plantarum ; Lb. fermentum* | *A. pasteurianus* | *-* |
| 69 | *Lb.plantarum ; Lb. fermentum* | *A. pasteurianus* | *Candida krusei* |
| 70 | *Lb.plantarum ; Lb. fermentum; Leuc. pseudomesenteroides; E.casseliflavus* | *A. pasteurianus; A. ghanensis; A. senegalensis,* | *Saccharomyces cerevisiae* |
| 71 | *Lb.plantarum ; Lb. fermentum; Leuc. pseudomesenteroides ; E.casseliflavus* | *A. pasteurianus; A. syzygii; A. tropicalis ;* | *Hanseniaspora guilliermondii* |

It will be clear according to the present invention that the above given compositions may be one or more times applied at the start and/or at particular stages of the fermentation process, e.g., at between 0 and 150h, 0 and 140h, 0 and 130h, 0 and 120h, 0 and 100h, 0 and 90h, 0 and 80h, 0 and 70h, 0 and 60h, 0 and 50h, 0 and 48h, 0 and 40h, 0 and 30h, 0 and 24h, 0 and 20h, 0 and 10h. 0 and 5h, 0 and 1h, at 0h or at between 10 and 150h, 20 and 150h, 24 and 150h, 30 and 150h, 40 and 150h, 48 and 150h, 50 and 150h, 60 and 150h, 70 and 150h, 80 and 150h, 90 and 150h, 100 and 150h, 110 and 150h, 120 and 150h, 130 and 150h, 140 and 150h, or at 150h after the start of fermentation. Different of the exemplified compositions can be added at the same or at different times.

In accordance with the present invention, use of any of the compositions as defined herein during fermentation of cocoa beans has effects on the physical properties (e.g. appearance), organoleptic properties (e.g. flavour) and (bio)chemical composition of the fermented cocoa beans.

In one embodiment, use of the present compositions enable to obtain a higher number of fermented cocoa beans which have good qualitative properties compared to hitherto known processes, e.g. up to 10% and preferably up to 20% more fermented cocoa beans which have good qualitative properties, e.g. which are not infected and/or broken, and have a satisfying colour and appearance. Qualitative properties of beans can be evaluated using standard methods such as appearance and cut tests (see example section below).

In another embodiment, use of the present compositions enable to obtain fermented cocoa beans having optimal levels of (bio)chemical components such as but not limited organic molecules, including lactic acid, acetic acid, citric acid, alcohols including ethanol, sugar alcohols including mannitol and erythritol, esters, polyphenols, alkaloids (theobromine, caffeine) etc...or lower or no levels of mycotoxins.

In an example, use of the present compositions enables to obtain fermented cocoa beans having amounts of lactic acid which are lower than 0.8% and preferably lower than 0.3% (weight % of dry beans). In another example, use of the present compositions enables to obtain fermented cocoa beans having amounts of acetic acid which are lower than 1% and preferably lower than 0.5% (weight % of dry beans). In another example, use of the present compositions enables to obtain fermented cocoa beans having amounts of citric acid which are lower than 0.8% and preferably lower than 0.3% (weight % of dry beans). In another example, use of the present compositions enable to obtain fermented cocoa beans having amounts of oxalic acid which are lower than 0.8% and preferably lower than 0.3% (weight % of dry beans). In yet another example, use of the present compositions enables to obtain fermented cocoa beans having amounts of ethanol which are lower than 0.5% and preferably lower than 0.1% (weight % of dry beans).

In still another example, use of the present compositions enables to obtain fermented cocoa beans having amounts of flavanols comprised between 10and 100mg/g (of dry beans). In another example, use of the present compositions enables to obtain fermented beans having amounts of polyphenols comprised between 5 and 150mg GAE/g (of dry beans). In another example, use of the present compositions enables to obtain fermented cocoa beans having amounts of caffeine comprised between 0.01 and 0.5 weight % (of dry beans). In yet another example, use of the present compositions enable to obtain fermented cocoa beans having amounts of theobromine comprised between 0.1 and 4 weight % (of dry beans). In another example, use of the present compositions enables to obtain fermented cocoa beans having amounts of catechin comprised between 0.1 and 10 mg/g (of dry beans). In yet another example, use of the present compositions enables to obtain fermented cocoa beans having amounts of epicatechin comprised between 1 and 30 mg/g (of dry beans).

The present fermentation process also enables to obtain an optimal balance between nonvolatile substances, such as for instance lactic acid, and volatile substances, such as for instance acetic acid. Such optimal balance advantageously permits to reduce the time needed for the conching process, when preparing chocolate from the cocoa beans fermented according to the present invention. Conching is the process whereby the refined mixture of ingredients for preparing the chocolate is filled in a container where it is undergoing a thermal and mechanical treatment by stirring and homogenisation. After the process is complete, the chocolate mass is stored in tanks heated to approximately 45-50 °C until final processing. In a preferred embodiment, in acccordance with the present invention, when using cocoa which has been fermented in acccordance with the present invention for preparing chocolate, the conching process can be considerably reduced, and can for instance be up to 10%, 20%, 30% or even 40% faster compared to hitherto known processes.

The present invention also provides fermented organic material obtainable by the herein before described method of the invention, preferably fermented plant material, more preferably plant material essentially consisting of beans and/or pulp derived from fruit pods of the species *Theobroma cacao*, and more preferably fermented cocoa beans.

In another aspect, the invention provides a method of preparing chocolate and cocoa products comprising the use of fermented cocoa beans produced as hereinbefore described in accordance with the invention. Typically, following methods well-known in the art, the fermented cocoa beans are dried, roasted, cracked, winnowed to remove shells and to produce the nibs, which are ground to give cocoa liquor which may be used to prepare chocolate, as known to a skilled person, or may be pressed to extract cocoa butter and the residual cake pulverised, cooled and sifted to give cocoa powder.

In a further aspect, the invention also provides chocolate (e.g., chocolate bars) and cocoa products prepared from the cocoa beans fermented as hereinbefore described in accordance with the invention.

In one embodiment, use of cocoa beans fermented in accordance with the present invention for preparing chocolate (e.g., chocolate bars) and cocoa products permits to obtain chocolate and cocoa products having good taste properties and optimal amounts of several bio(chemical) components including but not limited to organic molecules, such as lactic acid, acetic acid, citric acid; alcohols including ethanol; sugar alcohols; esters; polyphenols; alkaloids (theobromine, caffeine); etc... (see above).

The present invention is further illustrated by the following deposits of biological material:
The isolate deposited on September 7, 2005 under the Budapest Treaty with the Belgian Coordinated Collections of Microorganisms (BCCM) under accession number LMG P-23179 is representative of the bacterial species newly described herein as *Weissella ghanensis.*
The isolate deposited on September 7 2005 under the Budapest Treaty with BCCM under accession number LMG P-23177 is representative of the bacterial species newly described herein as *Acetobacter ghanensis.*
The isolate deposited on September 7, 2005 under the Budapest Treaty with BCCM under accession number LMG P-23175 is representative of the isolates/strains identified in this invention and attributed to the species *Acetobacter syzygii*
The isolate deposited on September 7, 2005 under the Budapest Treaty with BCCM under accession number LMG P-23176 is representative of the isolates/strains identified in this invention and attributed to the species *Acetobacter senegalensis.*
The isolate deposited on September 7, 2005 under the Budapest Treaty with BCCM under accession number LMG P-23178 is representative of the isolates/strains identified in this invention and attributed to the species *Leuconostoc pseudomesenteroides.*
The isolate deposited on September 7, 2005 under the Budapest Treaty with BCCM under accession number LMG P-23180 is representative of the isolates/strains identified in this invention and attributed to the species *Enterococcus casseliflavus.*

The invention, now being generally described, will be more readily understood by reference to the following examples, which are included merely for purposes of illustration of certain aspects and embodiments of the present invention and are not intended to limit the invention.

### DESCRIPTION OF FIGURES

**Figure 1A** **and** **1B** illustrate (upper graph) population dynamics of yeast, total bacteria, LAB, AAB and Lb. plantarum/enterococci and (lower graph) the ambient temperature, temperature of the fermenting beans in a heap, and rain fall in an exemplary spontaneous, natural cocoa bean heap fermentation. (A) Heap 1, farmer 1, mid-crop, (B) Heap 4, farmer 1, main-crop.
**Figure 2A** **and** **2B** illustrate PCR-DGGE analysis of AAB using WBAC primers. (A) Heap 1, farmer 1, mid-crop, M = marker, H1 = Heap 1, S = Sample (0 = 0 h, 1 = 6 h, 2 = 12 h, 3 = 18 h, 4=24h, 5=30h, 6=36h, 7=42h, 8=48h, 9=54h, 10=60h, 11 =66h, 12 = 72 h, 13 = 84 h, 14 = 96 h, 15 = 120 h, 16 = 144 h) (B) Heap 4, farmer 1, main-crop (0 = 0 h, 1 = 6 h, 2 = 12 h, 3 = 18 h, 4 = 24 h, 5 = 30 h, 6 = 36 h, 7 = 42 h, 8 = 48 h, 9 = 54 h, 10 = 60 h, 11 = 66 h, 12 = 72 h, 13 = 84 h, 14 = 96 h, 15 = 120 h, 16 = 144 h).
**Figure 3A and B** illustrates PCR- DGGE analysis of LAB using LAC primers. (A) Heap 1, farmer 1, mid-crop, M = marker, H1 = Heap 1, S = Sample (0 = 0 h, 1 = 6 h, 2 = 12 h, 3 = 18 h, 4 = 24 h , 5 = 30 h , 6 = 36 h , 7 = 42 h , 8 = 48 h , 9 = 54 h , 10 = 60 h, 11 = 66 h, 12 = 72 h, 13 = 84 h, 14 = 96 h, 15 = 120 h, 16 = 144 h); (B) Heap 4, farmer 1, main-crop, M = marker, H4 = Heap 4, S = Sample (0 = 0 h, 1 = 6h , 2 = 12 h , 3 = 18 h , 4 = 24 h , 5 = 30 h , 6 = 36 h, 7 = 42 h, 8 = 48 h, 9 = 54 h, 10 = 60 h, 11 = 66h, 12 = 72h, 13 = 84 h, 14 = 96 h, 15 = 120h, 16 = 144 h)
**Figure 4** illustrates the clustering analysis of the isolates identified as AAB
**Figure 5A** illustrates the clustering analysis of representative isolates based on 16S rRNA sequence
**Figure 5B** illustrates the 16S rRNA sequence *of A. ghanensis* (SEQ ID NO: 1)
**Figure 5C** illustrates the 16S rRNA sequence of *A. senegalensis* (SEQ ID NO: 2).
**Figure 6** illustrates the clustering analysis of the isolates identified as LAB.
**Figure 7A-E** illustrates the analysis of fermented cocoa beans with respect to several metabolites. Heap 1, farmer 1, mid-crop.
Figure 8 illustrates a dendrogram based on the numerical analysis of generated, digitized (GTG)₅-PCR fingerprints from fermented cocoa bean isolates identified as lactic acid bacteria. These banding patterns were clustered together with reference strains, using the unweighted pair-group method using arithmetic averages (UPGMA) with correlation levels expressed as percentage values of the Pearson correlation coefficient. Species validation of representative strains of each cluster included SDS-PAGE (indicated with †) and/or 16S rRNA sequence analysis (indicated with ‡).
**Figure 9** illustrates a dendrogram based on the numerical analysis of generated, digitized (GTG)₅-PCR fingerprints from fermented cocoa bean isolates identified as acetic acid bacteria. These banding patterns were clustered together with reference strains, using the unweighted pair-group method using arithmetic averages (UPGMA) with correlation levels expressed as percentage values of the Pearson correlation coefficient. Species validation of representative strains of each cluster included 16S rRNA sequence analysis (indicated with ‡) and/or DNA:DNA hybridizations (indicated with*).

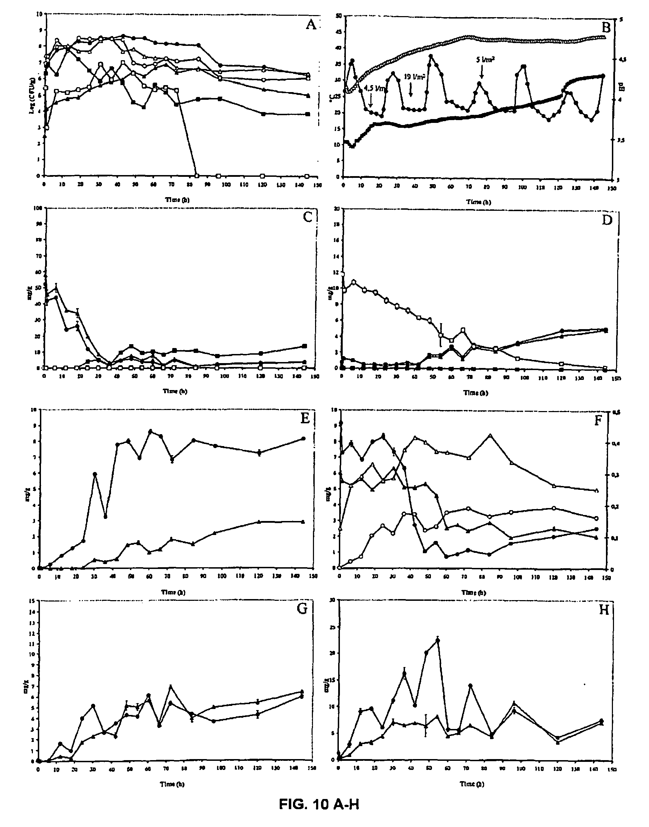

**Figure 10A-H** shows the course of spontaneous Ghanaian cocoa bean heap fermentation (heap 5). (A) Microbial succession of yeasts (MEA, ■), LAB (MRS, ●; M17, O; KAA, □), AAB (DMS, ▲), and total aerobic bacteria (PCA, Δ). (B) Temperature inside (Δ) and outside (●) the heap, and pH (■) inside the heap. Measurements of rainfall are indicated with arrows. (C). Course of residual glucose (●), fructose (▲), and sucrose (□), and of produced mannitol (■) in the pulp. (D). Course of residual glucose (●), fructose (▲), and sucrose (□), and of produced mannitol (■) in the beans. (E). Course of lactic acid produced in the pulp (●) and in the beans (▲). (F). Course of residual citric acid (full symbols, left axis) and of produced succinic acid (open symbols, right axis) in the pulp (●,O) and in the beans (▲,Δ). (G). Course of acetic acid produced in the pulp (●) and in the beans (A). (H). Course of ethanol produced in the pulp (●) and in the beans (▲).
**Figure 11** illustrates a comparison of different media for isolation of lactic acid bacteria from fermented cocoa bean samples. The media are as described herein. The dilutions where the different species were picked from are not shown.

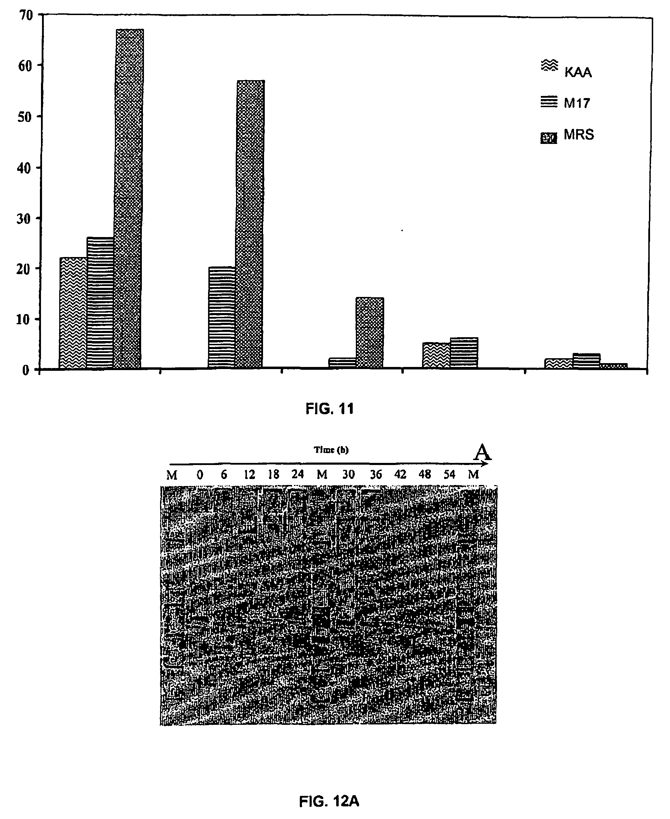

**Figure 12A-C** illustrates PCR-DGGE profiles (35-60% denaturant gradient) of LAB representing 16S rRNA gene fragments of heap 2 (A) and heap 5 (B), sampled during a 6-day cocoa bean fermentation (fermentation samples from 0-54 h and 0-84 h are shown for heaps 2 and 5, respectively), and of bulk cells of the population of LAB isolated using MRS agar after 6 and 12 h of fermentation (C); all 16S rRNA fragments were amplified by the LAC primer pair. (a) *L. plantarum* LMG 6907^{T}; (b) *L. acidophilus* LMG 9433^{T}; (c) *L. fermentum* LMG 6902^{T}; (d) *Leuc. mesenteroides* subsp. *mesenteroides* LMG 6893^{T}; (e) *P. acidilactici* LMG 11384^{T}; (f) *L. casei* LMG 6904^{T}. The closest relatives of the fragments sequenced (percentages identical nucleotides as compared to sequences retrieved from the GenBank database are represented between brackets) were as follows: *L. fermentum* (i, 100%), *Leuc. pseudomesentroides* (ii, 99.7%), *L. plantarum* (iii, 100%), and *'W. ghanensis'* (iv, 100% identity with the 16S rRNA of our isolate). Purification and sequencing of faint bands (v) did not prove successful.
**Figure 13A-B** are illustrations of cluster analyses of the PCR-DGGE profiles. Dendrograms were based on the Dice coefficient of similarity (weighted) and obtained with the UPGMA clustering algorithm. Samples are indicated by fermentation heap, sample number, and farmer. Identified clusters are indicated by numerals to the right of each panel. (A) Bacterial shift in cocoa bean heap fermentation 4 at farmer 1. (B) Bacterial shift in and effect of the heap on fermentations 4 and 5 at farmers 1 and 2, respectively.
**Figure 14** illustrates the amounts of acid metabolites including lactic acid, acetic acid and citric acid present in fermented, dried beans obtained in different heap fermenting processes (heap 1 to 7).
**Figure 15** illustrates the amounts of polyphenols present in fermented, dried beans obtained in different heap fermenting processes (heap 1 to 7, and 10-13).
**Figure 16A-G** further illustrate the amounts of caffeine and theobromine present in fermented, dried beans obtained in different heap fermenting processes (heap 1 to 7).
**Figure 17** provides a visual representation of the results of a flavor test performed by a test panel on different chocolates prepared from cocoa beans fermented according to the present invention (heap 1 to 7).

### EXAMPLES

### Example 1: Dynamics and biodiversity of lactic acid bacteria and acetic acid bacteria populations involved in spontaneous heap fermentation of cocoa beans

### 1. Experiment 1

### Population dynamics

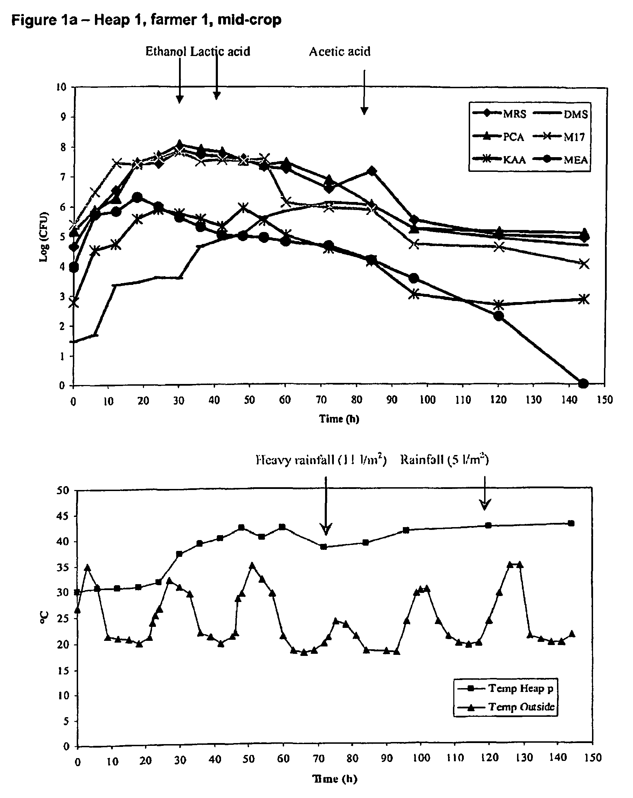

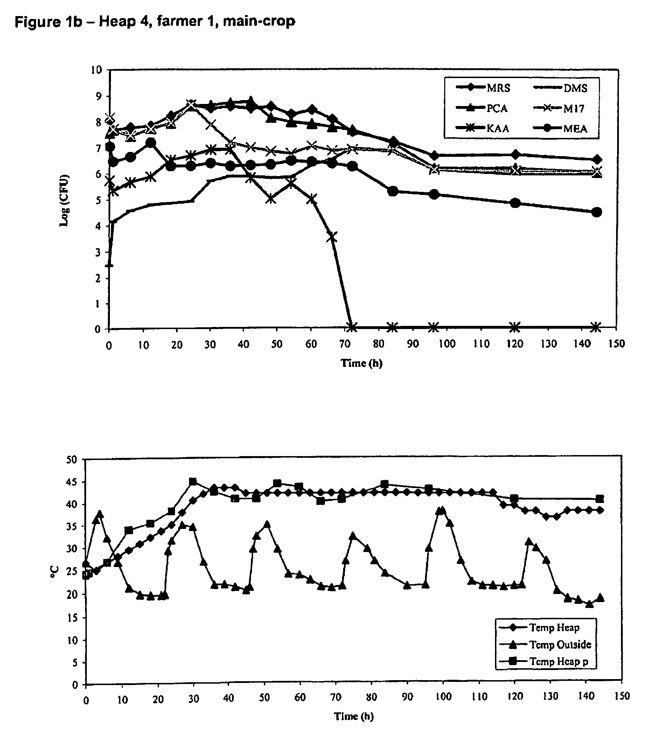

The population dynamics of spontaneous, natural cocoa bean fermentations were studied in the field: seven heap fermentations at two different locations (location 1: fermentations 1, 3, 4 and 6: location 2: fermentations 2, 5 and 7) during mid-crop June-July 2004 (fermentations 1, 2 and 3) and main crop October-November 2004 (fermentations 4, 5, 6 and 7), six days each, followed by drying during 10-14 days depending on the weather, both by cultivation-based enumeration and culture-independent methods. Also, ambient temperature and temperature of the fermenting beans in the heap as well as pH and rainfall were monitored on line. An example of this is shown in **Figure 1** (A, heap 1, farmer 1, mid-crop and B, heap 4, farmer 1, main-crop).

Cultivation media used were: Plate Count Agar (PCA) for the total bacterial count (37°C, 1-4 days); Malt Extract Agar (MEA) with 100 mg/l of oxytetracycline for yeasts (37°C, 1-4 days); Deoxycholate-Mannitol-Sorbitol (DMS) agar with 400 mg/l of cycloheximide for AAB (42°C, 1-4 days); de Man-Rogosa-Sharpe (MRS) medium and Medium 17 of Terzaghi & Sandine (M17) with 400 mg/l of cycloheximide for LAB (37°C, 1-4 days); Kanamycine Aesculin Azide (KAA) agar with 400 mg/l of cycloheximide for enterococci (37°C, 1-4 days).

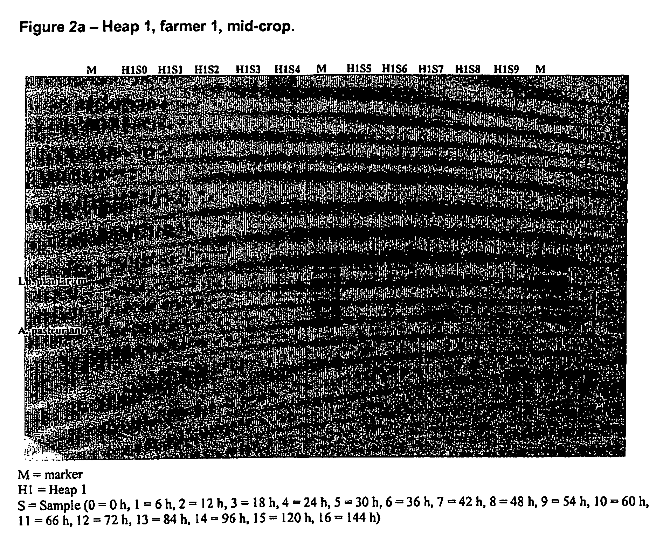

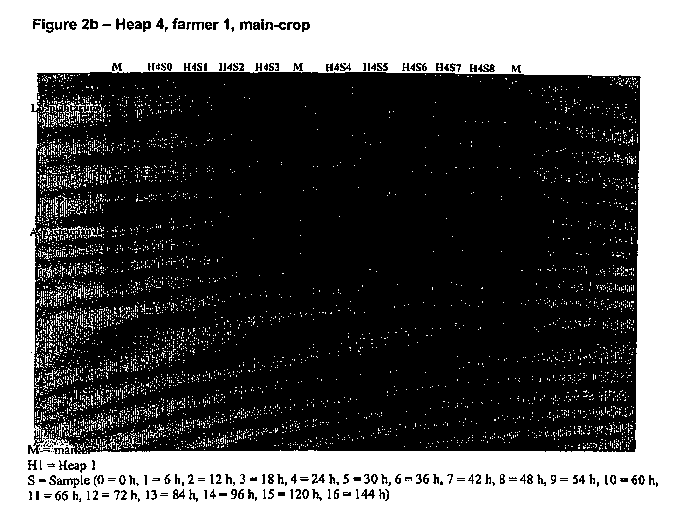

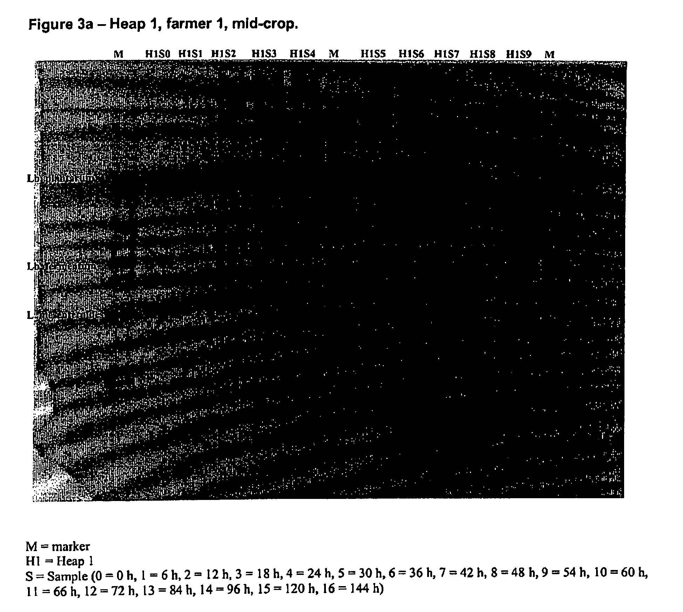

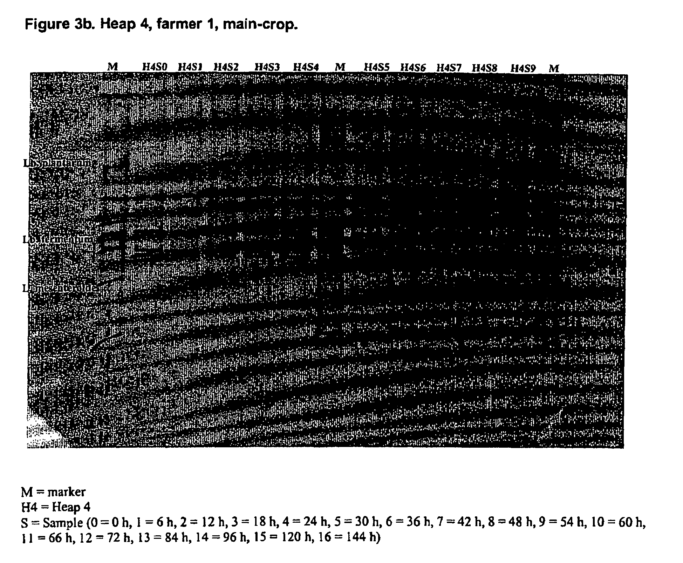

For DGGE (Denaturing Gradient Gel Electrophoresis), total DNA was extracted from fermentation samples, and the following primers were used ― for AAB: the WBAC primers of Lopez et al. (Design and Evaluation of PCR Primers for Analysis of Bacterial Populations In Wine by Denaturing Gradient Gel Electrophoresis. Applied and Environmental Microbiology 69: 6801-6807, 2003), an example of this analysis is shown in **Figures 2A** **and** **2B****;** for LAB: the LAC primers of Walter et al. (Detection of Lactobacillus, Pediococcus, Leuconostoc, and Weissella species in human feces by using group-specific PCR primers and denaturing gradient gel electrophoresis. Applied and Environmental Microbiology 67: 2578-2585, 2001), an example of this analysis is shown in **Figure 3A** **and** **3B****.**

Population dynamics, based on cultivation-based enumerations, revealed the following. Yeasts (10⁴-10⁵ Colony Forming Units or CFU/g) were present in the bean mass at the commencement of the fermentation, and grew to maximum populations of 10⁶-10⁷ CFU/g during the subsequent 20-26 h. Thereafter, the yeast population declined and after 6 days most of the yeasts were absent. There is a simultaneous development of yeast and AAB, the latter being slower developing, followed by a decrease of the yeast and less of the AAB (sometimes stabilizing) upon prolonged fermentation. AAB grew slower and reached lower maximum population densities (10⁶ CFU/g after 66 h of fermentation). The experiments done during the mid- and main-crop gave comparable results. There was a higher initial number and low increase of yeast as compared to AAB during the main-crop fermentations, the latter being slower developing than yeasts, but also slower as compared with the fermentations of the mid-crop expedition. The most dominant species (see below) present in the beginning of the cocoa fermentation were *Acetobacter pasteurianus* and *Acetobacter tropicalis.* Later in the fermentation *A. tropicalis* was replaced by *Acetobacter syzygii. A. pasteurianus* survived longer throughout the fermentations, because these strains are more resistant to heat than other AAB. Due to the growth of AAB, the oxidation of ethanol to acetic acid started. This oxidation process was responsible for the increase in temperature inside the heap. The temperature increased from 30°C at the start of the fermentation to a maximum temperature of 47°C. This temperature was reached when the population of AAB had reached its maximum.

The cocoa fermentations were characterized by a high level of LAB present at the start of the fermentation. LAB grew during fermentation to maximum populations of 10⁷-10⁸ CFU/g after 30 h of fermentation during the mid-crop fermentations. This was followed by a slight decrease of LAB (sometimes stabilizing) upon prolonged fermentation. The experiments done during the mid- and main-crop gave comparable results. There was a higher initial number and low increase of LAB during the fermentations of the main crop, the maximum populations reached being 10⁸-10⁸ CFU/g after 40 h of fermentation. The most dominant species (see below) found in all fermentations were *Lactobacillus fermentum* and *Lactobacillus plantarum group* (the latter comprising one or more of *Lactobacillus plantarum, Lactobacillus paraplantarum* and/or *Lactobacillus pentosus),* especially *Lb. plantarum.* These were the abundant species in the first 40 h of fermentation. *Leuconostoc pseudomesenteroides* and *Enterococcus casseliflavus* were found in the beginning of almost every fermentation.

The cultivation-independent DGGE analyses revealed that *Lb. plantarum* and *Lb. fermentum* were the most dominant species throughout fermentation. Moreover, *Lb. fermentum* increased while *Lb. plantarum* decreased as a function of time. Apparently, in the beginning of the fermentation, also *Leuconostoc pseudomesenteroides* was present, but disappeared rather rapidly. Concerning AAB, *A. pasteurinaus* dominated the fermentation process. All this is in accordance with the population dynamics mentioned above.

### Biodiversity

The biodiversity of seven spontaneous, natural heap fermentations of Ghanaian cocoa beans was studied. Isolates derived from the agar media, used for the cultivation-based enumeration above, were picked up and purified. In total, 910 isolates were collected. Preliminary identification of these isolates consisted of colony and cell morphology, mobility, Gram stain, catalase activity, and oxidase activity. Cellular fatty acids from a subset of isolates (63 strains) were identified using the Microbial Identification system of MIDI (Netwark, Delaware, USA) to evaluate the selectivity of the DEA medium for AAB. Also, the production of lactic acid (HPLC), acetic acid (HPLC and HPAEC-conductivity with ion suppression), ethanol (HPLC), gluconic acid (HPAEC-conducGvity with ion suppression), 2-keto gluconic acid (HPAEC-conductivity with ion suppression), and 5-keto gluconic acid (HPAEC-conductivity with ion suppression) was assessed for all isolated strains. It turned out that all isolates belonged to either the LAB (catalase-negative, oxidase-negative; production of lactic acid, acetic acid, and/or ethanol) or AAB (catalase-positive, oxidase-negative; possession of the cell envelop fatty acids C18:1 w7c and C16:0; production of acetic acid, gluconic acid, 2-keto gluconic acid, and/or 5-keto gluconic acid) group. Noteworthy to notice was the selective isolation of AAB on DEA and of *Lb. plantarum* on KAA under the conditions used.

De-replication and identification of all isolates were performed by:
for AAB: DNA isolation from pure cultures followed by (GTG)5-rep-PCR, and 16S rDNA sequence analysis of representatives of the different clusters obtained after numerical analysis of the rep-PCR patterns, and/or DNA-DNA hybridisations;
for LAB: DNA isolation from pure cultures followed by (GTG)5-rep-PCR, and SDS-PAGE of whole cell proteins and/or 16S rDNA sequence analysis of representatives of the different clusters obtained after numerical analysis of the rep-PCR patterns.

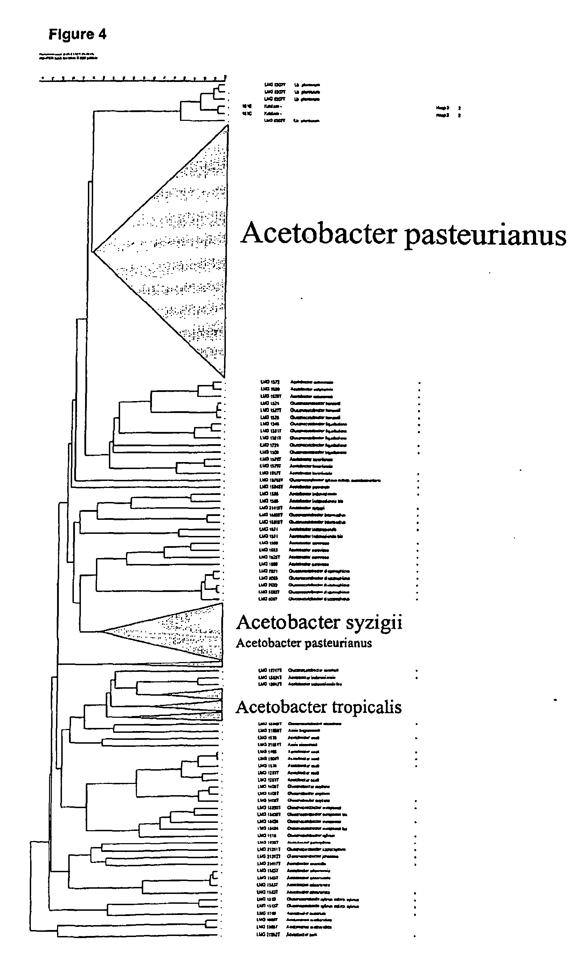

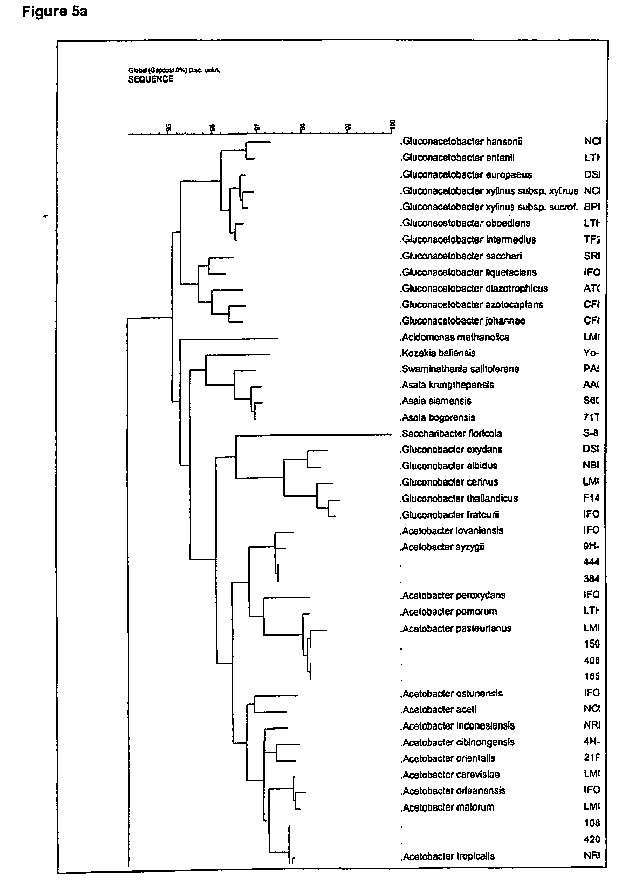

AAB dereplication and identification revealed three main clusters among the isolates (Figure 4): *Acetobacter pasteurianus* (99 strains), *Acetobacter syzygii*(*-Iike*) (23 strains), Acetobacter *tropicalis* (10 strains). As mentioned above, *A. pasteurianus* seemed to play a major role in the cocoa bean fermentation process. This species was found in cocoa bean fermentations before. *A. syzygii* and *A. tropicalis* were not found in cocoa fermentations previously and their finding in the present application is surprising. Moreover, several of the strains supposed to be *A. syzygii* found here could be discriminated from the reference strain of *A. syzigii* based on 16S rDNA sequence analysis and DNA-DNA hybridisation, revealing a new species, referred to as *A. syzygii-like* (herein, the present inventors propose a new denotation: *A. ghanensis*) **(****Figure 5A****).** A representative isolate of this taxon was deposited under accession number LMG P-23175 as detailed elsewhere in the application. **Figure 5B** shows the presumed 16S rRNA sequence of this new species as determined experimentally by the inventors. Similarly, several of the strains supposed to be *A. fropicalis* found here could be discriminated from the reference strain of *A. tropicalis* based on 16S rDNA sequence analysis and DNA-DNA hybridisation, revealing two new species, referred to as *A. tropicalis-like* (herein, the inventors could allocate one species to the newly proposed species *A. senegalensis*)*.* A representative isolate of this taxon was deposited under accession number LMG P-23176 as detailed elsewhere in the application. (16S rRNA sequence, SEQ ID NO:2; see **FIG. 5C**).

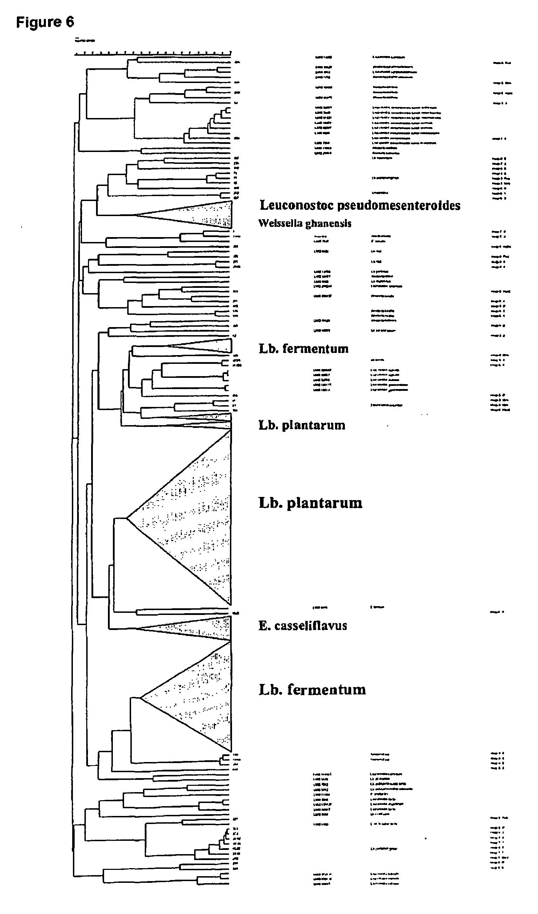

LAB dereplication and identification revealed four main clusters among the isolates (**Figure *6***)*: Lactobacillus plantarum* (117 strains), *Lactobacillus fermentum* (77 strains), *Leuconostoc pseudomesenteroides* (16 strains), *Enterococcus casseliflavus* (13 strains), and Weissella species (few strains). As mentioned above, *Lb. plantarum* and *Lb. fermentum* seem to play a major role in the cocoa bean fermentation process. Both species were found in cocoa bean fermentations before. However, as mentioned above through DGGE, we revealed here their evolution during the fermentation process and indicated that *Lb. plantarum* plays a major role at the start of the fermentation, followed by *Lb. fermentum* towards the end of the fermentation, or throughout the whole fermentation. We also found new taxa related to *Weissella;* herein, the inventors allocated one taxon to a new species with the denotation *W*. *ghanensis',* and deposited a representative isolate thereof with the accession number LMG P-23179.

### Metabolomics

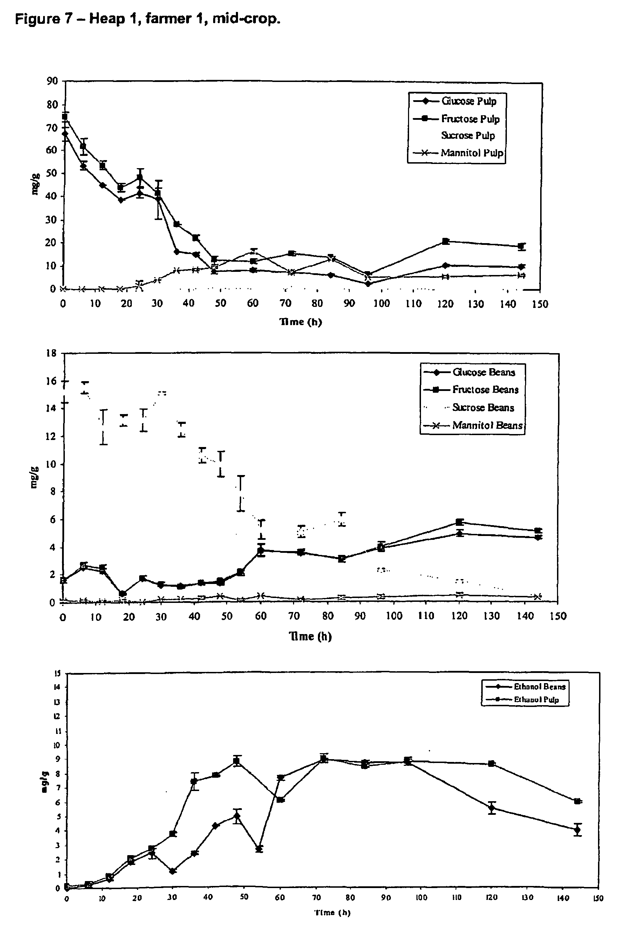

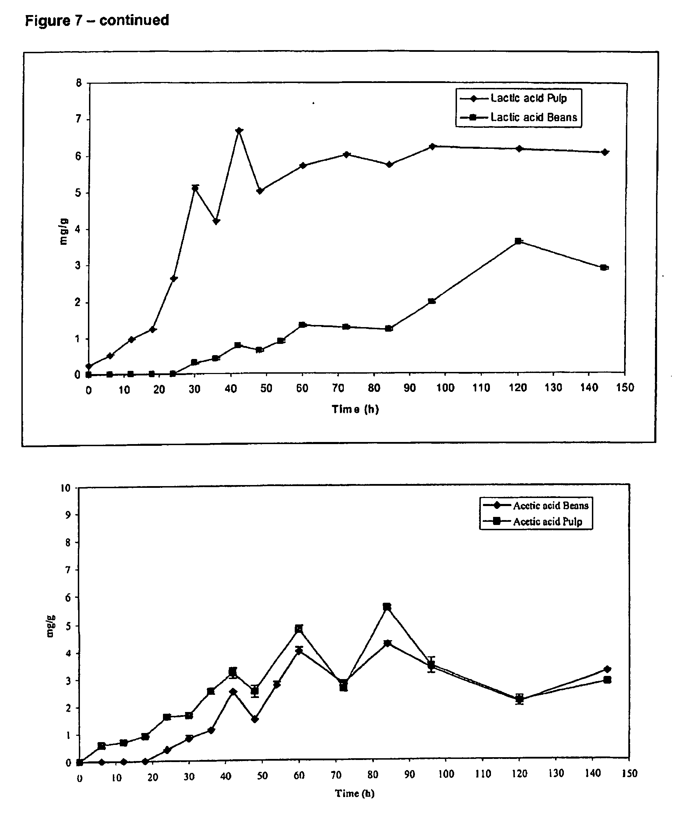

Fermented cocoa bean samples (both pulp and depulped beans) during fermentation were analysed with respect to the following metabolites: sugars and mannitol (HPAEC-PAD); organic acids (HPAEC-conductivity with ion suppression); short-chain fatty acids (GC/MS); ethanol (GC/MS); amino acids (LC/MS); sugar and amino acid derivatives as aroma precursors (both GC/MS and LC/MS); succinic acid (LC/MS); polyphenols (DPPH colour reaction, only depulped beans); theobromine and caffeine (HPLC, only depulped beans). For an example, see **Figure 7** (heap 1, farmer 1, mid-crop).

The amount of pulp sugars at the start of the fermentation depends on many factors such as the maturation of the pods, post-harvest pod age, etc. Almost all sucrose in the pulp was gone at the start of the fermentations, most probably due to yeast growth. Fructose and glucose disappeared simultaneously in the pulp, whereas mannitol increased upon time (most probably linked with the increase of the *Lb. fermentum* population). Sucrose was hydrolysed into glucose and fructose in the beans, probably as a result of endogenous enzymatic action and/or acid hydrolysis due to acetic acid penetration upon fermentation. Ethanol increased upon fermentation, almost simultaneously in pulp and beans, and reached a maximum after around 30 h of fermentation. Ethanol was oxidised into acetic acid, which reached a maximum after about 90 h of fermentation, again almost simultaneously in pulp and beans. Whereas the amounts of ethanol and acetic acid were typically slightly higher in the pulp than in the beans, the amount of lactic acid was considerably higher in the pulp than in the beans. Lactic acid reached a maximum in the pulp after about 40 h of fermentation.

### Quality analyses

The following quality analyses were performed: appearance of beans, cut test (index of fermentation), and sensorial analysis. The quality of beans was according to the Ghanaian standards.

### Chocolate manufacture

From each heap, chocolate was produced after roasting of the beans and conching of the cocoa mass. The following analyses were performed: roasted beans (sensorial analysis, theobromine, caffeine, polyphenols); after conching: mass, start and end-product; chocolate: taste (trained panel), color, rheology, crystallisation behaviour, theobromine, caffeine, polyphenols.

For further details, see Example 2.

### 2. Experiment 2

The following example illustrates a study of a Ghanaian cocoa bean heap fermentation process by a multiphasic approach, encompassing both microbiological and metabolite target analyses. A culture-dependent (plating and incubation, followed by rep-PCR analyses of randomly picked up colonies) and culture-independent (denaturing gradient gel electrophoresis of 16S rRNA amplicons, PCR-DGGE) method revealed a limited biodiversity and targeted population dynamics of both lactic acid bacteria (LAB) and acetic acid bacteria (AAB) during Ghanaian cocoa bean heap fermentation. Four main clusters were found among the LAB identified: *Lactobacillus plantarum, Lactobacillus fermentum, Leuconostoc pseudomesenteroides,* and *Enterococcus casselliflavus.* Other taxa encompassed for instance *Weissella.* Only four clusters were found among the AAB identified: *Acetobacter pasteurianus, Acetobacter syzygii*-like*,* and two small clusters of *A.* tropicalis-like. Particular strains of *L. plantarum, L. fermentum,* and *A. pasteurianus,* derived from the environment, were well adapted to the environmental conditions prevailing under Ghanaian cocoa bean heap fermentation, and apparently have a significant role in the cocoa bean fermentation process. Both yeasts and LAB fermented sugars; yeasts produced ethanol from sugars and LAB produced lactic acid, acetic acid, ethanol, and mannitol from sugars and/or citrate. Whereas *L. plantarum* strains were abundant in the beginning of the fermentation, *L. fermentum* strains converted fructose into mannitol upon prolonged fermentation. *A. pasteurianus* grew on mannitol and lactate and converted ethanol into acetic acid. A newly proposed *Weissella* sp., referred to as *'W ghanensis',* was detected through PCR-DGGE analysis in some of the fermentations and was only occasionally picked up through culturebased isolation. New *Acetobacter* spp. were found as well, namely the newly proposed species '*A. senegalensis'* (*A. tropicalis*-like) and an *A. syzygii*-like species.

The aim of the present experiment was to assess the population dynamics of LAB and AAB, in comparison with yeast, and the evolution of important fermentation parameters (temperature, pH, sugars, and metabolites), during spontaneous heap fermentations of cocoa beans in Ghana. Both culture-dependent and -independent methods were applied to monitor and identify LAB and AAB. Cluster analyses of the rep-PCR and bacterial DGGE profiles were performed to reveal differences between the fermentation processes. Through metabolite target analysis a link was made between the microbes identified and the molecules found in pulp and beans.

### MATERIALS AND METHODS

**Cocoa bean fermentation.** Two field experiments were set up to Ghana to sample spontaneous cocoa bean fermentations (heap method), one during the mid-crop (June-July, 2004; heaps 1, 2, and 3) and one during the main crop (October-November 2004; heaps 4, 5, 6, and 7), representing the two major harvest seasons. As traditional cocoa bean fermentation processes are supposed to be region- and site-specific, two small farms (A and B), located about 15 km from each other near New Tafo and Old Tafo, respectively, were chosen. During the mid-crop, fermentations at farm A were followed twice (heaps 1 and 3) and those at farm B once (heap 2), while during the main-crop fermentations at both farm A (heaps 4 and 6) and B (heaps 5 and 7) were followed twice.

Cocoa pods from mixed hybrid cocoa tree plantations (Criollo and Forastero) were harvested by traditional methods (such as manual harvest and transport in unwashed baskets) and used for fermentation within two to three days. Only matured pods were used for fermentation. Plantation workers cut the pods with unwashed machetes and beans plus surrounding pulp were scooped out manually; the placenta was not removed according to local practices and the husks were left to rot in the surroundings. At each farm, approximately 250 to 1000 kg of wet beans and pulp were placed on banana and plantain leaves, resulting in heaps of 95-180 cm diameter and 40-64 cm height, which were then covered with extra banana and plantain leaves, and left to ferment. The beans were not mixed during fermentation according to local practices. The entire fermentation took six days at both farms. Drainage of liquids produced during fermentation (sweatings) was allowed to penetrate into the ground. Drying of the fermented cocoa beans took around 10 to 14 days, depending on the weather. During fermentation there was an on line follow-up of the temperature outside and inside the heaps, pH (inside the heaps), and rain fall (pluviometer). Temperature and pH were measured by inserting a digital pH 340i sensor (WTW GmbH, Weilheim, Germany) in the middle of the fermenting cocoa beans. An overview of these and other important heap parameters is given in **Table 2.**

**Table 2** illustrates characteristic parameters of the heap fermentations carried out in accordance with the present invention. A questionnaire was used to get the necessary information from the farmers regarding their cocoa pods and fermentation heaps. Heap size (initial and final weight), heap dimensions (diameter and height), rainfall, and fermentation temperatures were measured.

**Table 2**

| Heap | Heap size (kg) | Heap dimensions (diameter in cm ― height in cm) | Pods ripening (days) | Rainfall (l/m² in 6 days) | Drying time (days) | Final weight (kg) | Initial temp (°C) | Max fermentation temp (°C) |
|---|---|---|---|---|---|---|---|---|
| **1** | ± 500 | 170 ― 55 | 2-3 | 16 | 14 | 60 | 30.0 | 42.2 |
| **2** | ± 300 | 135 ― 46 | 2-3 | 18 | 14 | 42 | 25.7 | 42.6 |
| **3** | ± 250 | 105 ― 45 | 2 | 56 | 10 | 40 | 28.7 | 42.8 |
| **4** | ± 300 | 120 ― 52 | 2-3 | 10 | 10 | 52 | 24.0 | 44.7 |
| **5** | ± 1000 | 180 ― 64 | 2-3 | 28.5 | 10 | 200 | 23.0 | 44.3 |
| **6** | ± 250 | 95 ― 40 | 3 | 29 | 10 | 33 | 26.0 | 44.1 |
| **7** | ± 500 | 135 ― 40 | 3 | 12 | 10 | 63 | 26.4 | 44.3 |

**Sampling.** Samples of the seven heaps were taken according to a fixed time schedule, namely at the start of the fermentation (time 0, fresh cocoa beans), and after 6, 12, 18, 24, 30, 36, 42, 48, 54, 60, 66, 72, 84, 96, 120, and 144 h of fermentation. Sampling was done always at the same depth of the bean mass (approximately 30 cm from the upper surface), but in different points of the heap. Each sample consisted of 600 grams of beans that were aseptically removed and transferred into sterile plastic bags and was destined for culture-dependent, culture-independent, and metabolite analyses (see below). A last sample was taken after the drying process of the fermented cocoa beans for quality assessment. Besides samples from the mucilaginous pulp of the opened pods, swab samples corresponding to a surface of 25 cm² were taken from the environment (surface of cocoa pods, banana leaves, baskets, machetes, and farmers' hands). For culture-independent and metabolite analyses, the 128 cocoa bean samples were cooled, frozen, and transported on dry ice to Belgium.

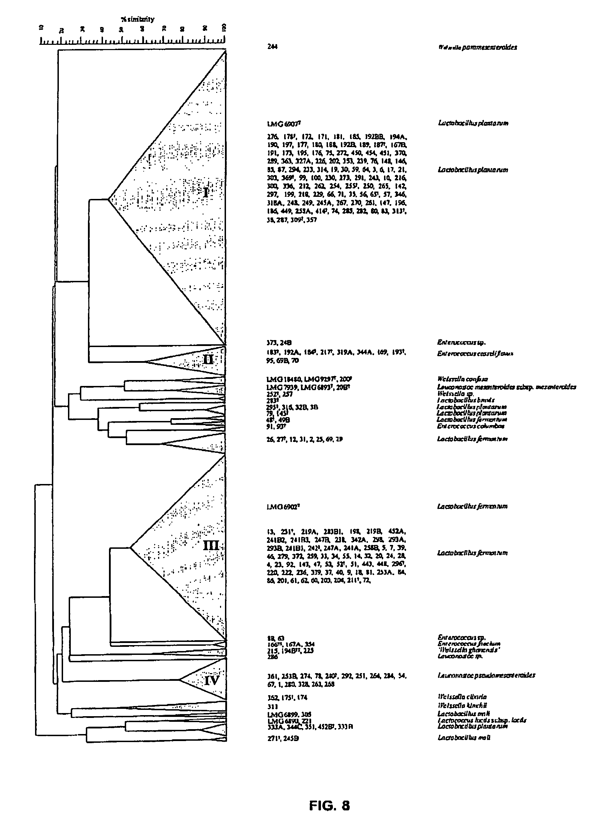

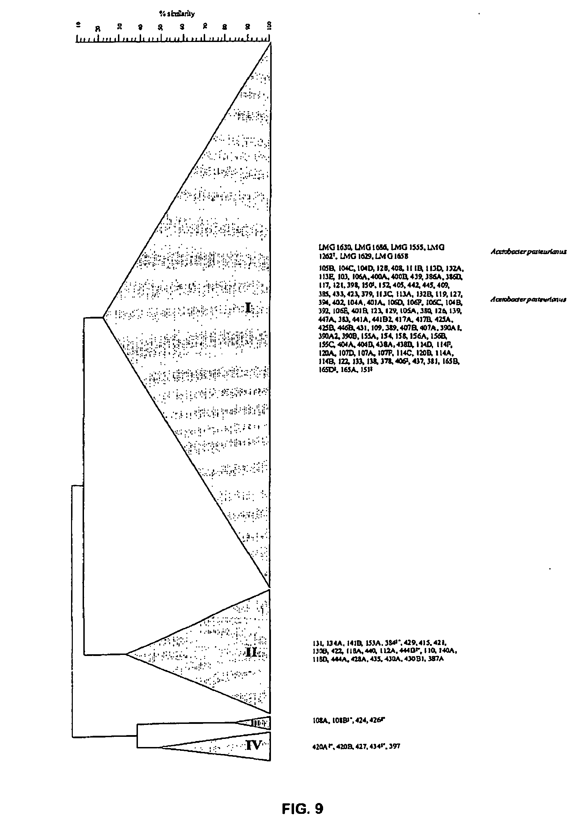

**Plating, enumeration, maintenance, and identification.** In Ghana the culture-dependent approach was performed immediately after sampling (fresh samples were transiently stored on ice and used in the laboratory within 1 h). Therefore, 180 ml of 0.1% (wt/vol) peptone water (Oxoid, Basingstoke, UK) were added to 20 g of pulp and beans in a sterile stomacher bag that was vigorously shaken for 3 min in a Stomacher 400 (Seward, Worthington, UK) to obtain a uniform homogenate. Samples (1.0 ml) of the homogenate were serially tenfold diluted in 0.1 % (wt/vol) peptone water, from which aliquots (0.1 ml) were plated on different selective agar media that were incubated aerobically at different temperatures during 1-4 days in a standard incubator, for the monitoring, isolation, and enumeration (by recording colony forming units, CFU) of specific groups of microorganisms responsible for fermentation: Plate Count Agar (PCA, Oxoid) for the total aerobic bacterial count (37°C), Malt Extract Agar (MEA, Oxoid) plus 100 mg l⁻¹ of oxytetracycline for yeasts (37°C), Deoxycholate-Mannitol-Sorbitol (DMS) agar (Guiraud, 1988; Série Agro-Alimentaire, Dunod, Paris) plus 400 mg l⁻¹ of cycloheximide for AAB (42°C), de Man-Rogosa-Sharpe (MRS) (de Man et al., 1960; J. Appl. Bacteriol. **23:**130-135) and Medium 17 (M17) agar (Oxoid) of Terzaghi & Sandine (1975; Appl. Microbiol. **29:**807-813) plus 400 mg l⁻¹ of cycloheximide for LAB (37°C); and Kanamycine Aesculin Azide (KAA, Oxoid) agar plus 400 mg l⁻¹_{,} of cycloheximide for enterococci (37°C). The swabs were transferred to 10 ml of 0.1 % (wt/vol) peptone water and mixed on a vortex for 2 min; 0.1-ml aliquots were spread on the agar media and incubated as described above. Morphologically different colonies were picked up from a suitable dilution of each sample on different agar media, grown in test tubes with the appropriate medium, purified through subculturing and plating, and stored in the same medium supplemented with 25 % (vol/vol) glycerol as cryoprotectant at -80°C; yeasts were stored on MEA plates or slants at 4ºC. This culture-dependent approach yielded 910 isolates (120 yeast isolates, 498 LAB isolates from MRS and M17, 40 LAB isolates from KAA, and 252 AAB isolates) for identification in Belgium. Yeasts were not identified during this study; the numbering of the identified bacterial strains is listed in **Fig. 8** **and** **9****.** It turned out that only approximately 15% of the LAB and AAB isolates could not be recovered due to transport from Ghana to Belgium. Also, material consisting of colonies washed with saline (0.85%, wt/vol, NaCl solution) from agar plates where the colonies were picked from was frozen and transported on dry ice to Belgium for culture-independent analyses.

In Belgium, all bacteria were checked for purity through successive transfers in and plating on the appropriate media, and subsequently identified through a polyphasic taxonomic approach, making use of both phenotypic (colony and cell morphology, mobility, Gram stain, catalase activity, oxidase activity, and organic acid production) and genotypic analyses. Potential LAB (382) and AAB (170) isolates were grown in MRS and Mannitol Yeast extract Peptone (MYP) medium [2.5% D-mannitol, 0.5% yeast extract, and 0.3% bacteriological peptone (Oxoid); wt/vol], respectively. Their identification was performed with an optimized Polymerase Chain Reaction (PCR) of repetitive DNA elements (rep-PCR) method, based on the (GTG)₅ primer, for both LAB (Gevers et al., 2001; FEMS Microbiol. Lett. 205:31-36) and AAB, in combination with sodium dodecyl sulphate-polyacrylamide gel electrophoresis (SDS-PAGE) of whole cell proteins for LAB (Pot et al. 1994, p. 493-521. In M. Goodfellow, and A. G. O'Donell (ed.), Chemical Methods in Prokaryotics Systematics. J. Wiley and Sons, Chichester, United Kingdom), 16S rRNA sequence analysis of representatives of the different clusters obtained after numerical analysis (BioNumerics version 4.0; Applied Maths, Sint-Martens-Latem, Belgium) of the rep-PCR profiles for both LAB and AAB (Gevers et al. 2001), and/or DNA:DNA hybridizations for AAB (Cleenwerck et al. 2002; Int. J. Syst. Evol. Microbiol. 52:1551-1558). For (GTG)₅-PCR and DNA:DNA hybridizations, total DNA was extracted from cells obtained through microcentrifugation (13,000 rpm for 20 min) of 10-ml overnight cultures of LAB in MRS medium and AAB in MYP medium, as described by Gevers et al. (2001), except that for AAB mutanolysin was substituted by proteinase K (VWR International, Darmstadt, Germany) in an amount of 0.0025 g ml⁻¹ TE buffer (10 mM Tris-HCl, 1 mM EDTA, pH 8.0). For more detailed identifications of AAB, the DNA base composition (Mesbah et al. 1989; Int. J. Syst. Bacteriol. 39:159-167) and supplementary phenotypic tests (Cleenwerck et al. 2002) were performed. Finally, cluster analysis of the rep-PCR profiles was performed according to season and farm.

**Direct extraction of DNA from fermented cocoa bean samples.** Twenty grams of frozen bean plus pulp samples were homogenized twice in a Stomacher 400 for 5 min, each time adding 70 ml saline. The combined fluid was removed (-+ 120 ml) by decanting and subsequently centrifuged at 170 x g at 4°C for 5 min to remove the majority of big particles. The supernatant was then filtered through a 20-µm pore-size filter (Whatman, Brentford, UK). In a next step the filtrate was centrifuged at 8,000 x *g* at 4°C for 20 min to pellet the cells, which were subsequently frozen at -20°C for at least 1 h. The thawed pellet was washed in 1 ml TES buffer (6.7%, wt/vol, sucrose; 50 mM Tris-HCl, pH 8.0; 1 mM EDTA) and resuspended in 300 µl STET buffer (8%, wt/vot, sucrose; 5%, wt/vol, Triton X-100; 50 mM Tris-HCl, pH 8.0; 50 mM EDTA). Seventy-five µl of lysis buffer (TES containing 1330 U ml⁻¹ mutanolysin and 100 mg ml⁻¹ lysozyme; Sigma-Aldrich, St. Louis, MO) and 100 *µ*l proteinase K (TE containing 0.0025 g ml⁻¹) were added, and the suspension was incubated at 37°C for 1 h. After addition of 40 µl preheated (37°C) 20% (wt/vol) SDS in TE buffer and a pinch of glass beads with a diameter of 150-212 µm (Sigma-Aldrich), cells were vortexed for 60 s, and incubated at 37°C for 10 min, followed by 10 min incubation at 65°C. One-hundred µlof TE buffer were added and the lysate was extracted with one volume of phenol/chloroform/isoamylalcohol (49:49:1) (Sigma-Aldrich) for 30 s. Phases were separated by microcentrifugation (13,000 rpm for 5 min at 4°C) using Phase Lock Gel tubes (Eppendorf AG, Hamburg, Germany). The aqueous phase was then further purified by using a NucleoSpin column according to the manufacturer's instructions (Macherey Nagel GmbH, Düren, Germany); this was primarily done to remove the remaining PCR-inhibiting compounds, as cocoa pulp contains several potentially inhibiting molecules such as polysaccharides, proteins, enzymes, and polyphenols. The DNA extraction method was tested with and without this extra purification step. DGGE of the PCR products revealed that more and stronger bands were visible from the DNA obtained with the extra step. Finally, the eluted phase was carefully mixed with 70 µl 5 M NaCl and 1 ml isopropanol, and the DNA was precipitated on ice for at least 15 min. The DNA was collected by microcentrifugation (13,000 rpm for 30 min at 4°C) and the pellet was washed in ice-cold 70% (vol/vol) ethanol. The DNA was dried in a vacuum excicator and resuspended in 100 µl TE. Three µl of DNase-free RNase (10 mg ml⁻¹; Sigma-Aldrich) were added and the whole was incubated at 37°C for 10 min. The samples were finally stored at -20°C until further use.

PCR. The primers used in this experiment were a primer pair that amplifies DNA from species of LAB (LAC1-LAC2; Walter et al., 2001) and a primer pair that amplifies DNA from species of AAB (WBAC1-WBAC2; Lopez et al., 2003), with the following sequences: LAC1 (5'-AGCAGTAGGAATCTTCCA-3') (SEQ ID NO: 3) and LAC2 (5'-ATTTCACCGCTACACATG-3') (SEQ ID NO: 4) targeting the V3-V4 region of 16S rRNA, and WBAC1 (5'-GTCGTCAGCTCGTGTCGTGAGA-3') (SEQ ID NO: 5), and WBAC2 (5'CCCGGGAACGTATTCACCGCG-3') (SEQ ID NO: 6) targeting the V7-V8 region of 16S rRNA. To facilitate DGGE separation, a GC-rich sequence (5'-CGCCCGCCGCGCCCCGCGCCCGGCCCGCCGCCCCCGCCCC-3') (SEQ ID NO: 7) was attached to one of the primers in each primer pair. PCR amplifications were performed using a DNA T3 thermal cycler (Biometra, Westburg, The Netherlands) in a final volume of 50 µl, containing 10 mM Tris-HCl, 50 mM KCl, 1.5 mM MgCl₂, 0.2 mM of each dATP, dCTP, dGTP, and dTTP, 0.2 µM of each primer, 1.25 IU of Taq DNA polymerase (Roche Diagnostics GmbH, Mannheim, Germany), and 3 µl of the extracted DNA (approximately 500 ng). One single PCR core program was used for all primer pairs: initial denaturation at 95°C for 5 min; 30 cycles of denaturation at 95°C for 20 s, annealing at a primer-specific temperature (LAC, 61 °C; WBAC, 67°C) for 45 s, and extension at 72°C for 1 min; and final extension at 72°C for 7 min, followed by cooling to 4°C. PCR amplification products were stored at -20°C. Amplicons (10 µl) were run in 1.5 x TAE (40 mM Tris-Acetate, 2 mM Na₂EDTA, pH 8.5) agarose (0.8%, wt/vol) gels at 100 V for 30 min, flanked by the EZ Load 100-bp molecular ruler (BioRad, Hercules, CA).

**DGGE analysis.** PCR products were analyzed on DGGE polyacrylamide gels by using a protocol based on that of Muyzer et al. (1993; Appl. Environ. Microbiol. 59:695-700.). The gels (160x160x1 mm) consisted of 8% (vol/vol) polyacrylamide (National Diagnostics, Atlanta, GA) in 1 x TAE buffer, using a 35-60% and a 50-70% denaturant gradient increasing in the direction of the electrophoretic run [100% denaturing polyacrylamide solution corresponds with 7 M urea (National Diagnostics) and 40% (vol/vol) formamide (Sigma)] for PCR products obtained with the LAC1-LAC2 and WBAC1-WBAC2 primers, respectively, found to be optimal for LAB and AAB, respectively (data not shown). Electrophoresis of PCR samples was carried out in 1.0 x TAE running buffer at 70 V for 16 h at a constant temperature of 60°C, using the Dcode System apparatus (BioRad). After electrophoresis, all gels were stained with ethidium bromide (50 µl of ethidium bromide in 500 ml of 1.0 x TAE buffer) for 10 min, followed by visualization of the DGGE band profiles under UV light. Digital capturing of images was performed by using the Gel Doc EQ system (BioRad). The resulting fingerprint pictures were analyzed using the BioNumerics version 4.0 software (Applied Maths). DGGE analyses were performed twice. Normalization of the gels was performed by using band ladders of known bacterial DNA in three lanes In all gels. Therefore, DNA originating from pure cultures of *Lactobacillus plantarum* LMG 6907^{T}, *Lactobacillus fermentum* LMG 6902^{T}, Leuconostoc *mesenteroides* subsp. *mesenteroides* LMG 6893^{T}, *Lactobacillus casei* LMG 6904^{T}, *Pediococcus acidilactici* LMG 11384^{T}, and *Lactobacillus acidophilus* LMG 9433^{T} on the one hand and of *L. plantarum* LMG 6907^{T}, *Enterococcus faecalis* LMG 7937^{T}, *Acetobacter pasteurianus* LMG 1262^{T}, Leuc. *mesenteroides* subsp. *mesenteroides* LMG 6893^{T}, and *Acetobacter syzygii* LMG 21419^{T} on the other hand, was mixed in equal volumes of the same concentration and used as a reference ladder in each DGGE gel for LAB and AA_{B}, respectively, after having positioned the corresponding PCR amplicons in a DGGE gel with the appropriate gradient. Finally, PCR-DGGE analysis was performed on DNA extracted from colonies recovered from agar plates of the corresponding heap samplings.

For cluster analysis of DGGE profiles, calculation of similarities in the profiles of bands was based on the Dice coefficient to provide a qualitative discrimination among the patterns. Dendrograms were obtained by means of the unweighted pair group method with arithmetic averages (UPGMA) clustering algorithm (BioNumerics version 4.0 software, Applied Maths).

For sequencing of DGGE bands, bands of interest were excised from the gels with a sterile blade, mixed with 50 µl of sterile water, and incubated overnight at 4°C to allow the DNA of the bands to diffuse out of the polyacrylamide gel blocks. Two µl of this aqueous solution was used to reamplify the PCR products with the same primers, including the GC-clamp. The amplicons were checked for purity by another DGGE run under the conditions described above with amplified DNA of the original sample as a control. Only reamplified PCR products migrating as a single band and at the same position with respect to the control were amplified with the primer without the GC damp and sequenced in a commercial facility using the capillary sequencing technology (VIB, Brussels, Belgium). Searches in the GenBank database were performed with the BLAST program (Altschul et al., 1997; Nucleic Acids Res. 25:3389-3402) to determine the closest known relatives of the partial 16S rRNA sequences obtained.

### Metabolite target analysis.

**(i) *Sample preparation.*** Frozen samples of beans plus pulp were used to prepare aqueous extracts. Beans were physically separated from pulp by manual peeling. Samples (20 g) of each fraction of pulp and beans separately were mixed with 80 ml of ultra pure water (MilliQ; Waters Corp., Milford, MA) with an Omnimixer (Phillips, Brussels, Belgium) for 5 min. The homogenate was centrifuged at 17,000 x g at 4°C for 15 min and the supernatant was retained. The sediment was washed with 20 ml of ultra pure water, centrifuged, and the washing supernatants were combined with the first supernatants to provide aqueous extracts for analysis. These extracts were clarified by filtration through 0.45-µm pore-size filters (Whatman) for further analyses.
(ii) ***HPLC.*** Organic acids from cultures of isolates were determined by high pressure liquid chromatography with a Waters chromatograph (Waters Corp.), equipped with a 2414 differential refractometer, a 600S controller, a column oven, and a 717plus autosampler. An ICSep ICE ORH-801 column (Interchim, Montluçon, France) was used with 10 mN H₂SO₄ as mobile phase at a flow rate of 0.4 ml min⁻¹. The column temperature was kept at 35°C. To remove proteins 700 µl trichloroacetic acid (20%, wt/vol) were added to 700 µl of sample. After centrifugation (16,060 x g for 15 min) the supernatant was filtered (0.2 µm; Minisart RC 4, Sartorius, Darmstadt, Germany) and appropriate dilutions were injected, run together with appropriate external standards.
(iii) ***GC-MS.*** Short-chain fatty acids (SCFA), branched SCFA, and other volatile compounds were measured through gas chromatography (GC) coupled with mass spectrometry (MS) according to the method described by Van der Meulen et al. (2006; Appl. Environ. Microbiol. 72:5204-5210) on an Agilent 6890-5973 N GC-MS (Agilent Technologies, Palto Alto, CA). The sample preparation was as follows: 100 µl of internal standard (0.3%, wt/vol, 2,6-dimethylphenol in ultra pure water) and 50 µl of H₂SO₄ were added to 500 µl of aqueous extract. After mixing for 15 s, 750 µl of diethyl ether were added to the sample, mixed thoroughly for 30 min, and centrifuged (16,060 x g for 15 min). Extraction with diethyl ether was performed twice before injection of the organic phase, together with the appropriate external standards. Ethanol, methanol, acetaldehyde, diacetyl, 1,3-butanediol, and acetoin were determined by GC-MS using the same protocol as described above, except that no H₂SO₄ was added, chloroform instead of diethyl ether was used as organic phase, and methanol (0.5 %, wt/vol) was used as internal standard.
(iv) ***HPAEC-PAD.*** The amounts of glucose, fructose, sucrose, mannitol, and erythritol were determined by high performance anion exchange chromatography with pulsed amperometric detection using a CarboPae™PA10 column (Dionex, Sunnyvale, CA). The mobile phase, at a flow rate of 1.0 ml min⁻¹, consisted of ultra pure water (0.015 µS cm⁻¹; eluent A) and 250 mM NaOH (eluent B). The following gradient was applied: 0.0 min, 85% A and 15% B; 10.0 min, 85 % A and 15% B; 20.0 min, 75% A and 25% B; 30.0 min, 65% A and 35% B; 50.0 min, 65 % A and 35% B; 51.0 min, 100% B; 56.0 min, 100% B; 57.0 min, 85% A and 15% B; 75.0 min, 85% A and 15% B. The aqueous extracts (700 µl) were treated with acetonitrile (700 ml) to remove proteins. The samples were appropriately diluted and filtered (0.2 µm; Minisart RC 4) prior to injection, and run together with the appropriate external standards.
*(v) **HPAEC-Conductivity.*** Organic acids (citric acid, acetic acid, lactic acid, gluconic acid, ketogluconic acids, formic acid, oxalic acid, malic acid, and fumaric acid) were determined by HPAEC-conductivity using an AS-19 column (Dionex). The mobile phase, at a flow rate of 1.0 ml min⁻¹ consisted of ultra pure water (0.015 µS cm⁻¹; eluent A) and 100 mM KOH (eluent B). The following gradient was applied: 0.0 min, 96% A and 4% B; 20.0 min, 96% A and 4% B; 60.0 min, 0% A and 100% B. The aqueous extracts were treated with acetonitrile as described above, appropriately diluted, and filtered (0.2 µm) prior to injection, and run together with the appropriate external standards.
(vi) ***LC-MS.*** Amino acids, amino acid metabolites, and succinic acid were quantified through LC-MS on a Waters 2695 LC coupled to a Quattro Micro™ mass spectrometer (Micromass, Waters Corp.). Succinic acid and amino acid metabolites were determined according to the method of Van der Meulen et al. (2006). In the case of amino acids a Symmetry column (Waters Corp.) was used. The mobile phase, at a flow rate of 0.2 ml min⁻¹ and linearly increasing to 0.5 ml min⁻¹ over a period of 45 min followed by a flow rate of 0.2 ml min⁻¹ afterwards, was composed of 0.1 % (vol/vol) formic acid in ultra pure water (eluent A) and 90 % (vol/vol) acetonitrile in ultra pure water (eluent B). The following gradient was used (vol/vol): 0.0 min: 90% A, 10 % B; 45.0 min: 10% A, 90% B; 46.0 min: 90% A, 10% B; 60.0 min: 90% A, 10% B. To 500 µl of aqueous extract 100 µl of internal standard (0.002 %, wt/vol, 2-aminobutyric acid in ultra pure water) was added. The amino acids were derivatized using an AccQ Fluor Reagent kit according to the manufacturer's instructions (Waters Corp.). The derivatized samples were injected together with the appropriate external standards.

Sample preparations and analyses as described above were performed in triplicate and the mean values ± standard deviations are represented as milligrams per gram of pulp or beans.

**Quality assessment of fermented, dried Ghanaian cocoa beans.** Fermented, dried cocoa beans were checked for appearance (bean count per 300 g of beans, bean size, physical damage, insect penetration) and quality in Ghana (Quality Division of the cocoa board; COCOBOD, Accra, Ghana) and by Barry Callebaut Belgium. The cut test, which is an index of fermentation and relies on changes in color, is the standard test used to assess the suitability of cocoa beans for chocolate manufacture. A total of 300 beans were cut lengthwise through the middle to expose the maximum cut surface of the cotyledons. Both halves were examined in full daylight and placed in one of the following categories: fully brown (fermented); partly brown, partly purple (partly fermented); purple (not fermented); slaty (over-fermented); insect damaged; moldy; or germinated.

### RESULTS

### Population dynamics of Ghanaian cocoa bean heap fermentations,: culture-dependent approach

The culture-dependent population dynamics of seven spontaneous cocoa bean heap fermentations were based on plate counts obtained through selective plating and incubation. **Figure 10A** shows a representative fermentation course with respect to succession of the different microbial groups selected for (only heap 5 is shown). In general, simultaneous development of yeasts, LAB, and AAB took place; no other major groups of microorganisms were involved as reflected by the PCA counts. Yeast counts of log 4.3―log 7.0 CFU g⁻¹ of pulp and beans were present in the heap at the beginning of the fermentation. The size of the yeast population increased during the first 12―18 h, and grew to maximum populations of log 6.6―log 7.5 CFU g⁻¹. Upon prolonged fermentation the yeast population declined to log 3.0― log 4.7 CFU g⁻¹, and in heap 1 no yeasts were left at the end of the fermentation. All fermentations were characterized by a high level of LAB right from the start of the fermentation (log 4.4―log 8.0 CFU g⁻¹) as reflected by both MRS and M17 counts. LAB grew during fermentation to maximum populations of log 7.5―log 8.9 CFU g⁻¹ after 30―48 h. Afterwards there was a slight decrease of the LAB population, sometimes stabilizing upon prolonged fermentation (log 5.0―log 6.5 CFU g⁻¹). KAA counts (log 3.6―log 5.7 CFU g⁻¹) were always lower than these on MRS and M17. However, KAA counts always decreased upon prolonged fermentation, even reaching zero levels after 72, 84, and 120 h of fermentation in heaps 4, 5, and 6, respectively. AAB, initially present in densities of log 2.0― log 4.6 CFU g⁻¹ grew slower and reached lower maximum population densities (log 6.4―log 7.5 CFU g⁻¹) after about 66 h of fermentation. As for LAB, the AAB population slightly decreased upon prolonged fermentation and sometimes stabilized (log 4.9―log 6.2 CFU g⁻¹).

All fermentations were characterized by an initial pH of approximately 3.5 that increased during the first 18 h of fermentation, sometimes preceded by a slight and short decrease, followed by a constant, almost linear increase to approximately pH 4.0-4.3 after 120-144 h **(****Fig.10B****).**

The ambient temperature during the day and night was 24.0-39°C and 19-24°C, respectively, maxima being slightly lowered in the case of rain fall **(****Fig. 10B****).** The temperature inside the heaps went from ± 26.3°C at the start of the fermentations to a maximum temperature of ± 43.5°C **(****Fig. 10B****).** Rain fall slightly influenced the temperature course of the heap with ups and downs of 1-4°C **(****Fig.10B****).**

In general, no significant differences were observed between the repetitions and the seasons for microbial counts, pH, and temperature during cocoa bean fermentation at both farms, indicating validness of the sampling, measurement and isolation procedures.

### Identification of the isolates

Phenotypic analyses indicated that all isolates from MEA were yeasts (big colonies and bigger cells as compared with LAB and AAB) and that 240 out of 382 isolates from MRS, M17, and KAA belonged to the LAB group (Gram-positive; rods or cocci; non-motile; catalase-negative; oxidase-negative; production of lactic acid, acetic acid, and/or ethanol), while 132 out of 170 isolates from DMS belonged to the AAB group (Gram-negative; rods; motile or non-motile; catalase-positive; oxidase-negative; production of acetic acid, gluconic acid, and 2-keto-gluconic acid). Noteworthy was the selective isolation of AAB on DMS and of *L. plantarum* on KAA under the conditions used, representing 90% and 72% of the isolates, respectively; almost no enterococci were found neither on KAA (15%) nor on the other media (Fig. 11). Numerical rep-PCR analysis and identification of all bacterial isolates as compared with reference strains, in combination with 16S rRNA sequencing (for LAB and AAB) and DNA:DNA hybridizations (for AAB) of representative strains of each cluster, revealed a limited biodiversity of both LAB and AAB in the fermentations analyzed **(****Fig. 8** **and** **9****).** However, new taxa of both LAB (e.g. *Weissella* sp., **Fig. 8****)** and AAB (e.g. *Acetobacter* spp., clusters III and IV, **Fig. 9****)** were found.

LAB identification **(****Fig.8****)** revealed four main clusters among the isolates: *L. plantarum* (cluster I, 114 strains), *L. fermentum* (cluster III, 76 strains), *Leuconostoc pseudomesenteroides* (cluster IV, 16 strains), and *Enterococcus casseliflavus* (cluster II, 11 strains). All these species were picked up from all heaps, except for *Leuc. pseudomesenteroides* that was not isolated from heaps 3 and 6 and *E. casseliflavus* that was not isolated from heaps 1 and 2 **(Table 3).** The remaining 23 LAB isolates were distributed among different taxa **(****Fig. 8****, Table 3).** For instance, different *Weissella* species were picked up in the beginning of the fermentations **(Table 3).** Interestingly, a bacterium (isolates 194B, 215, and 225) of which the 16S rRNA sequence was mostly related to the genus *Weissella,* further referred to as belonging to the newly proposed species '*W. ghanensis',* was picked up from heap 7. Also, isolates 252 (confirmed by 16S rRNA sequence analysis) and 257 belong to a new Weissella sp. Occasionally, isolates belonging to *E. faecium, Lactobacillus brevis, Lactobacillus mali,* and *Leuc. mesenteroides* were found, but only in the beginning of the fermentations **(Table 3).**

AAB identification **(****Fig. 9****)** revealed four main clusters among the isolates: *A. pasteurianus* (cluster I, 100 strains), *Acetobacter syzygii-like* (cluster II, 23 strains), *Acetobacter tropicalis-*like (cluster III, 4 strains), and A. *tropicalis-*like (cluster IV, 5 strains). DNA:DNA hybridizations between isolates 108B (cluster III) and 420A (cluster IV) revealed a DNA homology value of 75%. Hybridizations of both isolates against the type strains of A. *tropicalis* LMG 21419^{T} and the newly proposed species *'A. senegalensis'* LMG 1617^{T}, their phylogenetically closest neighbors, revealed a DNA homology value of 54-58%, which is below species level, and of 79-81%, which is above the accepted limit (70%) for species delineation (Stackebrandt et al., 2002; Int. J. Syst. Evol. Microbiol. 52:1043-1047), respectively, indicating that rep-PCR clusters III and IV represent two clusters of the newly proposed '*A. senegalensis'* species. The G+C content of DNA from isolates 108B and 420A was 55.6 and 55.9 mol%, respectively. These values were similar to the DNA G+C content obtained for the type strain of *'A. senegalensis'*. Isolates 108B and 420A showed the same phenotypic features as the type strain of *'A. senegalensis':* growth on yeast extract and 30% (wt/vol) D-glucose, growth with ammonium as the sole nitrogen source and ethanol as energy source, growth in the presence of 10% (vol/vol) ethanol, growth on glycerol as the sole energy source, but not on maltose or methanol; able to produce 2-keto-*D*-gluconic acid from D-glucose but not 5-keto-D-gluconic acid. All AAB species were picked up from all heaps, except for *A. syzygii-*like and A. *tropicalis-like* that were not picked up from heap 3 and heaps 1, 3, and 5, respectively.

Isolates with highly similar or even identical (GTG)₅-PCR fingerprints were frequently found within the set of LAB and AAB isolates recovered from the same fermentation heap, whereas the samples were always taken at the same depth but in different points of the heap, indicating clones of the same strain and a possible microbial succession during fermentation at the strain level (see below). Cluster analyses of the rep-PCR profiles according to farmer and season did not reveal significantly different results.

Based on the distribution of the identified isolates as a function of selective media used **(****Fig. 11****)** and fermentation time **(Table 3)**, the following observations were made. Most *L*. *plantarum* strains were isolated from MRS. The highest relative counts found in all fermentations were these of *L. plantarum* and *L. fermentum* (log 4―log 7 CFU g⁻¹). *L. plantarum* was the most abundant species in the first 42 h of fermentation, while *L. fermentum* was still isolated upon longer fermentation time. *Leuc. pseudomesenteroides* was isolated in the beginning of almost all heap fermentations (log 5―log 7 CFU g⁻¹), except for heaps 3 and 6 where it was absent. Also, *E. casseliflavus* was only isolated at the beginning of the fermentations (log 2―log 6 CFU ml⁻¹). *Leuc. pseudomesenteroides* (six isolates), E. *casseliflavus* (three isolates) as well as other enterococci (five isolates), *Weissella* spp. (five isolates), *Leuconostoc durionis* (one isolate), *L. mali* (one isolate), and *Lactococcus lactis* subsp. *lactis* (one isolate) were found on farmers' hands, baskets, machetes, and pods **(Table 3;** **Fig. 8****).** Baskets and leaves were contaminated with yeasts, *L. plantarum (24* isolates), and *L. fermentum* (eight isolates) too. Whereas all AAB isolates were derived from DMS agar, the most dominant isolates from the beginning of the cocoa fermentation belonged to *A. pasteurianus* and *A. tropicalis-like* species **(Table 3)**, both in amounts of log 3―log 6 CFU g⁻¹. Later on, isolates of *A. tropicalis-*like were replaced by *A. syzygii-*like, the latter occurring in amounts of log 4―log 6 CFU g⁻¹, but both spedes disappeared while A. *pasteurianus* survived longer throughout the fermentations **(Table 3).** It was difficult to recover LAB and AAB isolates from the end of the fermentations **(Table 3).**

**Table 3** represents sources of the different species of lactic acid bacteria (LAB) and acetic acid bacteria (AAB) that were identified, listed according to the number of isolates. Sample numbers S0, S1, S2, S3, S4, S5, S6, S7, S8, S9, S10, S11, S12, S13, S14, S15, and S16 represent samples taken after 0, 6, 12, 18, 24, 30, 36, 42, 48, 54, 60, 66, 72, 84, 96, 120, and 144 h of fermentation. Isolates from cocoa pods, baskets, machetes, banana/plantain leaves, and farmers' hands were taken as swabs.

**Table 3**

| Taxon (number of isolates) | Heap | Sample |
|---|---|---|
| LAB | | |
| *Lactobacillus plantarum* (114) | 1 | S0, S1, S2, S3, S4, S6, S8, S9, leaves |
| | 2 | S4, S9 |
| | 3 | S0, S1, S2, S3, S5, machete, hands, pods |
| | 4 | S0, S1, S2, S3, S4, S6, S7 |
| | 5 | S0, S1, S2, S3, S4, S5, S8, hands, baskets |
| | 6 | S0, S1, S2, S3, S4, S5, S6, S7, S8, S9, S10, baskets, hands |
| | 7 | S0, S2, S3, S5, S6, S7, S8, S13 baskets, hands |
| *Lactobacillus fermentum* (76) | 1 | S0, S1, S2, S4, S6, S9, leaves |
| | 2 | S0, S1, S2, S3, S6, S9, S11, S13 |
| | 3 | S1, S3, S4, S5, machete, pod |
| | 4 | S1, S3, S4, S5 |
| | 5 | S3, S4, S5, S8, machete |
| | 6 | S3, S5, S7, S9, S10, S11, S12, S14, hand, leaf |
| | 7 | S0, S8, S12 |
| *Leuconostoc pseudomesenteroides* | 1 | S4, S6 |
| (16) | 2 | S4 |
| | 3 | machete, hand |
| | 4 | S0, S1 |
| | 5 | S0, S1, S2, machete, basket, hand |
| | 6 | pod |
| | 7 | S6 |
| *Enterococcus casseliflavus* (11) | 4 | S0 |
| | 5 | hand |
| | 6 | S0, leaf, basket |
| | 7 | S0, S2 |
| *Weissella confusa* (1) | 6 | hand |
| *Weissella paramesenteroides* (1) | 6 | machete |
| *Weissella cibaria* (3) | 5 | S1, hand |
| *Weissella kimchii* (1) | 5 | S1 |
| *'Weissella ghanensis' (3)* | 7 | S2 |
| *Weissella* sp. (2) | 6 | S1, S3 |
| *Enterococcus faecium* (3) | 4 | S0 |
| Enterococcus spp. (5) | 1 | leaf |
| | 3 | S2,S3 |
| | 4 | S3 |
| *Enterococcus columbae (2)* | 3 | machetes |
| *Lactococcus lactis* (1) | 6 | pod |
| *Leuconostoc mesenteroides* (1) | 1 | S6 |
| *Leuconostoc* sp. (1) | 5 | hand |
| *Lactobacillus brevis* (1) | 5 | S4 |
| *Lactobacillus mali* (3) | 4 | S4 |
| | 5 | pod |
| AAB | | |
| *Acetobacter pasteurianus* (100) | 1 | S4, S5, S9, S10, S11, S12, S13, S14 |
| | 2 | S2, S9, S10, S11, S12, S13 |
| | 3 | S1, S2, S3, S7 |
| | 4 | S5, S6, S8, S9, S10 |
| | 5 | S5, S6, S8, S9, S10, S11, S12 |
| | 6 | S1, S2, S3, S7, S10, S12 |
| | 7 | S1, S2, S5, S6, S7, S12 |
| *Acetobacter syzygii-like* (23) | 1 | S3, S5, S11, S12 |
| | 2 | S10, S11, S12 |
| | 4 | S6 |
| | 5 | S1 |
| | 6 | S5, S6, S9, S12 |
| | 7 | S3, S4, S7, S8, S10 |
| *Acetobacter tropicalis-*like (9) | 1 | S5 |
| | 2 | S3 |
| | 4 | S5 |
| | 6 | S1, S2, S3, S6 |
| | 7 | S11 |

### Population dynamics of Ghanaian cocoa bean heap fermentations: culture-independent approach

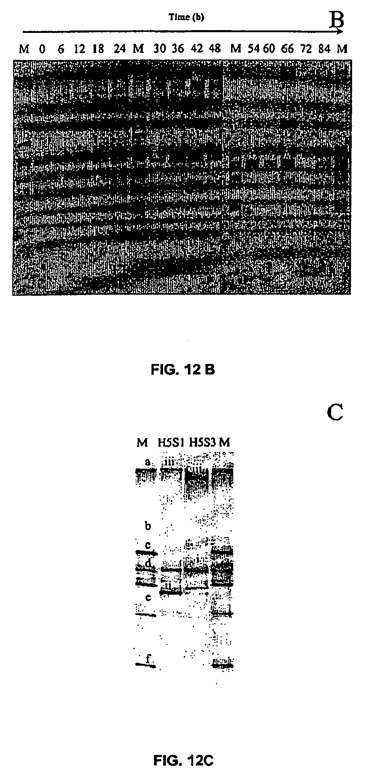

Preliminarily, each phase of the method used for DNA extraction from fermented cocoa bean samples and of the PCR-DGGE protocol was optimized, as described in the Materials and Methods section. **Figure 12** shows representative fermentation courses for all seven spontaneous cocoa bean heap fermentations as obtained by a total community analysis of DNA samples through 16S rRNA-PCR-DGGE with the LAC1-LAC2 primer pair as well as the results of the band sequencing (only the results of heaps 2 and 5 are shown). The number and intensity of visible bands varied among samples with fermentation time and among fermentations, which could be related to shifts of the bacterial compositions and hence reflection of the impact of certain strains on fermentation (cf. infra) as well as heterogeneous samplings. Concerning the microbial succession as determined by DGGE in general, it turned out that *L. fermentum* (band i) was the most dominant species throughout fermentation in all heaps **(****Fig. 12A,B),** although *L. plantarum* (band iii) was detected in heap fermentations 2, 6, and 7 mainly in the first part of the fermentation **(****Fig. 12A****).** In general, the intensity of the band corresponding with *L. fermentum* rose in the beginning of the fermentation and was most intense towards the end **(****Fig. 12A,B),** in some fermentations still visible after 66 h of fermentation **(****Fig. 12B****)** and in others already lower before **(****Fig. 12A****).** *Leuc. pseudomesenteroides* (band ii) was detected during the first 24 h of fermentation in heaps 2, 3, 4, 5, and 7 **(****Fig. 12A,B).** Also, an unidentified bacterium (band iv), of which the partial 16S rRNA sequence was identical to a *Weissella*-like species (the newly proposed species '*W. ghanensis')* appeared throughout heap fermentations 2, 3, 4, 5, and 6 up to 48 h **(****Fig. 12B****).** A too low number of LAB at the end of fermentation resulted in lanes without bands **(****Fig. 12A****).** Finally, PCR-DGGE analyses performed on DNA extracted from colonies recovered from agar plates where they were picked from revealed the same species identities **(****Fig. 12C****).** Using the WBAC primers, PCR-DGGE analyses revealed again *L. plantarum* and L. *fermentum* as the dominating species, while no AAB species were detected, although PCR-DGGE analysis of the reference strains was successful (data not shown). Taking into account all PCR-DGGE runs performed, no differences were observed between the repetitions.

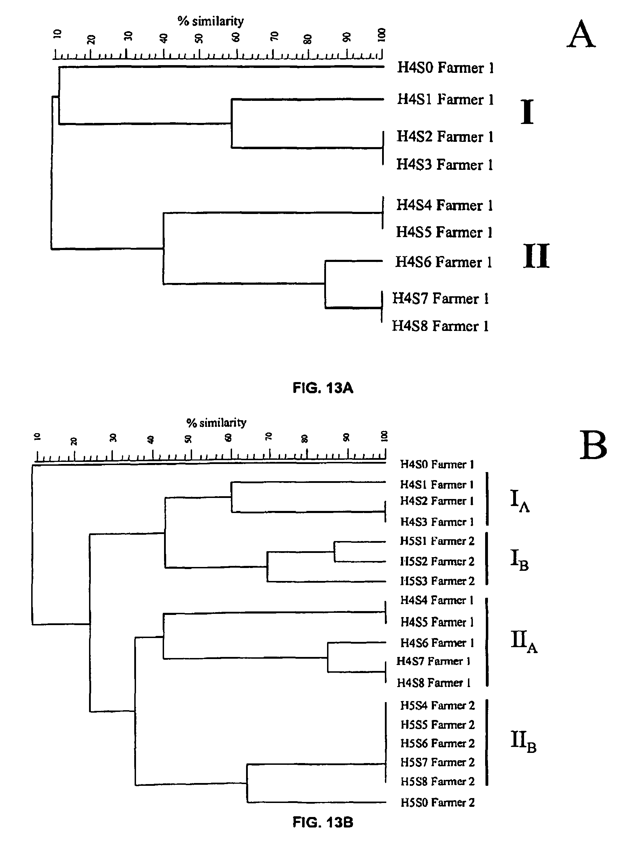

Cluster analysis of the PCR-DGGE profiles revealed no influence of the season . However, it revealed influences of the farm (heaps). For instance, cluster analysis of the PCR-DGGE profiles of heap fermentations 4 and 5 revealed a difference in bacterial ecology between both farms as revealed by the 60% similarity percentage limit **(****Fig. 13A****).** Also, a shift in the bacterial ecology from day 1 (samples 1 to 3) to day 2 (samples 4 to 8) occurred within a heap (clusters I and II), with respect to the differences between the farms (subclusters A and B). The start sample that occurred as outlier, according to the clustering performed, showed a shift in bacterial ecology (as revealed by its microbiological analysis), due to adaptation of the bacteria to the fermentation matrix **(****Fig. 13B****).**

### Metabolite target analyses of Ghanaian cocoa bean heap fermentations

In parallel with the plating and isolation (culture-dependent approach) and PCR-DGGE analyses (culture-independent approach), the substrates consumed and metabolites formed during fermentation were monitored through chromatographic analyses of fermented cocoa bean samples in Belgium **(Fig. 11C-H).** Preliminarily, standardization of sample collection, preparation, and extraction procedures, standardization and validation of the measurements, and limited data on profiles and natural variation of the aqueous extracts was tackled before these methods could be used on a routine basis (data not shown). Variations within one and between different fermentations was mainly due to sample collection, as the fermenting mass was not turned and hence was not homogenous, being more pronounced for smaller heaps.

In general, almost all sucrose in the pulp was gone at the start of fermentation **(Fig. 11C).** The citric acid of the pulp was rapidly consumed as well (within ± 30-48 h of fermentation) and paralleled LAB development **(Fig. 11F),** causing the pH value of the pulp to increase. The initial decrease in citric acid of the beans was less than that of the pulp **(Fig. 11F).** At the end of the fermentations, citric acid always slightly increased or stabilized both in pulp and beans. Fructose and glucose disappeared simultaneously in the pulp and were almost exhausted after ± 36-48 h of fermentation, whereas mannitol increased from ± 20 h of fermentation to stabilize from ± 70 h of fermentation and upon **(Fig. 11C).** Erythritol was not found. Sucrose was hydrolyzed into glucose and fructose in the beans, explaining its decrease and their increase, respectively **(Fig. 11D).** Ethanol increased upon fermentation, almost simultaneously in pulp and beans, and reached a maximum of ± 10-25 mg g⁻¹ after ± 30-36 h of fermentation, after which it declined **(Fig. 11H).** Acetic acid levels increased after ± 6 h of fermentation. Concentrations of ± 10-15 mg g⁻¹ of acetic acid were found in the fermented cocoa bean pulp after ± 90 h of fermentation.

The total amount of organic acids differed from heap to heap. Whereas the amounts of ethanol and acetic add were always slightly higher in the pulp than in the beans, the amounts of lactic acid were considerably higher in the pulp than in the beans. Lactic acid reached a maximum concentration of ± 1.0-9.0 mg g⁻¹ in the pulp after ± 48 h of fermentation. Succinic acid increased up to ± 0.10-0.25 mg g⁻¹ and ± 0.23-0.65 mg g⁻¹ in pulp and beans, respectively, after ± 40-60 h of fermentation. Ketogluconic acid, malic acid, and fumaric acid were not found; gluconic acid (± 2-4 mg g⁻¹ in pulp and ± 0.2-0.4 mg g⁻¹ in beans) and oxalic acid (± 1 mg g⁻¹ in pulp and ± 1-2 mg g⁻¹ in beans) remained stable as a function of time. Fatty acids (except for acetic acid), methanol, acetaldehyde, diacetyl, 1,3-butanediol, and acetoin were not found under the analysis conditions.

There was an increase in all free amino acids upon fermentation from ±.1-3.5 mg g⁻¹ to ± 4.4-4.8 mg g⁻¹ in the pulp and from ± 1.7-2.1 mg g⁻¹ to ± 5.2-5.9 mg g⁻¹ in the beans (depending on the amino acid), except for lysine and glutamine in the pulp, asparagine in pulp and beans, and aspartic acid in the beans. In general, hydrophobic amino acids increased and acid amino acids decreased upon fermentation time. The glutamic acid content increased in the pulp and first decreased and then increased in the beans. Arginine showed a decrease followed by an increase in the pulp, while histidine showed an increase followed by a decrease in the beans. Aromatic amino acid metabolites such as phenyllactic acid and OH-phenyllactic acid were not found under the analysis conditions.

### Quality assessment of fermented, dried Ghanaian cocoa beans

The Ghanaian fermented, dried beans were of good quality. All parameters tested were good or excellent. Bean counts varied from 253-286 per 300 g of beans, while broken beans only represented 1.59-3.43 g per 300 g of beans. Slaty beans were not detected, indicating that drying took place after fermentation. Only very slight differences were observed between the heaps concerning the other cut test parameters. For instance, heap 4 showed a slightly higher percentage of violet beans and heap 6 showed a slightly higher percentage of flat and violet beans.

### DISCUSSION

Cocoa bean fermentations continue to be conducted in a traditional manner, resulting in a great diversity in production methods and organoleptic characteristics. In the present experiment a multiphasic approach was used for the first time to systematically study the biodiversity, population dynamics, and metabolomics of spontaneous cocoa bean heap fermentations in Ghana.

The Ghanaian cocoa bean heap fermentations, performed at two different small holders in two different seasons in two different years, were characterized by a high initial level and rapid development of both yeast and LAB and a lower maximum temperature as compared with other cocoa bean fermentations reported in the literature (counts are generally between log 3-7 and log 7-9 to finally log 2-3 CFU g⁻¹, between log 4-5 and log 8-9 to log 1.5 CFU g⁻¹, and between log 3-4 and log 6-7 to 0 CFU g⁻¹ for yeast, LAB, and AAB, respectively, and the maximum temperature is often up to 50°C; Schwan et al., 1995, J. Appl. Bacteriol. Symp. Suppl. 79:96S-107S; Ardhana and Fleet, 2003; Int. J. Food Microbiol. 86:87-99). Ethanol was produced by yeasts through fermentation of sugars (sucrose, glucose, and fructose). LAB produced lactic acid from both sugars and citric acid. Oxidation of ethanol to acetic acid was performed by AAB. This oxidation process was responsible for the increase in temperature inside the heap, and hence a maximum population of AAB corresponded with a maximum fermentation temperature. Part of the acetic acid volatilized and part penetrated into the cotyledons of the beans and was, together with part of the ethanol and the heat, responsible for killing of the cocoa seed embryo and changes in the sub-cellular structure of the beans, being an important end-point of fermentation.

In general, cocoa bean fermentations are characterized by a succession of microbes, reflecting the environmental factors (temperature, pH, and oxygen tension) and the metabolism of substrates of the cocoa bean pulp or derived from the cocoa beans (depending on composition and harvest conditions), resulting in production moments of significant amounts of ethanol, lactic acid, and acetic acid (Schwan et al., 1995, Ardhana and Fleet, 2003). In the present experiment a clear three-phase fermentation process of a well ordered succession of microbial groups and a timely production of acids was difficult to recognize. As both initial yeast and LAB levels were high, sucrose was already gone at the start of fermentation (see below), yeasts further developed slightly, and LAB developed considerably from the start until two days of fermentation. Pod ripeness and post-harvest pod age (due to transportation and transient storage) and hence pH of the cocoa bean pulp will determine the initial amounts of yeasts and LAB. Moreover, yeast metabolism favors growth of aciduric LAB. The speed of AAB development will determine the time course within which a maximum fermentation temperature is reached and hence of the whole fermentation, as the heat generated through acetic acid formation will be responsible for the death of almost all microbes. This mainly depends on the size of the heap and the influence of turning, as both factors will influence sweating and aeration of the heap, and hence full development of yeast, LAB, and AAB.

Although the microbial ecology might be influenced by cocoa cultivar, pod age, fermentation method and site, as well as sample collection, the present experiment revealed that the biodiversity of both LAB and AAB in the Ghanaian cocoa bean heap fermentations analyzed was rather restricted, in contrast with a rich and varied yeast microbiota reported so far for (Ghanaian) cocoa bean fermentation (Sanchez et al., 1985; Lebesmitt. Wiss. Technol. 18:69-76; Schwan et al., 1995; Ardhana & Fleet, 2003; Jespersen et al., 2005; FEMS Yeast Res. 5:441-453; Nielsen et al., 2005; Yeast 22:271-284.). A road diversity of both LAB and AAB was also reporter earlier (Carr et al., 1979 Cocoa fermentation in Ghana and Malaysia I. Natural Resources Institute, Chatham, UK; Passos et al., -298; 1984a,b J. Food Sci. 49:1470-1474; J. Food Sci. 49:205-208;; Passos & Passos, 1985; Rev. Microbiol. 16:290; Thompson et al., 1997; p. 649-661. In M. P. Doyle, L. R. Beuchat, and T. J. Montville (ed.), Food Microbiology Fundamentals and Frontiers. ASM Press, Washington, DC).

In the present experiment both culture-dependent and -independent methods showed that *L. plantarum* and *L. fermentum* were the most dominant species in the Ghanaian cocoa bean heap fermentations performed. Moreover, *L. plantarum* decreased and *L. fermentum* increased upon fermentation time. These data were supported by both PCR-DGGE and enumeration on MRS, M17, and KAA, the latter medium being selective for *L. plantarum* under the applied conditions. To our knowledge this is the first report to show their individual succession. Culture-dependent microbiological analysis further indicated that *A. pasteurianus* fulfilled a key role in the Ghanaian cocoa bean heap fermentations performed, given its isolation throughout fermentation. *A. syzygii-*like and A. *tropicalis-*like strains were not always isolated and the latter ones disappeared faster than the former ones. Although AAB isolated from fermented material are difficult to grow in the laboratory (Ardhana & Fleet, 2003; Ndoye et al., 2006; Enz. Microbial Technol. 39:916-923), they grew or remained viable under anaerobic conditions of the heaps, as has been found in wine fermentations (Gonzalez et al., 2006; FEMS Microbiol. Lett. 254:123-128). DGGE analyses of AAB did not produce satisfactory results; again *L. plantarum* and *L. fermentum* were detected with the WBAC primers used that were actually developed to monitor both LAB and AAB during wine fermentation (Lopez et al., 2003; Appl. Environ. Microbiol. 69:6801-6807). This underlines the importance of the detection limit to carry out PCR-DGGE, which is generally between log 4 and log 6 CFU g⁻¹ or higher, depending on the bacteria investigated, and hence detecting the >90% most numerous species of a community without discriminating living from dead cells or cells in a viable but not cultivable state (Ercolini, 2004; J. Microbiol. Meth. 56:297-314; De Vero et al., 2006; Food Microbiol. 23:809-813). The amount of the total AAB during the Ghanaian cocoa bean heap fermentations performed was never higher than log 7 CFU g⁻¹ and this occurred only at a certain point in the middle of the fermentations. A possible solution to the problem could be the use of different species-specific primers targeting other regions of the 16S rRNA gene or other genes (Trc̃ek, 2005; Syst. Appl. Microbiol. 28:735-745), or increasing the intensity of the PCR amplicons produced from DNA by applying a nested PCR (González et al., 2006). Both the agar medium used for isolation and the high temperature of the heaps are responsible for the selective isolation of *A. pasteurianus,* as the latter species prefers calcium lactate and is more heat-resistant and ethanol-tolerant. A. *syzygii* and *A. tropicalis* have not been found during cocoa bean fermentation before.The clusters corresponding with *A. syzygii-*like (cluster II) and *A. tropicalis*-like (clusters III and IV) isolates indicate new species of *Acetobacter.* Isolates of clusters III and IV belonged to *'A. senegalensis',* a newly proposed heat-resistant AAB species isolated from mango fruit; isolates of cluster II are under investigation.

In the beginning of the fermentation *Leuc. pseudomesenteroides* and *E. casseliflavus* were present too, but they disappeared rather rapidly. These species are often associated with plant material. The newly proposed species *'W. ghanensis'* appeared throughout some of the fermentations as revealed by PCR-DGGE. Therefore, it can be concluded that *'W*. *ghanensis'* plays some role in the Ghanaian cocoa bean heap fermentation process. All dominant microbes mentioned above come from the environment as well. *L. plantarum* and *L. fermentum* are associated with plant material. *A. pasteurianus, A. syzygii,* and *A. tropicalis* (including the newly proposed species *'A. senegalensis')* are generally isolated from fermented foods, flowers and fruits, and fruits and fermented foods, respectively, often from tropical countries (Lisdiyanti et al., 2000, 2001, 2003; Microbiol. Cult. Coll. 19:91-98). Some of these heat-resistant strains are interesting for industrial vinegar production at higher temperature (Ndoye et al., 2006).

Only few strains of the better adapted populations of *L. plantarum, L. fermentum, 'W. ghanensis',* and A. *pasteurianus* outnumbered the rest of the microbiota and were responsible for spontaneous fermentation of the cocoa beans, as only a limited number of strains within a heap and among heaps were found (as revealed by their (GTG)₅ PCR DNA fingerprints). Moreover, as no differences were observed between the season and only slight differences could be detected between the farms (as revealed by cluster analysis of the PCR-DGGE profiles), it can be concluded that the cocoa bean heap fermentations performed during this experiment were dominated by certain strains and were hence very reproducible, which supports the general high quality standard of Ghanaian fermented cocoa beans.

Metabolite target analysis during this study revealed that sugars, in particular sucrose, were utilized by the yeasts, being converted to ethanol and carbon dioxide, while glucose, fructose, and citrate were used by LAB, being converted to lactic acid, acetic acid, ethanol, and mannitol. During fermentation sucrose inversion took place, due to cotyledon invertase activity and/or induced acid hydrolysis as a result of acetic acid penetration into the beans upon fermentation, while glucose was preferentially fermented above fructose (see below) following sucrose hydrolysis. Although citrate has been mentioned as an important carbon source during cocoa bean fermentation, only few yeasts (e.g. *Pichia fermentans*) can assimilate citrate and of the dominant species such as *Candida krusei* found in Ghanaian cocoa bean heap fermentations, only some isolates are able to assimilate citrate within a reasonable time. This indicates that citrate assimilation was due to LAB development being favored at low pH values and it hence is considered as an important selective parameter among LAB strains for cocoa bean fermentation. *Leuconostoc* spp., *Enterococcus* spp., and *L. plantarum* metabolize citrate. This explains their survival in the first part of the cocoa bean heap fermentation. Although citrate consumption has been shown to enhance the growth of *Leuconostoc* spp. and not of *L. plantarum,* the latter species is more acid- and ethanol-tolerant than the former one, explaining its dominance against *Leuc. pseudomesenteroides* in the first part of the fermentation course. However, citrate consumption caused the pH of the pulp to increase from pH 3.5 to about pH 4.3, which is slightly lower than reported elsewhere, but important for the proteolysis stage of the fermentation. The production of succinic acid is ascribed to citrate-fermenting LAB as well, or to the conversion of fumaric and malic acids. The aciduric and ethanol-tolerant character of *L. fermentum* explains its survival and dominance of the whole cocoa bean fermentation process. In addition, strains of the species *L. plantarum* and *L. fermentum* are able to produce antimicrobial substances, contributing to bacterial ecology. The increased population of *L. fermentum* upon fermentation explains the simultaneous accumulation of mannitol (see below). Part of fructose that was used as alternative electron acceptor by LAB to produce mannitol could not be converted to nonvolatile lactic acid. Reversely, production of mannitol enabled the production of extra acetic acid and ATP, contributing to both volatile acidity (desired for cocoa beans) and competitive growth, respectively. Both physiological characteristics influence the quality of fermented cocoa beans. Finally, AAB, in particular *A. pasteurianus,* grew better on mannitol and lactate and converted ethanol into acetic acid.

As known free amino acids increased upon fermentation time, although they were present in lower amounts than reported elsewhere. However, the use of aqueous extracts in the present study reflects the bioavailability of nutrients for microbial fermentation of the pulp instead of their total extractable concentrations (important for further processing of the beans). Different patterns have frequently been observed for different amino acids. While the increase of hydrophobic free amino acids and hydrophilic oligopeptides is due to cocoa bean proteolytic activity, it is well known that LAB and AAB use (acid) free amino acids as carbon/nitrogen source and nitrogen source, respectively. In general, proteolysis primarily depends on the fermentation conditions, namely duration and intensity of acidification, temperature, and aeration. Also, oxidation, condensation, and complexation (with polypeptides) of polyphenols occur. Consequently, the fermentation conditions determine the amount of free amino acids, oligopeptides, reducing sugars, and polyphenols of fermented, dried cocoa beans, which all play an important role in aroma precursor formation that is further developed during cocoa processing. Our results indicate that a successful fermentation process is reached after about 72 h.

To conclude, the use of a mutiphasic approach as applied during this experiment increases the understanding of spontaneous food fermentation processes. The combination of both microbiological analyses, encompassing culture-dependent and ―independent methods, and metabolite analyses, encompassing fermentation and pure culture samples, allows to carefully profile population dynamics and fermentation courses. This approach permits the identification of specific populations and metabolites useful to improve fermentation (with respect to reproducible and standardized end-products and fermentation time) and organoleptic (with respect to reproducible consistency, color, flavor, and taste) profiles. Although cocoa bean fermentation is a heterogeneous process *per se,* the organoleptic quality of Ghanaian fermented cocoa beans is frequently reported as excellent. Yet, fermentation of cocoa beans depends on production methods, batch sizes, pod ripeness and storage, and fermentation conditions. As shown in this experiment particular competitive strains of both LAB and AAB dominate the Ghanaian cocoa bean heap fermentation process. With the purpose of selecting starter cultures for controlled cocoa bean fermentations, the results of the present experiment indicate that these prearbly are acid-tolerant, ethanol-tolerant, and citrate-utilizing strains in the case of LAB (preferably a combination of *Lb. plantarum* and *Lb. fermentum)*, and acid-tolerant, ethanol-tolerant, and heat-resistant strains in the case of AAB (preferably *A*. *pasteurianus*).

Summarised, the following conclusions can be drawn from the present experiment:
- biodiversity of both LAB and AAB in the Ghanaian cocoa bean heap fermentations analyzed was rather restricted, in contrast with a rich and varied yeast microbiota reported so far for (Ghanaian) cocoa bean fermentation;
- a clear three-phase fermentation process of a well ordered succession of microbial groups and a timely production of acids was difficult to recognize: yeast and LAB develop quasi simultaneously, development of AAB is somewhat delayed;
- yeasts are not responsible for citrate break down but for depectinisation and ethanol production;
- LAB are responsible for citrate break down and such LAB fermentation is important for the course of the cocoa bean fermentation, e.a. for proteolysis and for the production of (known) precursor molecules such as sugar, pyruvate carbolites and amino acid catabolites. Advantageously, larger amounts of such precursor molecules can be obtained when regulating the fermentation of cocoa in accordance with the present invention.
- AAB, and in particular *A. pasteurianus,* need lactate and mannitol for their development; they are responsible for acetic acid production and such AAB fermentation is important for the course of the cocoa bean fermentation, e.a. for ending the fermentation and for the production of precursor molecules, in particular acetate;
- there is a shift in bacterial ecology during fermentation, which can differ depending on the heap (see cluster analyses of the PCR-DGGE profiles); through the use of a starter culture (composition) as defined herein this microbial sucdession can be influences, steering the fermentation towards reproducible and desirable courses and end-products.
- there is a succession of bacterial species of LAB (first *Leuconostoc pseudomesenteroides* and *Lactobacillus plantarum,* followed by *Lactobacillus fermentum,* which can be explained based on the citrate break down, acid tolerance and ethanol tolerance) as well as of AAB (*Acetobacter pasteurianus* is the most important one, which can be explained based on acid tolerance, ethanol tolerance and heat resistance).
- new species, including "*Weissella ghanensis*", "*Acetobacter ghanensis"* (deposited as A. *syzygii-*like) and "*Acetobacter senegalensis*" (deposited as *A. tropicalis*-like) were isolated and they play a role together with other species such as *Leuc. pseudomesenteroides* and *E. casseliflavus* during cocoa bean fermentations.

### 3. Experiment 3

The following example illustrates a study from Ghanaian cocoa bean heap fermentations aimed at the unraveling of the influence of the environment on the microbial succession as well as the influence of turning. Concerning the former it is assumed that the dominant microorganisms that are responsible for cocoa fermentation are coming from the direct environment (e.g. cocoa pods). Concerning the latter it is assumed that turning will keep the fermenting cocoa bean mass more homogeneous, reducing temperature, pH, substrate, and metabolite gradients, and will allow more air penetration, possibly stimulating acetic acid bacteria development and hence the speed of fermentation. Therefore, six additional fermentations (heaps 8 to 13) were carried out during a third field experiment (main-crop 2005, October-December 2005). From heaps 8 and 9 all samples were lost and only the fermented, dried cocoa beans were available. Heaps 10 and 11 were performed in open air at the facilities of Barry Callebaut Ghana. Therefore, cocoa pods were transferred from the plantation in the neighbourhood of farmers 1 and 2 (see Experiment 2) to the company's facilities. At the company's facilities a heap was prepared with local banana/plantain leaves and the pods were opened with sterile machetes. Heaps 12 and 13 were performed at farmer 2 (as for heaps 2, 5, and 7 that were described in Experiment 2). Heaps 10 and 12 were turned twice (after 48 and 72 h of fermentation). For heap 13 the placenta was removed from the beans before fermentation was started. Microbiological and metabolite analyses were as described in Experiment 2.

From the results obtained, the following conclusions can be drawn:
**Plating, isolation, and rep-PCR Identification of LAB isolates** confirmed the restricted biodiversity of both LAB and AAB during Ghananian cocoa bean heap fermentations. *Lactobacillus plantarum* was found in heaps 8, 9, 10, and 13, *Lactobacillus fermentum* was picked up from all heaps, and *Enterococcus casseliflavus* was recovered from heap 9 at the start of the fermentation. Concerning AAB isolates, again *A. pasteurianus, A. syzygii-*like (*A*. *ghanensis*), and *A. tropicalis-*like (*A*. *senegalensis*) were found. It was remarkable that high counts of AAB were found from the beginning of heap fermentation 10 (turned, company's facilities), 11 (not turned, company's facilities), and 12 (turned, plantatation field). However, only in the fermentations with turning there was a considerable increase of AAB (upto 7.6 log) and a remarkable stabilization.
**PCR-DGGE of the lactic acid bacteria population** revealed the following data:
   - Heap 10 (turned, company's facilities): Only *Lb*. *plantarum* and *Lb*. *fermentum* were present. *Lb*. *fermentum* was present from the beginning of the fermentation and dominated the whole LAB fermentation process.
   - Heap 11 (not turned, company's facilities): Again *Lb*. *plantarum* and *Lb*. *fermentum* were dominating the fermentation process, although *Lb*. *fermentum* was less dominant compared with the fermentation where the heap was turned regularly. Its dominance was more pronounced after 36 h of fermentation. It can be assumed that turning favoured development of *Lb*. *fermentum*.
   - Heap 12 (turned, plantation field): '*W. ghanensis'* and *Lb*. *fermentum* were found, the latter being dominant throughout the fermentation. It can be assumed that turning favoured development of *Lb*. *fermentum* as compared to *Lb*. *plantarum.*
   - Heap 13 (not turned, without placenta, plantation field): Both *Lb*. *plantarum* and *Lb*. *fermentum* dominated the fermentation process during the first three days.
**Metabolite target analyses** gave the following results:
   - Heap 10 (turned, company's facilities): The course of citric acid and lactic acid was as seen before. The course of acetic acid resulted in higher peak concentrations of 16 mg/g in the pulp and 12 mg/g in the beans. Also, the maximum fermentation temperature was higher (47.6°C). Both coincide with a more pronounced development of AAB. Sugars were metabolized faster and a considerable amount of mannitol was formed from the beginning of the fermentation (10 mg/g in the pulp), indicating faster development of LAB as well, in particular of *Lb*. *fermentum.*
   - Heap 11 (not turned, company's facilities): The course of citric acid and lactic acid was as seen before. The amount of acetic acid produced was lower (5 mg/g in the pulp) as well as the maximum fermentation temperature (45°C), although the relatively high amounts of AAB present at the beginning of the fermentation. This indicates the importance of turning and hence AAB development. Sugars were metabolized slower and mannitol reached high concentrations (21.6 mg/g in the pulp) at a later stage of the fermentation.
   - Heap 12 (turned, plantation field): During this fermentation citric acid was consumed slowly and incomplete. Also, less lactic acid was formed (2 mg/g in the pulp and 0.67 mg/g in the beans). However, considerable amounts of acetic acid were formed (16 mg/g), corresponding with a high maximum fermentation temperature (47°C). This indicates that turning favours *A*. *pasteurianus,* the latter being more competitive in an acid and ethanol-rich environment, due to poor or no growth of *Lb*. *plantarum* and normal activities of yeast.
   - Heap 13 (not turned, without placenta, plantation field): This fermentation was characterized by a pronounced development of LAB, as reflected by the breakdown of citric acid in the beginning of the fermentation, the high amounts of lactic acid (4.6 mg/g in the pulp and 1.4 mg/g in the beans) and a lower maximum fermentation temperature of 42°C. It is assumed that emoval of the placenta is responsible for this, as this promotes more anaerobic conditions due to a better packing of the heap.
**Analyses of cultures of LAB cocoa bean isolates** (different strains as revealed by their (GTG)5 banding pattern) as to their capacity to ferment citrate revealed that:
   - All facultatively heterofermentative *Lb*. *plantarum* strains slightly fermented citrate and hence produced mainly lactic acid, while some strains appeared to be rather heterofermentative and fermented all citrate and hence produced equal amounts of lactic acid and acetic acid;
   - All heterofermenative *Lb*. *fermentum* strains fermented citrate and hence produced equal amounts of lactic acid and acetic acid, while some strains appeared facultatively heterofermentative and slightly fermented citrate and hence produced mainly lactic acid.

From the above it is clear that turning strongly influences microbial development and hence fermentation course, fermentation temperature (higher due to stronger development of acetic acid bacteria), and metabolite profiles. Also, the microbial environment appears to play a role.

In summary, we can conclude that microbial succession mainly depends on environmental factors such as pH of the heap, temperature of the heap, oxygen availability (turning versus non-turning) and sugar and citric acid availability, and in particular that:
- *Lactobacillus plantarum* (acid-resistant, ethanol-tolerant) is responsible for lactic acid production;
- *Lactobacillus fermentum* (acid-resistant, ethanol-tolerant) produces less lactic acid and is responsible for mannitol production.
- *Acetobacter pasteurianus* (acid-resistant, ethanol-tolerant, thermoresistant) is responsible for acetic acid production.

### Example 2: Metabolic characteristics of fermented cocoa beans obtainable by a fermentation method according to the present invention.

Example 2 illustrates results of metabolite analyses of fermented, dried cocoa beans, obtained by applying the seven heap fermentation processes as described in Experiment 2. In addition, this example also provides some further results of metabolite analyses of fermented, dried cocoa beans, obtained in additional heap fermentation processes, denoted as heap 8 to 13.

**Table 4** and **Table 5** represent a summary of the results of metabolite analyses performed on cocoa beans obtained from heaps 1 to 7, as described in Experiment 2

**Table 4**

| Heap | Acetic acid in dried beans | Lactic acid in dried beans | Citric acid in dried beans | Polyphenols in dry fat free beans | Caffeine in dry fat free beans | Theobromine in dry fat free beans |
|---|---|---|---|---|---|---|
| **1** | (5) | (4) | (3) | | | |
| **2** | (4) | (6) | Lowest (7) | | | |
| **3** | Lowest (7) | (2) | (6) | | | |
| **4** | (3) | Highest (1) | (5) | (2) | (2) | Highest (1) |
| **5** | (6) | (3) | (4) | Highest (1) | (3) | (2) |
| **6** | Highest (1) | (5) | (2) | (3) | Highest (1) | (3) |
| **7** | (2) | Lowest (7) | Highest (1) | Lowest (4) | Lowest (4) | Lowest (4) |

**TABLE 5**

| Heap | LAB present | AAB present | PCR-DGGE | Acetic acid pulp | Acetic acid beans | Lactic acid pulp | Lactic acid beans | Ethanol pulp | Ethanol beans | Citric acid pulp | Citric acid beans |
|---|---|---|---|---|---|---|---|---|---|---|---|
| | *L*. *mesenteroides:1* | | | Lowest (7) | Lowest (7) | (3) | (3) | (5) | (5) | (4) | (4) |
| | *L. pseudomesen:1* | *A.pasteurianus*:*23* | | | | | | | | | |
| **1** | *Lb. fermentum:19* | *A. syzygii: 6* | *Lb.fermentum* | | | | | | | | |
| | *Lb. plantarum: 16* | | | | | | | | | | |
| | *L. pseudomesen:1* | *A.pasteurianus:21* | *L. pseudomesen* | (4) | (3) | (4) | Highest (1) | Highest (1) | Highest (1) | Lowest (7) | Highest (1) |
| **2** | *Lb. fermentum:* 9 | *A. syzygii: 4* | *Lb. fermentum* | | | | | | | | |
| | *Lb. plantarum: 7* | *A. tropicalis: 2* | *Lb. plantarum* | | | | | | | | |
| | *L. pseudomesen:2* | | *L. pseudomesen* | Highest (1) | Highest (1) | (5) | (5) | Lowest (7) | Lowest (7) | (5) | (3) |
| **3** | *Lb. fermentum: 8* | *A. pasteurianus: 9* | *Lb. fermentum* | | | | | | | | |
| | *Lb. plantarum: 28* | | *Lb. plantarum* | | | | | | | | |
| | *L. pseudomesen:4* | | | (2) | (4) | (6) | (6) | (4) | (6) | (2) | (6) |
| | *Lb. fermentum: 6* | *A*. *pasteurianus: 7* | *L.pseudomesen* | | | | | | | | |
| **4** | *Lb. plantarum: 18* | *A. syzygii: 1* | *Lb. fermentum* | | | | | | | | |
| | *E. casseliflavus: 2* | *A. tropicalis: 1* | | | | | | | | | |
| | *Lb. mali: 2* | | | | | | | | | | |
| | *L. pseudomesen:6* | | | (5) | (5) | Highest (1) | (2) | (3) | (2) | (3) | (2) |
| | *Lb. fermentum:12* | *A.pasteurianus:21* | | | | | | | | | |
| **5** | *Lb. plantarum: 21* | *A. syzygii: 1* | *L.pseudomesen* | | | | | | | | |
| | *E. casseliflavus: 1* | | *Lb. fermentum* | | | | | | | | |
| | *Lb. brevis: 2* | | | | | | | | | | |
| | *Lb. fermentum:17* | *A.pasteurianus:10* | | (3) | (2) | (2) | (4) | (6) | (4) | (6) | (5) |
| **6** | *Lb. plantarum: 39* | *A. syzygii: 4* | *Lb. fermentum* | | | | | | | | |
| | *E. casseliflavus: 3* | *A. tropicalis: 5* | | | | | | | | | |
| | *New weisella: 2* | | | (6) | (6) | Lowest (7) | Lowest (7) | (2) | (3) | Highest (1) | Lowest (7) |
| | *Lb. fermentum: 4* | *A*. *pasteurianus:* 8 | *New weisella* | | | | | | | | |
| **7** | *Lb. plantarum: 15* | *A*. *syzygii: 7* | *Lb. fermentum* | | | | | | | | |
| | *L. pseudomesen:1* | *A*. *tropicalis: 1* | *L. pseudomesen* | | | | | | | | |
| | *E. casseliflavus: 4* | | | | | | | | | | |

**Table 6** illustrates results of quality tests performed on beans obtained in different fermentation processes, including analysis of the appearance of the beans and a cut test.

**Table 6**

| | **Heap 4** | | **Heap 5** | | **Heap 6** | | **Heap 7** | |
|---|---|---|---|---|---|---|---|---|
| Slaty | 0 | 0 | 0 | 0 | 0 | 0 | 0 | 0 |
| Violet | 15 | 16 | 6 | 7 | 13 | 16 | 9 | 7 |
| Infected | 0 | 0 | 0 | 0 | 1 | 0 | 0 | 0 |
| Flat | 7 | 7 | 7 | 8 | 14 | 13 | 9 | 8 |
| Clear | 0 | 0 | 1 | 0 | 0 | 0 | 0 | 0 |
| Bean count(/300 g) | 253 | | 286 | | 262 | | 273 | |
| Broken (g) | 2.39 | | 1.95 | | 1.59 | | 3.43 | |

In the above given table, slaty refers to beans with a grayish color; violet refers to beans with a purple color, infected refers to beans showing spores of insect or worm infestation, flat refers to empty beans, clear refers to beans which are ochre, i.e. more yellow than dark brown, the bean count refers to the number of beans in a sample of 300 grams, and the term broken refers to the number of beans that are broken in the sample taken for the bean count.

**Table 7** illustrates compositions of cocoa beans which have been submitted to 13 different heap fermentation processes.

**Table 7**

| **Heap** | **Fat content** | **polyphenols (%)** | **epicathechin (mg/g)** | **catechin (mg/g)** | **theobromin (%)** | **Caffeine (%)** |
|---|---|---|---|---|---|---|
| **1** | 53.02 | 3.09 | 1.02 | 0.07 | 0.98 | 0.11 |
| **2** | 55.04 | 2.99 | 0.75 | 0.06 | 0.99 | 0.13 |
| **3** | 53.37 | 2.41 | 0.51 | 0.06 | 0.93 | 0.10 |
| **4** | 56.37 | 2.69 | 1.18 | 0.10 | 1.09 | 0.09 |
| **5** | 56.17 | 2.20 | 0.50 | 0.07 | 1.08 | 0.08 |
| **6** | 54.84 | 2.42 | 0.70 | 0.06 | 1.06 | 0.09 |
| **7** | 55.80 | 1.97 | 0.46 | 0.04 | 1.00 | 0.08 |
| **8** | 55.97 | 3.21 | 1.41 | 0.09 | 1.09 | 0.07 |
| **9** | 55.53 | 3.23 | 1.63 | 0.07 | 1.16 | 0.09 |
| **10** | 57.30 | 3.54 | 1.10 | 0.08 | 1.05 | 0.09 |
| **11** | 56.40 | 3.26 | 1.46 | 0.09 | 1.12 | 0.09 |
| **12** | 54.24 | 2.35 | 1.31 | 0.08 | 0.99 | 0.07 |
| **13** | 53.82 | 3.46 | 1.48 | 0.06 | 1.00 | 0.08 |

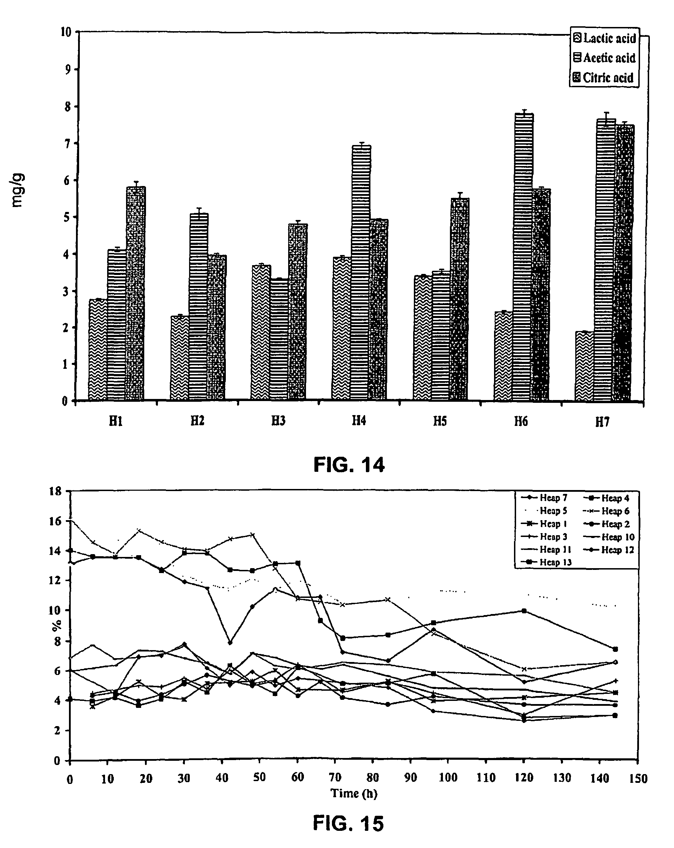

**Figure 14** illustrates the amounts of acid metabolites including lactic acid, acetic acid and citric acid present in dried beans obtained in different heap fermenting processes (heap 1 to 7).

**Figure 15** illustrates the amounts of polyphenols present in dried beans obtained in different heap fermenting processes (heap 1 to 7, and 10-13). A difference is hereby noted between the polyphenols content of heaps 4, 5, 6, and 7 on the one hand and the other heaps on the other hand.

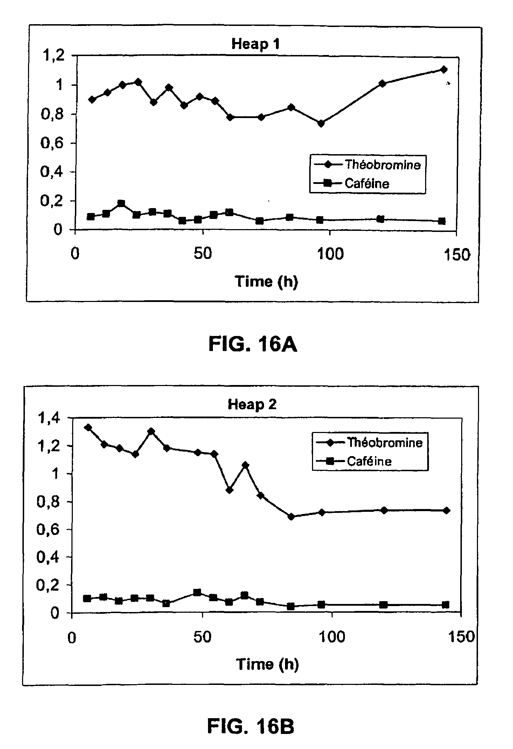

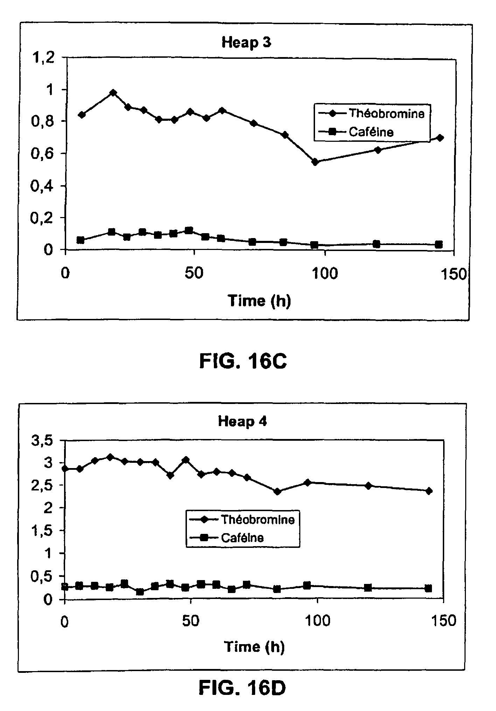

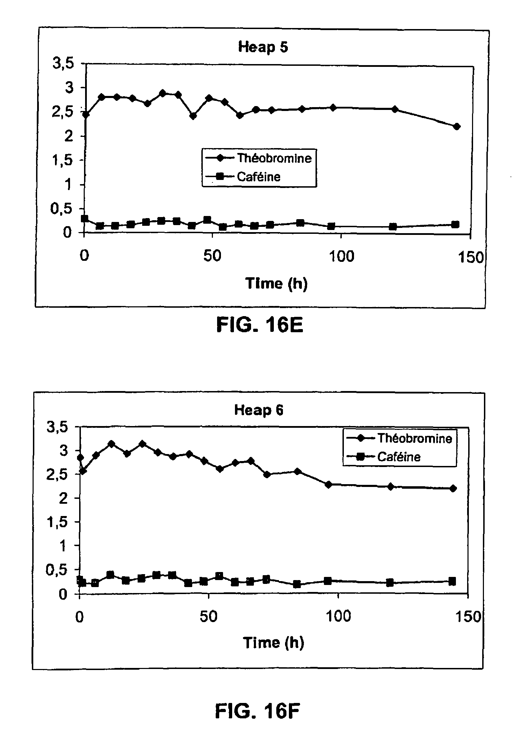

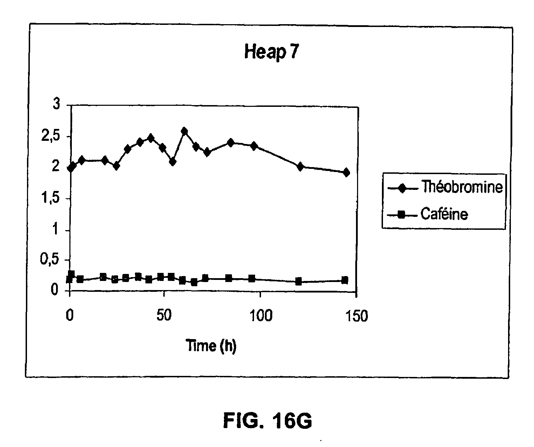

**Figure 16** further illustrates the percentual amounts of caffeine and theobromine present in dried beans obtained in different heap fermenting processes (heap 1 to 7).

### Example 3: Metabolic characteristics of chocolate products obtainable by using cocoa beans that have been fermented according to a method of the present invention.

Example 3 illustrates results of taste and metabolite analyses of several chocolate products. These chocolate products have been obtained by roasting cocoa beans that have been fermented in seven heap fermentation processes as described in Experiment 2. The beans were roasted at a temperature of 130-140°C during 30 min, and then conched at 50°C during 2 h. The prepared chocolate products contained (in weight %): 42% cocoa liquor, 11.37% cocoa butter, 46% sugar, 0.6% lecithin, and 0.03% vanillin.

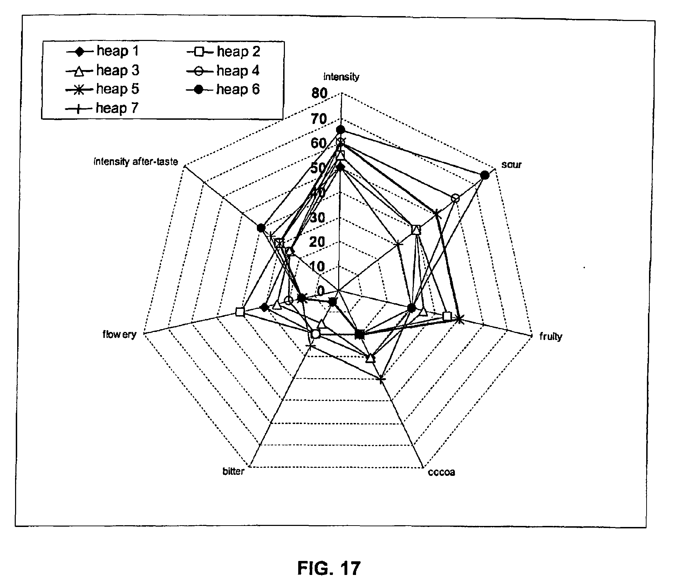

**Table 8** illustrates taste properties of the obtained chocolates as determined by a taste panel. **FIG. 17** provides a visual representation of the tastes as determined for the different chocolates indicated in **table 8**.

**Table 8**

| **Taste Panel** | **Heap1** | **Heap2** | **Heap3** | **Heap4** | **Heap5** | **Heap6** | **Heap7** |
|---|---|---|---|---|---|---|---|
| **Intensity** | 50 | 55 | 55 | 60 | 60 | 65 | 50 |
| **Sour** | 40 | 40 | 40 | 60 | 50 | 75 | 30 |
| **Fruity** | 30 | 45 | 35 | 30 | 50 | 30 | 30 |
| **Cocoa** | 30 | 20 | 30 | 20 | 20 | 20 | 40 |
| **Bitter** | 20 | 20 | 15 | 20 | 5 | 5 | 25 |
| **Flowery** | 30 | 40 | 25 | 20 | 15 | 15 | 15 |
| **Intensity after-taste** | 25 | 30 | 25 | 25 | 30 | 40 | 35 |
| **After-taste** | bitter | flowery | | slightly flowery | fruity | lemon acid | inpure cocoa |

The tastes noted for the different chocolates were as follows:
for heap 1: Very sour, slightly caramel, almost no pure cocoa-taste;
for heap 2: Fruity, flowery, no sour;
for heap 3: Less fruity, less flowery than 2, no sour;
for heap 4: Less sour than 1, but still very sour;
for heap 5: Very slightly smoky (especially in the after-taste), slightly sour;
for heap 6: Slightly sour, no fruity or flowery- taste; and
for heap 7: Slightly impure, slightly sour, no clear cocoa-taste.

From the present results it can be concluded that:
- The flavour of chocolate derived from cocoa beans of heap 6 is the most sour and intense. This can be correlated with the results obtained by chromatography (Experiment 2). The production of lactic acid and acetic acid is very high in this fermentation. Also, the reached fermentation temperature is the highest of the seven fermentations; e.g. ethanol converted into acetic acid, energy release, almost all ethanol was converted.
- Cocoa beans of heap 7 provide chocolate with the most cocoa taste, and has overall the best qualities to produce a good chocolate. It is the heap with the smallest amount of lactic acid present in the dried beans. However, on the other hand these beans have the lowest amounts of polyphenols present.
- The flavour of chocolate derived from cocoa beans of fermentation heap 2 is flowery and fruity (yeasts). Production of ethanol is the highest in this heap, which indicates that many yeasts were present. On the other hand, production of acetic acid and lactic acid is very low. The reached fermentation temperature was the lowest of all fermentations. Fermentation in heap 2 is a typical yeast fermentation, which is not desired.
- The flavour of chocolate derived from cocoa beans of fermentation heap 5 was characterized by a fruity, less sour, and intense taste. It is assumed that the development of *Lb*. *fermentum* (producing more mannitol and less lactic acid), the lower densities of *Lb*. *plantarum* and the prevalence of the more aciduric '*W*. *ghanensis'* is reponsible for this.

It can be concluded from the present results that there is a direct correlation between the presence of certain AAB and LAB during fermentation of the cocoa beans and the sour flavor in chocolate products derived from such cocoa beans. Also, there is a direct correlation between the presence of certain yeasts during fermentation of the cocoa beans and the fruity flavor in chocolate products derived from such cocoa beans.

### Example 4: Development of a (GTG)₅₋rep-PCR fingerprinting technique for rapid identification, classification and typing of acetic acid bacteria, with a focus on isolates from Ghanaian fermented cocoa beans

Rep-PCR fingerprinting of DNA using the (GTG)₅ primer, referred to as (GTG)₅-PCR fingerprinting, is a promising genotypic tool for rapid and reliable speciation and typing of acetic acid bacteria (AAB). The method was evaluated with 64 AAB reference strains, including 31 type strains, and 132 isolates from Ghanaian, fermented cocoa beans, and was validated with DNA:DNA hybridization data. Most reference strains grouped according to their species designation, and exclusive patterns were obtained for most strains, indicating the usefulness of this technique for identification to the species level and characterization below species level or typing of AAB strains. The (GTG)₅-PCR fingerprinting allowed us to differentiate four major clusters among the fermented cocoa bean isolates, namely *A*. *pasteurianus* (cluster 1, 100 isolates), *A. syzygii-* or *A*. *lovaniensis-*like (cluster II, 23 isolates), and *A*. *tropicalis-*like (clusters III and IV containing 4 and 5 isolates, respectively). *A syzygii-*like and *A. tropicalis-*like strains were isolated from cocoa bean fermentations for the first time. Validation of the method and indications for existence of new *Acetobacter* species were obtained through 16S rRNA sequencing analyses and DNA:DNA hybridizations.

### MATERIALS AND METHODS

Strains and growth conditions. Two sets of AAB were included in this study. A first group consisted of AAB reference strains (64 in total, including 31 type strains) These AAB were obtained, together with the type strain of *Lactobacillus plantarum* LMG 6907^{T}, from the BCCM/LMG Bacteria Collection (Ghent University, Gent, Belgium). All reference strains were grown according to the provider's specifications (hftp://www.belspo.be/bccm/), unless indicated otherwise. Tested strains consisted of 132 AAB isolates from seven traditional heap fermentations of cocoa beans performed during the main- and mid-crop of 2004 in Ghana. They were maintained frozen at -80°C in MYP medium (2.5% [wt/vol] *D*-mannitol, 0.5% [wt/vol] yeast extract, and 0.3% [wt/vol] bacteriological peptone [Oxoid, Basingstoke, UK]), supplemented with 25 % (vol/vol) glycerol as cryoprotectant, and recovered by incubation at 30°C in MYP medium under aerobic conditions for 1-4 days.

**Phenotypic tests**. All isolates were tested for their Gram reaction, cell shape, cell size, and mobility, from cells grown at 30°C in MYP medium under aerobic conditions for 1-4 days. Catalase activity was detected by the appearance of oxygen gas bubbles from 20% (vol/vol) hydrogen peroxide solution by colonies on MYP agar (MYP medium supplemented with 1.5% agar, wt/vol). The oxidase test was performed using the Oxidase DrySlide test kit (Becton Dickinson, Cockeysville, MD). *Lactobacillus plantarum* LMG 6907^{T} (catalase-negative, oxidase-negative lactic acid bacterium) and *Acetobacter aceti* LMG 1504^{T} (catalase-positive, oxidase-negative acetic acid bacterium) were used as controls.

Cellular fatty acids from a subset of 63 isolates, obtained from three cocoa bean fermentations performed during the mid-crop of 2004, with the aim to evaluate the selectivity of the Deoxycholate-Mannitol-Sorbitol medium used for their isolation, as well as from five reference strains (*A*. *aceti* LMG 1261^{T}, *A. lovaniensis* LMG 1579^{T}, *A. orleanensis* LMG 1583^{T}, *A. pasteurianus* LMG 1262^{T}, and *G. oxydans* LMG 1408^{T}), were analyzed using the Microbial Identification system of MIDI (Netwark, DE). Therefore, bacteria were grown on MYP agar at 28°C for 24 h. Instructions of the manufacturer were followed exactly for fatty acid extraction and analysis. *Stenotrophomonas maltophilia* LMG 958^{T} was used as positive control.

The production of acetic acid from ethanol and gluconic acid from glucose were tested following growth of the strains at 30°C for 4 days in basal medium [0.05% yeast extract and 0.3% vitamin-free casamino acids (Difco, Detroit, MI); wt/vol] plus ethanol (0.3%, wt/vol) and GY medium (5% *D*-glucose and 0.5% yeast extract, wt/vol), respectively. The production of 2-and 5-keto-gluconic acid from ethanol and glucose was tested following growth in basal medium plus ethanol and GY medium, respectively, at 30°C for 4 days. Residual substrates and metabolites were quantified by high pressure liquid chromatography (HPLC) with refractive index detection for glucose and ethanol, and high pressure anion exchange chromatography (HPAEC) with ion suppression and conductivity detection for acetic acid, gluconic acid, 2-keto-gluconic acid, and 5-keto-gluconic acid.

All tests were done in duplicate.

**DNA preparation.** Total genomic DNA was extracted from the reference strains by the method of Wilson (1987, p. 2.4.1.-2.4.5. In Ausubel et al. (ed.), Current Protocols in Molecular Biology. Green Publishing and Wiley-Interscience, New York, NY.), with minor modifications (Cleenwerck et al., 2002; Int. J. Syst. Evol. Microbiol. 52:1551-1558). For the isolates it was extracted as described by Gevers et al. (2001; FEMS Microbiol. Lett. 205:31-36), except that mutanolysin was substituted by proteinase K (VWR International, Darmstadt, Germany) in an amount of 0.0025 g per ml of Tris-HCl (10 mM)-EDTA (1 mM) buffer (pH 8.0). All isolates were grown in MYP medium for 1-4 days, except for some strains that grew better in GY medium. *Asaia siamensis* and *Asaia bogorensis* were grown in M17 medium (5% glucose, 3% calcium carbonate, 1% yeast extract, wt/vol).

**rep-PCR genomic fingerprinting**. A set of 64 reference strains and 132 isolates was subjected to rep-PCR fingerprinting with the oligonucleotide primer pair REP1R-I (5'-IIIICGICGICATCIGGC-3') (SEQ ID NO: 8) and REP2-I (5'-IIICGNCGNCATCNGGC-3') (SEQ ID NO: 9) and with the single oligonucleotide primer (GTG)₅ (5'-GTGGTGGTGGTGGTG-3') (SEQ ID NO: 10). The reproducibility of (GTG)₅-PCR was tested by amplifying DNA from twelve randomly chosen strains several times. In addition, each PCR reaction was controlled for reproducibility by inclusion of the type strain of *L*. *plantarum* LMG 6907^{T}. Optimal PCR conditions for each of the primer sets used were as described by Versalovic et al. (1994; Meth. Mol. Cell. Biol. 5:25-40). PCR amplifications were performed with a DNA T3 thermal cycler (Biometra, Westburg, The Netherlands) using Taq DNA polymerase (Roche Diagnostics GmbH, Mannheim, Germany). The PCR products were electrophoresed in a 1.5% (wt/vol) agarose gel (15x20 cm) for 16 h at a constant voltage of 55 V in 1 x TAE buffer (40 mM Tris-Acetate, 1 mM EDTA, pH 8.0) at 4°C. The rep-PCR profiles were visualized under UV light after staining of the gel with ethidium bromide, and digital image capturing was done using the Gel Doc EQ system (Biorad, Hercules, CA). The resulting fingerprints were analyzed using the BioNumerics version 4.0 software package (Applied Maths, Sint-Martens-Latem, Belgium). The similarity among digitized profiles was calculated using the Pearson correlation, and an average linkage (UPGMA or unweighted pair group method with arithmetic averages) dendrogram was derived from the profiles.

**DNA:DNA hybridizations**. Only high-molecular-mass DNA with A₂₆₀/A₂₈₀ and A₂₃₄/A₂₆₀ absorption (A) ratios of 1.8-2.0 and 0.40-0.60, respectively, was used for DNA:DNA hybridizations. DNA:DNA hybridizations were performed using a modification of the microplate method described by Ezaki et al. (1989; lnt. J. Syst. Evol. Microbiol. 39:224-299) (Goris et al., 1998 Syst. Appl. Microbiol. 4:338-368; Cleenwerck *et al*., 2002). The hybridization temperature was 49 ± 1°C. Reciprocal reactions (e.g. AxB and BxA) were performed and their variation was within the limits of this method (Goris et al., 1998 Can. J. Microbiol. 44:1148-1153). The DNA binding values reported are the mean of a minimum of four hybridization experiments, including the reciprocal reactions.

**16S rRNA sequencing**. A fragment of the 16S rRNA gene (corresponding with the positions 8-1541 in the *Escherichia coli* numbering system) was amplified by PCR using the conserved primers pA (5' AGA GTT TGA TCC TGG CTC AG 3') (SEQ ID NO: 11) and pH (5' AAG GAG GTG ATC CAG CCG CA 3') (SEQ ID NO: 12). PCR-amplified 16S rRNA genes were purified using the NucleoFast^{®} 96 PCR Cleanup Kit (Macherey-Nagel, Düren, Germany). Sequencing reactions were performed using the BigDye^{®} Terminator Cycle Sequencing Kit (Applied Biosystems, Foster City, CA) and purified using the Montage™ SEQ₉₆ Sequencing Reaction Cleanup Kit (Millipore, Bedford, MA). Sequencing was performed using an ABI Prism^{®} 3100 Genetic Analyzer (Applied Biosystems). Nearly complete sequences were determined using the eight sequencing primers listed in Coenye et al. (1999; Int. J. Syst. Bacteriol. 49:405-413). Sequence assembly was performed using the program AutoAssembler (Applied Biosystems). Sequence similarities between the consensus sequences and small ribosomal subunit sequences collected from the international nucleotide sequence library EMBL (EMBL-EBI, Hinxton, Cambridge, UK) were calculated pairwise using an open gap penalty of 100% and a unit gap penalty of 0% with the software package BioNumerics version 4.5 (Applied Maths).

### RESULTS

### Phenotypic characterization of Isolates from Ghanaian, fermented cocoa beans

Cells of all isolates recovered from DMS (78%) were motile or non-motile rods, Gram-negative, and catalase-positive, with the ability to oxidize ethanol to acetic acid as revealed by HPLC. Their oxidase activity through the oxidase test was difficult to interpret due to poor growth during this test procedure. Identification of cellular fatty acids performed on some reference strains as well as on a subset of fermented cocoa bean isolates revealed that the predominant fatty acid found in all strains tested was the straight-chain, unsaturated C18:1ω7c fatty acid, which accounted for approximately 50% of the fatty acid content (data not shown). Other fatty acids common to all isolates were C16:0, C16:0 2OH (except strain 113C), C14:0 2OH (except strains 120B and 106A), C14:0 (except strains 120B, 106A, 121, 109,119, 103, 129, 151, 154, 150, 158, 165B, 105B, 117, 114F, and 113C) and C19:0 cyclo ω8c (except strain 106A). All isolates grew in basal medium plus ethanol and in GY medium. They all oxidized ethanol to acetic acid and produced gluconic acid from glucose in the respective media. Most of the isolates produced 2-keto-gluconic acid from glucose in very small amounts and did not produce 5-keto-gluconic acid. Based on these phenotypic results and the fact that they were derived from fermented cocoa beans, the isolates could be identified as *Acetobacter* spp.

### Rep-PCR genomic fingerprinting: method development

The (GTG)₅ primer as well as the REP1R-I and REP2-I primer set generated DNA fragments of 300 to 4000 bp. The (GTG)₅ primer resulted in banding patterns containing generally between 10 and 30 visualized PCR products, while the REP1R-I and REP2-I primer set generated banding patterns containing generally less than 10 bands. As the goal of this study was the development of a rapid method for identification as well as typing, the (GTG)₅ primer was thus preferred for further evaluation. The similarity between the (GTG)₅-PCR patterns, obtained through amplification of DNA from twelve strains several times, ranged between 89 and 96% (results not shown). The inclusion of the type strain of *L*. *plantarum* LMG 6907^{T} in each PCR reaction and banding pattern maintained a high reproducibility.

### Identification, classification, and typing of AAB with (GTG)₅-PCR fingerprinting

***Reference strains.*** Almost all reference strains grouped in separate clusters according to their respective taxonomic designations. Comparing the results obtained by (GTG)₅-PCR fingerprinting with the DNA:DNA hybridization results, it can be noticed that in most cases strains that did not cluster with their respective taxa showed less or approximately 70% DNA homology, the generally accepted limit for species delineation (Stackebrandt et al., 2002), with the other strains from the taxon they were classified in.

***Fermented cocoa bean isolates.*** To evaluate the applicability of (GTG)₅-PCR for identification of unknown isolates, 132 fermented cocoa bean isolates, assigned to the genus Acetobacter on the basis of their phenotypic characteristics, were subjected to (GTG)₅-PCR fingerprinting. The (GTG)₅-PCR banding patterns of the isolates clustered with those of the AAB reference strains and were found to be dispersed over four major clusters. The biggest group of isolates (cluster I, containing 100 isolates) clustered with *A. pasteurianus* reference strains and was therefore assigned to that species. The 32 remaining isolates were dispersed over three clusters, with 23 (cluster II), 4 (cluster III), and 5 (cluster IV) isolates, respectively. These clusters did not contain any of the reference strains and therefore these strains could not be identified to species level.

Isolates 150, 406, and 165D of cluster I were subjected to 16S rRNA sequencing analysis and DNA:DNA hybridizations to test the validity of their identification as *A. pasteurianus.* They showed 99.5-99.8% 16S rRNA sequence similarity with the type strains of *A*. *pomorum* and *A. pasteurianus,* and less than 98.1% with the type strains of other *Acetobacter* species (the EMBL accession numbers for the 16S rRNA sequences of isolates 150, 406, and 165D are DQ887334, DQ887335, and DQ887336, respectively) and a DNA relatedness at species level amongst each other and with the type strain of *A. pasteurianus* of 68-80% and below species level with the type strain of *A. pomorum* of 42-47%. These results prove that cluster I consisted of *A. pasteurianus* strains.

To verify if clusters II, III, and IV represented possible new AAB species, representative isolates of each of them were subjected to 16S rRNA sequence analysis and the phylogenetic closest neighbors were determined. The 16S rRNA sequence similarities obtained showed that isolates 444B and 384 (cluster II) were most closely related to *A*. *syzygii* (99.7%) and *A*. *lovaniensis* (99.5%), while isolates 108B and 420A (cluster III) and isolates 434 and 426 (cluster IV) were most closely related to *A. tropicalis* (99.9%). The EMBL accession numbers for the 16S rRNA sequences of isolates 444B, 384, 108B, 420A, 434, and 426 are DQ887337-DQ887340.

DNA:DNA hybridizations between isolate 444B and *A*. *syzygii* LMG 21419^{T} and *A. lovaniensis* LMG 1617^{T} revealed a DNA homology value of 46% and 47%, respectively. DNA:DNA hybridizations between isolates 108B, 420A, and *A*. *tropicalis* LMG 19825^{T} revealed a high DNA homology value between both isolates (75%) and intermediate values between these isolates and *A*. *tropicalis* LMG 19825^{T} (54-58%).

### DISCUSSION

In the present example, usefulness of the (GTG)₅-rep-PCR fingerprinting technique was tested with reference strains of most of the species of AAB and it was found that it generated the most discriminative and complex banding patterns in comparison with the REP primer pair. Most reference strains grouped according to their species designation and exclusive patterns were obtained for most strains, indicating the usefulness of this technique for identification to the species level and characterization below species level or typing of AAB strains.

To evaluate the (GTG)₅-PCR technique for species identification, (GTG)₅-PCR DNA fingerprinting data were compared with DNA:DNA hybridization data. The latter data are recognized as the data needed for species delineation and for accurate identification of *Acetobacter* and *Gluconacetobacter* strains. Perfect matches occurred between these data and the (GTG)₅-PCR clusters, the clustering being in line with the 70% DNA relatedness limit, which is generally accepted for species delineation (Stackebrandt et al., 2002). The results presented in this example thus show that (GTG)₅-PCR DNA fingerprinting is useful for identification and classification of AAB to the species level.

In many cases, the (GTG)₅-PCR fingerprints generated for AAB were strain-specific and (GTG)₅-PCR fingerprinting can therefore be used for typing of AAB as well. The (GTG)₅-PCR fingerprinting allowed to differentiate four major clusters among the 132 isolates from fermented cocoa beans tested, with decreasing number of isolates encompassing *A*. *pasteurianus* (cluster I), *A*. *syzygii-* or *A. lovaniensis*-like (cluster II), and *A. tropicalis-like* (clusters III and IV), respectively. *A syzygii* and *A*. *tropicalis* have never been associated with cocoa bean fermentation. Moreover, *A*. *syzygii* mainly occurs in flowers and fruits and is seldom isolated from fermented foods. Results of this example indicate that clusters II, III, and IV represent three novel *Acetobacter* species.

To summarize, as far as we know this is the first study in which a fast molecular method for AAB was validated by DNA:DNA hybridization data. The (GTG)₅-PCR fingerprinting technique presented in this example offers significant advantages over identification methods based on phenotypic characteristics of AAB, but also over many currently used molecular techniques, hence enabling its implementation as high-throughput methodology. Manual (GTG)₅-PCR fingerprinting allows identification of AAB in one working day and can be used for classification and identification of AAB at the species level, as well as for characterization of AAB below species level (typing). (GTG)₅-PCR fingerprinting can quickly increase knowledge of the ecology of AAB and help to more accurately determine their growth behavior and beneficial or undesirable role during various stages of food fermentation such as cocoa bean fermentation.

## Claims

1. A method for regulating fermentation of plant material comprising beans and/or pulp derived from fruit pods of the species *Theobroma* cacao, said method comprising adding to the plant material a composition comprising at least one strain of lactic acid bacteria and/or at least one strain of acetic acid bacteria.

2. The method according to claim 1, wherein said composition comprises at least one strain of lactic acid bacteria and at least one strain of acetic acid bacteria.

3. The method according to any one of claims 1 or 2, wherein said composition is added at the start of the fermentation.

4. The method according to claim 1, wherein one or more compositions comprising one or more same or different strains of lactic acid bacteria and/or one or more same or different strains of acetic acid bacteria are added at the start of the fermentation and/or during the first 24 hours of the fermentation and/or after 24 hours of the fermentation.

5. The method according to claim 4, wherein any one or more or all of said one or more compositions comprise at least one strain of lactic acid bacteria and at least one strain of acetic acid bacteria.

6. The method according to any one of claims 1 to 5, wherein the at least one strain of lactic acid bacteria belongs to a species of a genus chosen from the group comprising *Bifidobacterium, Brevibacterium, Carnobacterium, Enterococcus, Lactobacillus, Lactococcus, Leuconostoc, Oenococcus, Pediococcus, Propionibacterium, Streptococcus, Tetragenococcus, Vagococcus* and *Weissella,* preferably wherein the at least one strain of lactic acid bacteria belongs to a species chosen from the group comprising *Lactobacillus acidophilus, Lactobacillus brevis, Lactobacillus buchneri, Lactobacillus casei, Lactobacillus casei* subspecies *pseudoplantarum, Lactobacillus collinoides, Lactobacillus cellobiosus, Lactobacillus delbrueckii, Lactobacillus fermentum, Lactobacillus fructivorans, Lactobacillus gasseri, Lactobacillus hilgardii, Lactobacillus kandleri, Lactobacillus lactis, Lactobacillus mali, Lactobacillus parabrevis, Lactobacillus paraplantarum, Lactobacillus pentosus, Lactobacillus plantarum, Lactococcus lactis, Leuconostoc carnosum, Leuconostoc durionis, Leuconostoc mesenteroides, Leuconostoc oenos, Leuconostoc paramesenteroides, Leuconostoc pseudomesenteroides, Oenococcus oeni, Pediococcus acidilactici, Pediococcus cerevisiae, Pediococcus dextrinicus, Weissella cibaria, Weissella confusa, Weissella kimchii, Weissella paramesenteroides, Enterococcus casseliflavus, Enterococcus columbae,* and *Enterococcus faecium,* more preferably wherein the at least one strain of lactic acid bacteria belongs to a species chosen from the group comprising *Lactobacillus fermentum, Lactobacillus plantarum, Leuconostoc pseudomesenteroides, Enterococcus casseliflavus,* and *Weissella ghanensis,* said *Weissella ghanensis* corresponding to an isolate deposited with the Belgian Coordinated Collections of Microorganisms (BCCM) under accession number LMG P-23179, or a mutant or variant of said isolate.

7. The method according to claim 6, wherein the at least one strain of lactic acid bacteria belongs to a species chosen from the group comprising *Lactobacillus fermentum* and *Lactobacillus plantarum,* and preferably is *Lactobacillus fermentum.*

8. The method according to any one of claims 1 to 7, wherein the at least one strain of acetic acid bacteria belongs to a species of a genus chosen from the group comprising *Acetobacter, Acidiphilium, Acidocella, Acidomonas, Craurococcus, Gluconacetobacter, Gluconobacter, Paracraurococcus, Rhodopila, Roseococcus, Stella, Zavarzinia, Asaia, Kozakia, Neoasia, Saccharibacter* and *Swaminathania,* preferably wherein the at least one strain of acetic acid bacteria belongs to a species chosen from the group comprising *Acetobacter rancens, Acetobacter xylinum, Acetobacter ascendens, Acetobacter lovaniensis, Acetobacter aceti, Acetobacter aceti* subsp. *liquefaciens, Acetobacter cerevisiae, Acetobacter cibinongensis, Acetobacter estunensis, Acetobacter indonesiensis, Acetobacter lovaniensis, Acetobacter malorum, Acetobacter oeni, Acetobacter orientalis, Acetobacter orleaniensis, Acetobacter pasteurianus, Acetobacter peroxydans, Acetobacter pomorum, Acetobacter suboxydans, Acetobacter roseum, Acetobacter syzygii, Acetobacter tropicalis, Acidomonas methanolica, Asaia bogorensis, Asaia siamensis, Gluconacetobacter azotocaptans, Gluconacetobacter diazotrophicus, Gluconacetobacter europaeus, Gluconacetobacter hansenii, Gluconacetobacter intermedius, Gluconacetobacter johannae, Gluconacetobacter liquefaciens, Gluconacetobacter oboediens, Gluconacetobacter rhaeticus, Gluconacetobacter sacchari, Gluconacetobacter swingsii, Gluconacetobacter xylinus, Gluconobacter oxydans,* and *Gluconobacter oxydans* subsp. *suboxydans*, more preferably wherein the at least one strain of acetic acid bacteria belongs to a species chosen from the group comprising *Acetobacter pasteurianus*, *Acetobacter syzygii*, *Acetobacter tropicalis, Acetobacter ghanensis* and *Acetobacter senegalensis*, said *Acetobacter ghanensis* corresponding to an isolate deposited with the Belgian Coordinated Collections of Microorganisms (BCCM) under accession number LMG P-23177, or a mutant or variant of said isolate, and said *Acetobacter senegalensis* corresponding to an isolate deposited with the Belgian Coordinated Collections of Microorganisms (BCCM) under accession number LMG P-23176, or a mutant or variant of said isolate.

9. The method according to claim 8, wherein the at least one strain of acetic acid bacteria belongs to the species *Acetobacter pasteurianus.*

10. The method according to any one of claims 1 to 9, wherein said composition comprises at least one strain of *Lactobacillus fermentum* and at least one strain of *Acetobacter pasteurianus,* and optionally and preferably further comprises at least one strain of *Lactobacillus plantarum.*

11. The method according to any one of claims 1 to 10, wherein said composition further comprises at least one strain of yeast, preferably wherein the at least one strain of yeast belongs to a species of a genus chosen from the group comprising *Arxiozyma, Brettanomyces, Candida, Citeromyces, Cryptococcus, Debaryomyces, Dekkera, Geotrichum, Hanseniaspora, Hansenula, Holleya, Issatchenkia, Kloeckera, Kluyveromyces, Kodameae, Lodderomyces, Pichia, Rhodotorula, Saccharomyces, Saccharomycopsis, Saturnispora, Schizosaccharomyces, Scytaladium, Starmera, Tetrapisispora, Torulaspora, Torulopsis, Trichosporon, Williopsis* and *Zygosaccharomyces,* more preferably wherein the at least one strain of yeast belongs to a species chosen from the group comprising *Candida bombi, Candida pelliculosa, Candida rugopelliculosa, Candida rugosa, Candida intermedia, Candida krusei, Candida parapsilosis, Candida quercitrusa, Candida silvicola, Candida stellimalicola, Candida boidinii, Candida cacoai, Candida guilliermondii, Candida reukaufii, Candida amapae, Candida diddensiae, Candida friedrichii, Candida humicola, Candida mycoderma, Candida neodendra, Candida tammaniensis, Candida tropicalis, Candida valida, Candida zemplinina, Geotrichum candidum, Kloeckera apiculata, Kloeckera africana, Kloeckera corticis, Kloeckera javanica, Kluyveromyces marxianus, Kluyveromyces thermotolerans, Lodderomyces elongisporus, Pichia fermentans, Saccharomyces cerevisiae, Saccharomyces cerevisiae* var. *chevalieri, Torulaspora pretoriensis, Cryptococcus humicola, Cryptococcus laurentii, Hanseniaspora guilliermondii, Hanseniaspora opuntiae, Pichia galeiformis, Pichia guilliermondii, Pichia kluyveri, Pichia membranifaciens, Rhodotorula glutinis, Rhodotorula rubra, Trichosporon asahii, Brettanomyces clausenii, Kloeckera apis, Schizosaccharomyces malidevorans, Issatchenkia hanoiensis, Saccharomycopsis vini* or *Scytaladium hyalinum,* and preferably *Candida bombi, Candida pelliculosa, Candida rugosa, Candida intermedia, Candida krusei, Candida cacoai, Candida guilliermondii, Pichia fermentans, Pichia guilliermondii, Pichia membranifaciens, Hanseniaspora guilliermondii, Hanseniaspora opuntiae, Saccharomyces cerevisiae, Kluyveromyces marxianus,* and *Kluyveromyces thermotolerans,* even more preferably wherein the at least one strain of yeast belongs to a species chosen from the group comprising *Candida krusei, Pichia membranifaciens, Hanseniaspora guilliermondii, Kluyveromyces marxianus,* and *Saccharomyces cerevisiae.*

12. A composition suitable for regulating fermentation of plant material comprising beans and/or pulp derived from fruit pods of the species *Theobroma cacao,* such as a composition which is a starter culture or a high density culture, said composition comprising at least one strain of lactic acid bacteria and/or at least one strain of acetic acid bacteria, preferably comprising at least one strain of lactic acid bacteria and at least one strain of acetic acid bacteria, and optionally further comprising at least one strain of yeast, more preferably wherein the at least one strain of lactic acid bacteria is as defined in claims 6 or 7 and/or the at least one strain of acetic acid bacteria is as defined in claims 8 or 9 and/or the at least one strain of yeast is as defined in claim 11.

13. The composition according to claim 12, wherein said composition comprises at least one strain of *Lactobacillus fermentum* and at least one strain of *Acetobacter pasteurianus,* and optionally and preferably further comprises at least one strain of *Lactobacillus plantarum.*

14. Fermented plant material comprising beans and/or pulp derived from fruit pods of the species *Theobroma cacao,* preferably fermented cocoa beans, obtainable using the method as defined in any one of claims 1 to 11.

15. A method for preparing chocolate and cocoa products comprising the use of the fermented cocoa beans as defined in claim 14.

16. Chocolate and cocoa products prepared using the fermented cocoa beans as defined in claim 14.

17. A bacteria strain according to any one of the following definitions:
- a lactic acid bacteria strain *Weissella ghanensis* represented by the isolate deposited with the Belgian Coordinated Collections of Microorganisms (BCCM) under accession number LMG P-23179, or a mutant or variant thereof, or a bacterium having at least 93% 16S rRNA sequence similarity to the said strain;
- an acetic acid bacteria strain *Acetobacter ghanensis* represented by the isolate deposited with the Belgian Coordinated Collections of Microorganisms (BCCM) under accession number LMG P-23177, or a mutant or variant thereof, or a bacterium having at least 93% 16S rRNA sequence similarity to the said strain; or
- an acetic acid bacteria strain *Acetobacter senegalensis* represented by the isolate deposited with the Belgian Coordinated Collections of Microorganisms (BCCM) under accession number LMG P-23176, or a mutant or variant thereof, or a bacterium having at least 93% 16S rRNA sequence similarity to the said strain.

18. Use of any one strain according to claim 17 for regulating fermentation of plant material comprising beans and/or pulp derived from fruit pods of the species *Theobroma* cacao.
